# EUROPEAN PATENT SPECIFICATION

(11) **EP 2 069 493 B1**
(45) Date of publication and mention of the grant of the patent: **10.07.2013**
(21) Application number: 07815528.0
(22) Date of filing: 09.11.2007
(51) Int. Cl.: A61K 39/145, C12N 9/80, C12N 15/74

(54) **METHODS AND DEVICES FOR THE DELIVERY OF PEPTIDES INTO THE GASTRIC MUCOSA**
VERFAHREN UND VORRICHTUNGEN ZUR ZUFÜHRUNG VON PEPTIDEN IN DIE MAGENSCHLEIMHAUT
PROCEDES ET DISPOSITIFS PERMETTANT L'ADMINISTRATION DE PEPTIDES DANS LA MUQUEUSE GASTRIQUE

(30) Priority: 10.11.2006 US 558570; 14.03.2007 AU 2007901331 P
(43) Date of publication of application: 17.06.2009
(73) Proprietor: Ondek Pty Ltd, Rushcutters Bay, NSW 2011 (AU)
(72) Inventor: Marshall, Barry J., Subiaco WA 6008 (AU)
(74) Representative: Cabinet Plasseraud
(86) International application number: PCT/AU2007/001725
(87) International publication number: WO 2008/055316

(56) References cited:
- WO-A-2006/015445
- WO-A1-2006/015445
- US-A- 5 972 336
- US-A1- 2003 007 980
- FERRERO R L ET AL: "RECOMBINANT ANTIGENS PREPARED FROM THE UREASE SUBUNITS OF HELICOBACTER SPP.: EVIDENCE OF PROTECTION IN A MOUSE MODEL OF GASTRIC INFECTION", INFECTION AND IMMUNITY, AMERICAN SOCIETY FOR MICROBIOLOGY, WASHINGTON, US, vol. 62, no. 11, 1 November 1994 (1994-11-01), pages 4981-4989, XP002011225, ISSN: 0019-9567
- ZHAO WENFENG ET AL.: 'Oral vaccination with liposome-encapsulated recombinant fusion peptide of urease B epitope and cholera toxin B subunit affords prophylactic and therapeutic effects against H. pylori infection in BALB/c mice' VACCINE vol. 25, no. 44, 01 November 2007, pages 7664 - 7673, XP022294870
- TOMB J.-F. ET AL.: 'The complete genome sequence of the gastric pathogen Helicobacter pylori' NATURE vol. 388, no. 6642, 07 August 1997, pages 539 - 547, XP002950919

## Description

### FIELD

The present invention relates generally to the field of Helicobacter-based vector, plasmid vector and shuttle vector systems, as novel Helicobacter constructs that include a non-Helicobacter peptide are provided. The invention also relates to the field of peptide delivery for the treatment of non-Helicobacter associated disease, wherein the peptide is delivered at the mucosa in *vivo*

### BACKGROUND

*Helicobacter pylori* are a gram-negative spiral shaped bacterium found almost exclusively in the human gastric mucosa. The acidity of the human stomach is an effective barrier to colonization by essentially all bacteria, with the exception of *Helicobacter* species.

*H. pylori* have been described as a causative agent of chronic infection. In particular, Helicobacter has been established to play a critical role in peptic ulcer, gastric adenocarcinoma, and primary gastric lymphoma.

*H. pylori* have the unique ability to colonize and persist for decades within the human gastric mucosa, despite development of a mucosal inflammatory and immune response. This characteristic renders *H. pylori* an interesting candidate for the delivery of peptides though the mucosa. However, this particular application has not found application in mucosal delivery systems in part owing to its involvement in a variety of diseases. A need continues to exist for a delivery system for peptides employing these important organisms having a reduced risk of pathology to the host.

The development of mucosal delivery has also been hindered by the poor immunogenicity of antigens delivered by conventional approaches because of natural barrier functions of the host that prevent access to the mucosal compartment. Hence, a need continues to exist in the medical arts for improved delivery mechanisms for pharmacologically active molecules such as peptides at the mucosal surface sufficient to elicit a useful and beneficial immunogenic response. Such would provide an effective *in vivo* delivery system for pharmacological active agents, as well as an effective method for immunization, i.e., antigen exposure at a mucosal surface sufficient to elicit a general humoral and mucosal immune response.

US2003007980 and US5972336 use the urease gene or subunits as an antigen to vaccinate the subject against Helicobacter

WO2006015445 describes a Helicobacter based vaccine wherein the HopE Helicobacter sequence may be fused to a non Helicobacter molecule of interest and said construct is delivered into the gastrointestinal tract by using a Helicobacter bacterium which is transformed with said fusion protein.

### SUMMARY

In its broadest sense, the present invention is directed to subject-matter as defined in the appended claims.

The present invention is directed to overcoming the above-mentioned challenges and others related to the use of *Helicobacter* and in the treatment of disease. The present invention is exemplified in a number of implementations and applications, some of which are summarized below.

In accordance with some aspects, compositions, methods and systems are provided for preparing and using a Helicobacter-based construct comprising a *Helicobacter* sequence having a promoter region and a non-Helicobacter sequence encoding a non-Helicobacter molecule of interest. This construct in some embodiments is described as a vector or a plasmid vector, wherein the promoter sequence is capable of controlling the expression of the non-Helicobacter sequence encoding a molecule of interest.

### Helicobacter Constructs:

In one aspect, the disclosure provides a composition comprising a *Helicobacter* construct, particularly a *Helicobacter pylori* nucleic acid construct. In order to deliver peptides into the stomach wall (gastric mucosa) DNA sequences are inserted within and between the urease genes of *Helicobacter pylori* so that the bacterium can express the encoded peptide.

In some embodiments, the *Helicobacter* nucleotide sequence of the *Helicobacter* construct comprises a first *Helicobacter* sequence, Y1, a second *Helicobacter* sequence Y2, and a non-Helicobacter sequence X encoding a non-*Helicobacter* molecule of interest. A schematic of one embodiment of this construct appears in Formula 1: Formula 1:

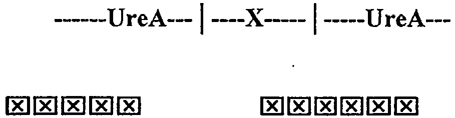

The non-Helicobacter nucleotide sequence of interest, "X", which may comprise a nucleic acid sequence that encodes a peptide of interest. In some embodiments, the non-Helicobacter sequence X is heterologous to the *Helicobacter pylori* species. This molecule of interest may be further described in some embodiments as capable of providing a beneficial and/or therapeutic effect to an animal delivered as an expressed product from a recombinant *Helicobacter* containing the construct that is introduced into an animal.

In the invention, the construct comprises a first *Helicobacter* sequence Y1 defined as a first portion of a native UreA or UreB gene sequence, a second *Helicobacter* sequence Y2 defined as a second portion of a native UreA or UreB gene, and a non-*H*, *pylori* nucleotide sequence of interest, "X".

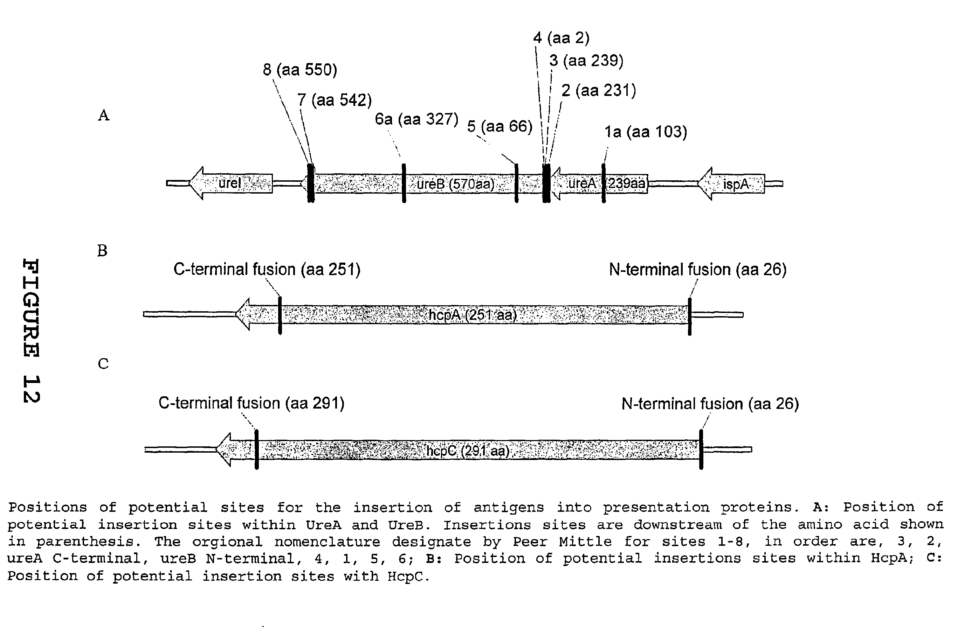

In some embodiments, the *Helicobacter* construct comprises a construct as depicted in Figure 12.

In other embodiments, *Helicobacter* construct is further defined as an attenuated *Helicobacter pylori* construct.

The *H. pylori* nucleic acid construct may further comprise, in some embodiments, a promoter sequence, a secretory sequence and/or a reporter gene sequence. In particular embodiments, a recombinant cell transformed with the *Helicobacter* construct is provided. In some embodiments, these recombinant cells are recombinant E. *coli* cells or *H. pylori* cells.

In some aspects, the *Helicobacter-based* vector and vector plasmid constructs that contain the *Helicobacter* construct comprise a pharmacologically active molecule of interest defined as a protein, peptide or any other molecule. In some embodiments, the isolated nucleic acid molecule may be further described as comprising cDNA, genomic DNA, RNA, or a hybrid molecule thereof. In particular embodiments, the nucleic acid is cDNA.

By way of example, a protein and/or peptide of interest may comprise ghrelin, amylin, insulin, motilin, β-glucosidase, a chemical chaperone, or other molecule useful in the treatment and/or management of Gauchers disease, cell wasting, human immunodeficiency disease (AIDS), appetite suppression, preparations useful in the treatment of diabetes, etc.

### Recombinant Cells:

In other aspects, a composition comprising a recombinant cell is provided comprising cells transformed with the plasmid vectors and/or vectors that include a *Helicobacter* construct as described herein. In some embodiments, the recombinant cell comprises a sequence encoding a non-*Helicobacter pylori* pharmacologically active molecule of interest. In other embodiments, the nucleic acid sequence encoding the *non-Helicobacter pylori* pharmacologically active molecule of interest comprises a secretory signal polypeptide. In some embodiments, the recombinant cell is capable of secreting an expressed product corresponding to the *non-Helicobacter* molecule of interest at the surface of the recombinant cell. In this manner, the expressed product of the molecule of interest may be delivered at or through the mucosal surface of an animal, such as at the intestinal mucosa. In some embodiments the recombinant cell is a recombinant *Helicobacter pylori* cell such as a *Helicobacter* strain 26695 or B128.

### Pharmaceutical Preparations:

The present disclosure provides a variety of pharmaceutically acceptable preparations formulated for delivery to a patient, such as, for example, delivery gastrically, orally, or intranasaly. In particular embodiments, the compositions are suitable for delivery at a mucosal surface. In particular embodiments, the composition is suitable for delivery to the mucosal surface or lining. In some embodiments, the mucosal surface is the gastric mucosal surface.

The various delivery forms of the compositions are readily prepared for use in the practice of the present invention given the specific types and ratios of specific *Helicobacter, Helicobacter* constructs and other delivery vehicles described herein, and those formulation techniques known to those in the formulary arts, such as are described in Remington's Pharmaceutical Sciences, 20th edition, Mack Publishing Company.

It is envisioned that the delivery system may be employed in animals, particularly primates, including humans, equines, bovines, ovines, and rodents, fish and birds. It is also anticipated that the preparations may be used on both infants and adults, as well as parentally or for administration to pregnant or lactating animals. The preparations and methods may be further described as suitable for both male and female animals.

### Vaccines:

In some embodiments, the composition is further defined as a vaccine in a pharmacologically acceptable carrier solution. As part of a vaccine, the composition comprising the *Helicobacter* construct is introduced into an animal in a manner such that the expressed product, i.e., the molecule of interest "X", is capable of making contact with or at a mucosal surface of an animal. By way of example, a mucosal surface of an animal may include the gastric mucosa, the nasal mucosa, etc.

In some embodiments, the *Helicobacter* based vaccine comprises cells transformed with a *Helicobacter* based construct, such as a plasmid vector as described herein. By way of example, the cells transformed with the *Helicobacter* based plasmid vector may comprise E. *coli* cells or *Helicobacter pylori* cells. In some embodiments, the vaccine may be further defined as a live attenuated vaccine. In particular embodiments, the composition will include an adjuvant. In some embodiments, the vaccine is capable of providing delivery of the non*-Helicobacter* molecule of interest at a mucosal surface.

### Vaccination/Immunization:

In yet another aspect, a method is described for vaccinating an animal. In some embodiments, the method comprises administering a composition comprising a vaccine comprising cells transformed with the *Helicobacter*- based vector and/or plasmid vectors as described herein. In other embodiments, the method provides for the delivery of an effective amount of the pharmacologically active molecule of interest sufficient to eliminate or inhibit a disease or particular physiological and/or pathological condition in the animal, or sufficient to elicit an immune response specific for the pharmacologically active molecule of interest.

By way of example, the non-Helicobacter molecule of interest that may be provided to an animal in the vaccine preparations of the present invention may comprise a mammalian or non-mammalian protein, peptide, enzyme, hormone, or any combination of these. In particular embodiments, the molecule of interest is further defined as a pharmacologically active molecule of interest that is a human pharmacologically active molecule of interest. In some embodiments the pharmacologically active molecule of interest is a human pathogen molecule/antigen, human protein antigen, such as amylin or an analog or derivative thereof, ghrelin, or an analog or derivative thereof.

In particular embodiments, the vaccines of the present invention provides immunity and/or an enhancement of disease resistance to the human pathogen, Ebola virus, HIV virus, Marburg virus, influenza virus, and the like. Replication competent vaccines based on attenuated recombinant vesicular stomatitis virus vectors have been described by Jones et *al.* (2005)⁴³ that include Ebola glycoprotein and Marburg glycoprotein. Hence, vaccine preparations containing constructs of the Helicobacter-based vector systems and plasmid vector systems described herein, with these and other glycoproteins associated with human pathogens, may also be provided according to the present invention.

In another aspect, a method of immunizing an animal is described. In some embodiments, the method comprises providing a composition comprising the *Helicobacter* vaccine as described herein to an animal and administering to the animal an effective amount of the composition sufficient to elicit an acceptable immune response in the animal. In some embodiments, the acceptable immune response is elicited upon the administration of a treatment regimen comprising one or more effective doses of the composition. These methods may be used in veterinary immunization as well as in the immunization of humans.

The following nucleic acid and amino acid sequences are referenced throughout the description of the present invention:
SEQ ID NO: 1 - Nucleotide sequence of plasmid pHP1 (2796 nucleotides) +ve strand.
SEQ ID NO: 2 - Nucleotide sequence of pHP1 (2796 nucleotides) -ve strand.
SEQ ID NO: 3 - Nucleotide sequence of plasmid pHP3 (3444 nucleotides).
SEQ ID NO: 4 - Hepatitis C virus antigen (HCV) nucleotide Sequence (580 nucleotides).
SEQ ID NO: 5 - Nucleotide sequence 135 bp (45 amino acids) immunogenic coding sequence from the Hepatitis C virus (HCV) core antigen.
SEQ ID NO: 6 - Nucleotide sequence (1108 nucleotides) of the surface exposed loop of the HopE gene (at nt504, aa position 168) of *H. pylori.*
SEQ ID NO: 7 - Upstream primer (29 nucleotides).
SEQ ID NO: 8 - Downstream Primer (28 nucleotides).
SEQ ID NO: 9 - Oligonucleotide Primer (15 nucleotides).
SEQ ID NO: 10 - Nucleotide sequence of H. pylori insertion construct, HopE gene with nucleotide sequence of interest, "X". (580 bp HopE - "X" bp - 502 bp HopE). The nucleotide sequence of interest, "X", may comprise a nucleic acid sequence that encodes a molecule of interest, such as a biologically valuable molecule of interest. A biologically valuable molecule of interest may comprise an enzyme, protein, peptide, or other molecule that is capable of providing a beneficial or therapeutic effect to an animal as delivered as an expressed product though or at a mucosal surface, such as the gastric mucosa. In some embodiments, "X" is a nucleic acid sequence comprising 60 nucleotide bases to 150 nucleotide bases, or 69 nucleotide bases to 138 nucleotide bases.
SEQ ID NO:11 - Nucleotide sequence for fusion protein of HopE and p60, insertion of p60 nucleic acid sequence (23amino acids) at nucleic acid position 504 (corresponding to amino acid (aa) position 168) in HopE sequence.
SEQ ID NO:12 - Nucleotide sequence for fusion protein of HopE and HCCA, insertion of HCCA nucleic acid sequence (46 amino acids) at nucleic acid position 504 (corresponding to amino acid (aa) position 168) in HopE nucleic acid sequence.
SEQ ID NO:13 - nucleic acid sequence for p60 (69 nucleotide bases).
SEQ ID NO:14 - nucleic acid sequence for HCCA (138 nucleotide bases).

### BRIEF DESCRIPTION OF THE DRAWINGS

The invention will be described in conjunction with the accompanying drawings, in which:
FIG. 1, in accordance with one embodiment of the invention, illustrates the vector constructs, pHPA1 (2.8 kb).
FIG. 2, in accordance with one embodiment of the invention, presents a schematic diagram of the plasmid construct pHP3 (3.4 kb).
FIG 3, in accordance with one embodiment of the invention, illustrates the vector construct, pTM103-8.
FIG. 4, in accordance with one embodiment of the invention, illustrates the chemical structure of sulfasalazine (SSN).
FIG. 5, in accordance with one embodiment of the invention, illustrates a schematic using an ion exchange resin (Amberlite XE-96) conjugated with a dye (Azure-A).

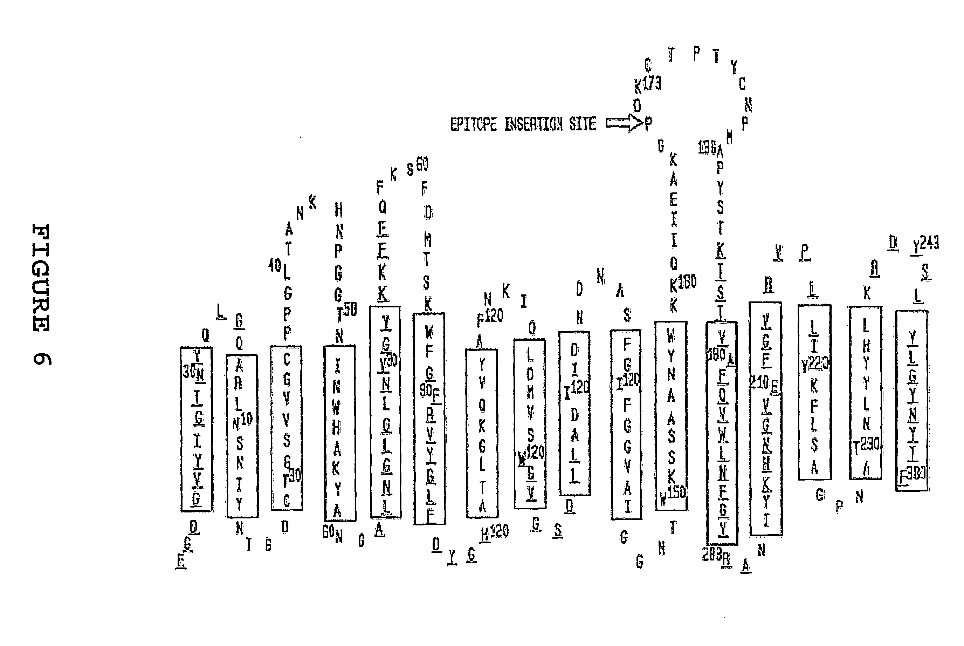

FIG. 6, in accordance with one embodiment of the invention, illustrates the predicted structure of HopE, showing the insertion site for a sequence of interest, such as a nucleic acid sequence encoding HCCA or p60 epitopes. Adapted from Bina and associates

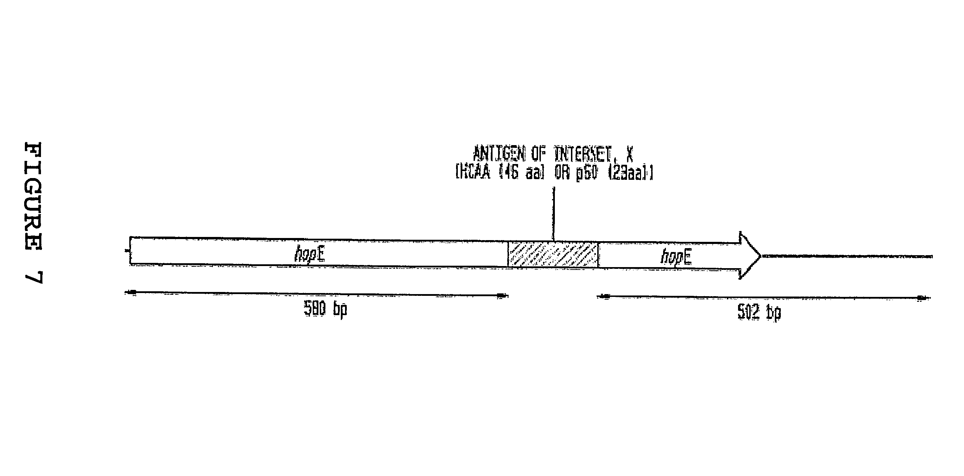

FIG. 7, in accordance with one aspect of the disclosure, illustrates recombinant DNA molecules produced using SOE PCR. DNA coding either the HCCA or p60 epitope was inserted into *hopE* at the position corresponding to aa 168 of HopE. To allow homologous recombination and replacement of the genomic copy of hopE in *H. pylori,* sequences homologous to genomic *hopE* were included flanking the insertion site for epitope coding DNA.

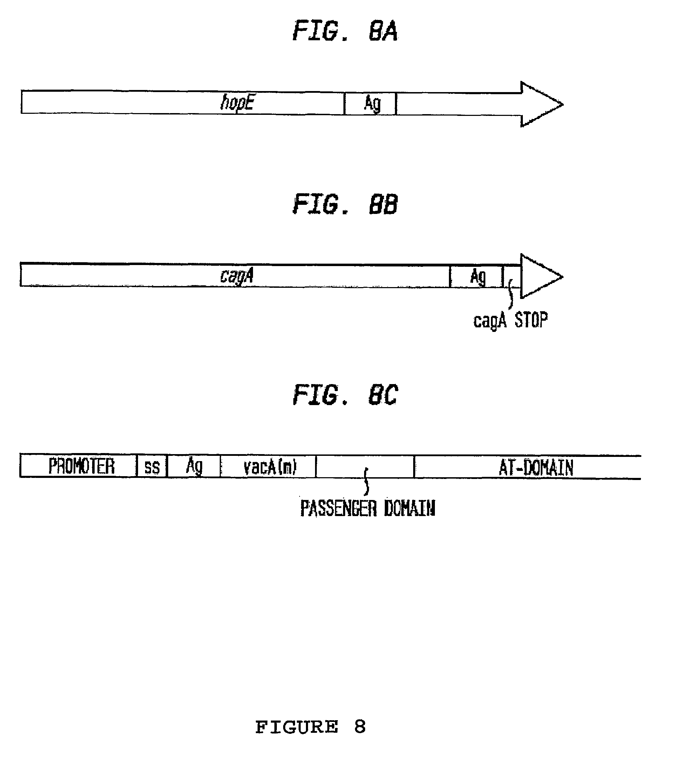

FIG. 8, in accordance with one embodiment of the disclosure provides a diagrammatic representation of recombinant genes in *H. pylori.* Column A: Antigen (Ag) coding DNA was inserted into *hop*E at position corresponding to aa 168, within a region corresponding to a putative surface expose loop; Column B: Antigen coding DNA was inserted at the *cagA* at the 5' terminus directly upstream of the *cagA* stop condon; Column C: B128 *vac*A was replaced DNA Coding the 26695 *vacA* promoter sequence, signal sequence (ss), mature (m) *vacA,* passenger domain and autotransporter (AT) domain. Antigen coding DNA was inserted directly upstream of the signal sequence.

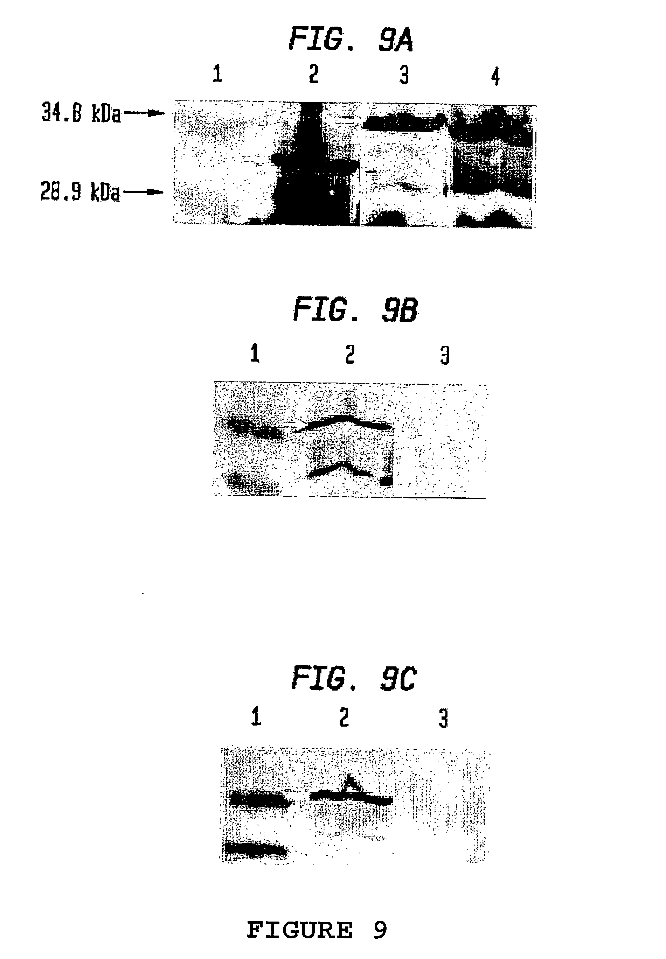

FIG. 9, in accordance with one embodiment of the disclosure, provides a Western Blot analysis of *H. pylori* B128 (7,13) containing HCCA (B128:HCCA:*hop*E, Blot A. 1° antibody: α-HopE; 2° antibody; α-rabbit - AP conjugate, Lane 1: marker; Lane 2: B128; Lane 3: B128:HCCA:*hop*E; Lane 4: B128:p60:*hop*E. Blot B: 1° antibody: α-HCCA; 2° antibody; α- mouse - AP- conjugate. Lane 1: marker; Lane 2:B128:HCCA;*hop*E; Lane 3: B128. Blot C. 1° antibody: α-p60; 2° antibody; α-mouse - Ap conjugate (2° antibody). Lane 1: marker; Lane 2: B128:p60*:hop*E; Lane 3: B128. The white arrow indicates the band corresponding to either HopE or fusion protein.

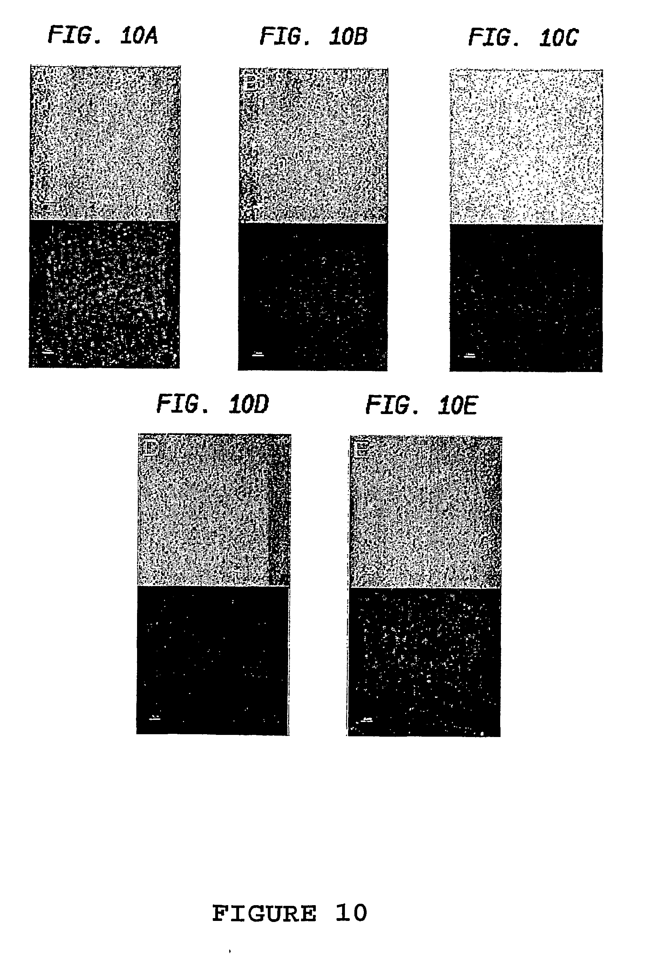

FIG. 10, in accordance with one embodiment of the disclosure , presence an immunofluorescence based microscopy analysis of H. pylori B128 containing HCCA inserted into HopE (B128:HCCA:*hop*E). Upper Row: Phase contrast microscopy; Lower Row: Fluorescence microscopy. Column A: *H. pylori* B128. 1° antibody: α-HopE; 2° antibody; α-rabbit Alexafluor 488 (AF-488). Column B: *H. pylori* B128. 2° antibody; α-rabbit AF-488. Column C. *H. pylori* B128. 1° antibody: α-HCCA; 2° antibody: α-mouse AF-488. Column D: *H. pylori* B128:HCCA:hopE. 2° antibody: α-mouse AF-488. Column E. *H. pylori* B128:HCCA-hopE. 1° antibody: α-HCCA: 2° antibody: α-mouse AF-488.
FIG. 11, in accordance with one embodiment of the disclosure , presence a whole cell based ELISA analysis of recombinant *H. pylori* 26695 and B128 (7.13). Column A: Recombinants 26695:HCCA:hopE or 26695:p60:hopE; Column B: Recombinants B128:HCCA:*hopE* B128:p60:*hopE*; Detection of p60 antigen: 1° antibody: α-p60; 2° antibody: α-mouse-alkaline phosphatase (AP) conjugate; Detection of HCCA: 1° antibody: α-HCCA; 2° antibody: α-mouse-AP conjugate. Error bars: SEM (n=3).
Fig. 12 shows the positions of potential sites for the insertion of antigens into presentation proteins.
Fig. 13 shows the process of DNA assembly using SOE PCR. A: Protocol for the assembly of two amplicons. B: Protocol for the assembly of 3 DNA amplicons.
Fig. 14 shows deleted regions of ureA or ureB in strain H. pylori strain X47, replaced with the rpsL.ermB cassette (ref). Each recombinant of X47 only contained one insertion.
Fig. 15 shows selectivity of urea plates for bacteria producing functional urease. Left (growth): wild type X47 (urease positive); Right (no growth): X47 with the rpsLermB cassette disrupting UreA and UreB production (urease negative).
Fig. 16 shows molecular structures of the H. pylori urease.
Fig. 17 shows protocol for the transformation of H. pylori using the streptomycin contra-selection system.
Fig. 18 shows the insertion sites used for in frame fusion to the two haemagglutinin epitopes and FLAG epitope are indicated by their corresponding number 1a to 8. Linkers are indicated as well as the small GPSL linker between epitopes. The genomic organisation of the urease operon is showed. Arrow heads represent ureA, ureB, ispA and ureI genes. The length of the displayed DNA fragment is indicated by the base pair unit (bp).
Fig.19 shows a Western blot analysis of recombinant urease. Lane 1: Protein molecular weight marker; Lane 2: Site 1a; Lane 3: Site 3; Lane 4: Site 4; Lane 5: Site 8; Lane 6: Site 1a; Lane 7: Site 3; Lane 8: Site 4; Lane 9: Site 8. Lanes 2 - 5 are samples diluted 1:6. Lanes 6 - 9 are samples diluted 1:2. Results indicated that the FLAG tagged epitope is presented at sites 1a, 4 and 8 of urease. At the higher dilution the epitope for site 3 cannot be detected; the epitope was detectable at the higher dilution (Lanes 3 and 7 respectively).

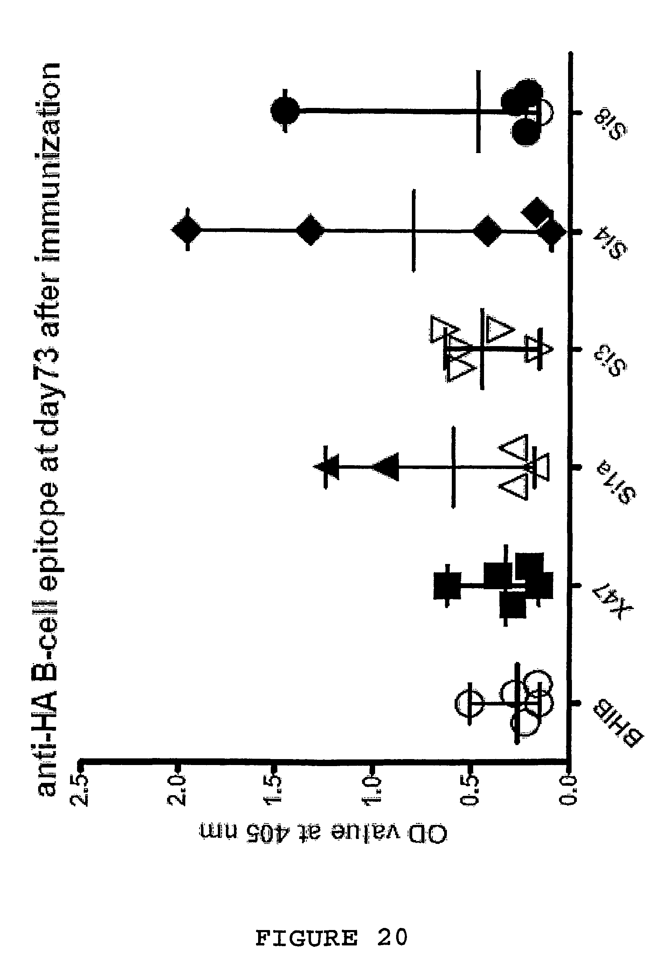

Fig.20 shows that Female C57BL/6 mice (n=5) were immunized with 200µl of 10⁹ cfu / ml intra-gastrically on day 0 with *H. pylori.* 73 days later, mice were bled and serum collected. IgG titres were determined by ELISA to measure HA-B cell epitope specific IgGs production.BHIB indicates the control group immunized with the growth medium BHIB, X47 the group immunized with X47 wild type *H. pylori,* Si1a, Si3, Si4 and Si8 are groups immunized with recombinant *H. pylori* expressing the haemagglutinin epitopes at insertion site 1a, 3, 4 and 8, respectively. The black plain labels show mice colonized with *H. pylori* and the empty white labels indicate mice that were not colonized at 73 days.

### DETAILED DESCRIPTION

The present invention is believed to be applicable to a variety of different types of bacterial and vaccine constructs that include a *Helicobacter* or *Helicobacter-*based vector system of delivery. It is advantageous to define several terms before describing the invention. While the present invention is not necessarily limited to such applications, various aspects of the invention may be appreciated through a discussion of various examples using this context.

Before describing the present invention in detail, it is to be understood that this invention is not limited to particularly exemplified methods and may, of course, vary. It is also to be understood that the terminology used herein is for the purpose of describing particular embodiments of the invention only, and is not intended to be limiting which will be limited only by the appended claims.

Furthermore, the practice of the present invention employs, unless otherwise indicated, conventional immunological and molecular biological techniques and pharmacology within the skill of the art. Such techniques are well known to the skilled worker, and are explained fully in the literature. See, *e.g.,* Coligan et al., (Current Protocols in Protein Science (1999) Volume I and II (John Wiley & Sons Inc.); Sambrook et al., (Molecular Cloning: A Laboratory Manual, 2nd & 3rd Editions. Cold Spring Harbor Laboratory press (1989) (2001); and Bailey, S F. and Ollis, D.F., Biochemical Engineering, Fundamentals. McGraw-Hill Book Company, NY, 1986.

It must be noted that as used herein and in the appended claims, the singular forms "a," "an," and "the" include plural reference unless the context clearly dictates otherwise. Thus, for example, a reference to "a nucleic acid" includes a plurality of such nucleic acids, and a reference to "an isolated peptide" is a reference to one or more peptides, and so forth.

Unless defined otherwise, all technical and scientific terms used herein have the same meanings as commonly understood by one of ordinary skill in the art to which this invention belongs. Although any materials and methods similar or equivalent to those described herein can be used to practice the present invention, the preferred materials and methods are now described.

Delivery of therapeutic compositions and nucleic acids to specific target sites within the animal body is an ongoing challenge faced by the drug development industry. The present inventor has developed a Helicobacter-based bacterial delivery system capable of carrying vectors encoding biologically active agents, wherein these agents are expressed on the surface of the bacterium or secreted there from. In one embodiment, the bacterium is a species of *Helicobacter, H. pylori.* In some embodiments, the strain of *H. pylori* can be any strain known in the field. In some embodiments, the *H. pylori* strain is a non-pathogenic strain such as genomic strain 26695. Another strain that may be used is *H. pylori* strain B128, particularly variant 7.13.

In another embodiment, a bacterium, other than *Helicobacter,* is utilized wherein the bacterium has been genetically altered such that it has *Helicobacter* or H. pylori features including the ability to chronically colonize the gastric mucosa or other areas of gastrointestinal tract, urinary tract, bronchial epithelium or other mucosal surface, without significant toxicity to the host.

In one embodiment, the *H. pylori* have been manipulated so that some of the pathogenic features have been removed and/or attenuated. For example, the vacuolating cytotoxin and the cag pathogenicity island genes can be removed so that the *H. pylori* are less pathogenic. Attenuating mutations can be introduced into *Helicobacter* using nonspecific mutagenesis either chemically, using N-methyl-N-nitro-N-nitrosoquanidine, or using recombinant DNA technologies.

The skilled person will appreciate that the methods of the present invention could be used to deliver biologically active agents. Examples of suitable agents include ones which are capable of functioning locally or systemically, *e.g.,* an agent capable of exerting endocrine activities affecting local or whole-body metabolism and/or an agent which is capable of regulating the activities of cells belonging to the immuno/hematopoeitic system and or an agent which is capable of affecting the viability, growth and differentiation of a variety of normal or neoplastic cells in the body or affecting the immune regulation or induction of acute phase inflammatory responses to injury and infection and/or an agent which is capable of enhancing or inducing resistance to infection of cells and tissues mediated by chemokines acting on their target cell receptors, or the proliferation of epithelial cells or the promotion of wound healing and/or an agent which modulates the expression or production of substances by cells in the body.

Specific examples of such biologically active agents include insulin, growth hormone, prolactin, calcitonin, luteinizing hormone, parathyroid hormone, somatostatin, thyroid stimulating hormone, vasoactive intestinal polypeptide, a structural group 1 cytokine adopting an antiparallel 4 α helical bundle structure such as IL-2, IL-3, IL-4, IL-5, IL-6, IL-7, IL-9, IL-10, IL-11, IL-12, IL-13, CM-CSF, M-CSF, SCF, IFN-γ, EPO, G-CSF, LIF, OSM, CNTF, GH, PRL or IFN α/β, a structural group 2 cytokine which are often cell-surface associated, form symmetric homotrimers and the subunits take up the conformation of β-jelly roll described for certain viral coat proteins such as the tumor necrosis factor (TNF) family of cytokines, e.g. TNF α, TNF β, CD40, CD27 or FAS ligands, the IL-1 family of cytokines, the fibroblast growth factor family, the platelet derived growth factors, transforming growth factor β and nerve growth factors, a structural group 3 cytokine comprising short chain α/β molecules, which are produced as large transmembrane pre-cursor molecules which each contain at least one EGF domain in the extracellular region, *e.g.,* the epidermal growth factor family of cytokines, the chemokines characterized by their possession of amino acid sequences grouped around conserved cysteine residues (the C--C or C--X--C chemokine subgroups) or the insulin related cytokines, a structural group 4 cytokine which exhibit mosaic structures such as the heregulins or neuregulins composed of different domains, *e.g.,* EGF, immunoglobulin-like and kringle domains.

Alternatively, the biologically active agent can be a receptor or antagonist for biologically active agent as defined above.

In some embodiments, the *H. pylori-*based vector and/or vector plasmid construct is employed to create a transformed cell (such as an *E. coli* cell or *Helicobacter* cell) that permits the expression and secretion of a non-*Helicobacter* pharmacologically active molecule of interest at the mucosal membrane of a host to which the transformed cell preparation is administered. The isolated nucleic acid molecule contained within the transformed cell (or vector) may comprise one or more nucleic acid constructs in which nucleic acid encoding the pharmacologically active molecule of interest is under control of *H. pylori* regulatory sequences.

Suitable vectors and shuttle vector sequences can be chosen or constructed to contain appropriate regulatory sequences, including promoter sequences, terminator fragments, enhancer sequences, marker genes and other sequences as appropriate. Vectors may be plasmids, viral, e.g. phage, or phagemid, as appropriate. For further details, for example, see Sambrook et *al., supra.* Many techniques and protocols are known for the manipulation of nucleic acid, for example in preparation of nucleic acid constructs, mutagenesis, sequencing, introduction of DNA into cells and gene expression, and analysis of proteins, as described in detail in Short Protocols in Molecular Biology, Second Edition, Ausubel et al. eds., John Wiley & Sons, 1992.

In some embodiments, the coding sequence(s) for the pharmacologically active molecules of interest is contained in an operon, i.e., a nucleic acid construct for multi-cistronic expression. In an operon, transcription from the promoter results in a mRNA which comprises more than one coding sequence, each with its own suitably positioned ribosome binding site upstream. Thus, more than one agent (pharmacologically active molecule of interest) can be translated from a single mRNA. Use of an operon enables expression of the pharmacologically active molecule of interest to be coordinated.

A nucleic acid construct or vector comprising a coding sequence for a pharmacologically active molecule of interest is preferably under the control of a promoter for expression in *H. pylori.*

In one embodiment, the promoter employed in accordance with the present invention is expressed constitutively in *H. pylori.* Use of a constitutive promoter avoids the need to supply an inducer or other regulatory signal for expression to take place. Preferably, the promoter directs expression at a level at which the *H. pylori* host cell remains viable, *i.e.,* retains some metabolic activity, even if growth may be reduced. Advantageously then, such expression may be at a low level. For example, where the expression product accumulates intracellularly, the level of expression may lead to accumulation of the expression product at less than about 10% of cellular protein, preferably about or less than about 5%, for example about 1-3%.

In some embodiments, a method is provided comprising delivering a messenger nucleic acid sequence, such as an mRNA sequence, corresponding to a nucleic acid sequence encoding a molecule of interest to an animal. By way of example, such a messenger nucleic acid sequence (mRNA) corresponding to a therapeutic peptide, protein, hormone or pro-hormone may be prepared so as to provide a peptide, protein, or hormone to the animal upon expression of that messenger nucleic acid sequence in the animal. For example, such a hormone may be insulin, and such a pro-hormone may be pro-insulin. Thus, it is envisioned that the present invention has application as a gene therapy method for the treatment of human disease, such as for the treatment of diabetes.

The promoter may be homologous to the *H*. *pylori* strain employed, *i.e.* one found in that strain of *H. pylori* in nature. In some embodiments, the promoter is an arabinose inducible promoter. Other promoters include FlaB sigma 54 promoter (Josenhans et at., 1998, FEMS Microbiol Lett, 161(2): 263-73), T7 promoter, and nir B promoter of *Salmonella* (Chatfield et al., 1992, Biotechnology, 10(8): 888-92).

In another embodiment the promoter is inducible. Inducible promoters that may be used with clinical grade vectors include, but are not limited to, an inducible promoter as described in U.S. Pat. No. 6,242,194 issued to Kullen *et at.,* a lactose inducible promoter such as that used in E. *coli* plasmids *(e.g.,* pBluescript™ from Stratagene) or the endogenous lactose promoter in *Lactobacillus,* and promoters induced during anaerobic growth, such as the promoter for alcohol dehydrogenase (adhE), as described in Aristarkhov et at., (1999) J. Bacteriology, 178(14), 4327-4332).

In one embodiment, the constructs of the present invention also include a toxic gene. These toxic genes are preferably under the control of an inducible promoter so that, on completion of treatment, the *Helicobacter* can be readily eliminated by inducing the expression of the toxic gene. Non-limiting examples of toxic genes include bacterial autolysins under the control of an inducible promoter. The autolysing gene may then be triggered at the appropriate time and place in the gastrointestinal tract through the use of one or more of the inducible promoters as described herein.

In some embodiments, the engineered *Helicobacter* vector and plasmid vector constructs are sensitive to oxygen. This oxygen sensitivity is another method for limiting dissemination of the clinical grade vectors of the present invention. The environment of the human gut is very low in oxygen, suitable for growth of anaerobic and microacrophulic microorganisms, including *Helicobacter.* Thus, an efficient means of eliminating *Helicobacter,* once they have exited the human body upon discharge of intestinal waste into the oxygen-rich outside environment, is to engineer genes into the transformed microorganisms that confer oxygen sensitivity.

The nucleic acid construct or constructs of the present invention may also comprise a secretory signal sequence. Thus, in some embodiments, the nucleic acid encoding the pharmacologically active molecule of interest (for example, a non*-Helicobacter* polypeptide) may provide for secretion of the molecule at a cell membrane by appropriately coupling a nucleic acid sequence encoding a secretory signal sequence to the nucleic acid sequence encoding the molecule (polypeptide). The ability of *Helicobacter* harboring the nucleic acid to secrete the polypeptide may be tested *in vitro* in culture conditions, which maintain viability of the *Helicobacter.*

Suitable secretory signal sequences include any of those with activity in Gram negative organisms such as *Escherichia, Klebsiella* and *Salmonella.* Secretory signal sequences include the *Staphylokinase* enzyme secreted by some strains of *Staphylococcus,* which is known to function in both Gram-positive and Gram-negative hosts (see "Gene Expression Using Bacillus", Rapoport (1990), Current Opinions in Biotechnology, 1:21-27).

Other secretory signal sequences that can be used include, for example, the β-lactamase gene (Talmadge et at., 1980, Proc. Natl. Acad Sci. USA 77:3369-3373) or the enteroinvasive *E. coli* hemolysin A (hlyA) (Su et at., 1992, Microbial Pathogen, 13:465-476). An illustrative list of secretory signal sequences is presented in Pugsley, 1988, Protein secretion across the outer membrane of gram-negative bacteria. In: Protein Transfer and Organelle Biogenesis, R. C. Dand and P. W. Robbins (eds). Academic Press, Inc., San Diego, pp 607-652.

Selectable markers provide researchers and technicians a convenient means for distinguishing transformed microorganisms from non-transformed ones in a mixed population. One means of identifying transformed organism is to incorporate a selectable marker nucleic acid sequence into the plasmid containing the gene of interest. The selectable marker sequence is generally inserted downstream of the gene of interest and is driven off the same promoter. As a result, cells successfully transformed with the gene of interest will also be transformed with the selectable marker nucleic acid sequence. When antibiotic resistance is used as the selectable marker, only transformed cells will survive and/or grow in media containing the antibiotic.

Thus, antibiotic resistance is a convenient and much used phenotype when developing transformants. However; vectors having antibiotic resistant genes as selective markers are capable of horizontal gene transfer that can endow other organisms with antibiotic-resistant phenotypes. This risk is especially acute when *Helicobacter* is used as part of a therapeutic vector.

In order to use *Helicobacter* as a gene delivery system to animals, the present disclosure presents, in some embodiments, a clinical grade vector system that does not use an antibiotic selection marker. One of the alternatives to using antibiotic resistance genes provided by the present delivery systems includes clinical grade vectors having chromosomal deletions or lethal mutations in a "house-keeping" gene. Next, a functional analogous house-keeping gene is inserted into a plasmid encoding for the pharmacologically active molecule of interest. Consequently, the house-keeping gene becomes the selectable marker allowing for the rapid isolation and identification of transformants.

Examples of "house keeping genes" include genes that encode for any number of metabolic regulators and/or enzymes including, but not limited to kinases, proteases, synthetases, dehydrogenases and others. Another alternative to antibiotic resistance genes provided by the present invention includes clinical grade vectors having reporter genes incorporated into the plasmid containing the gene encoding for the pharmacologically active molecule of interest. Other examples of reporter genes used in accordance with the teachings of the present invention include Green Fluorescent Protein (GFP), β-galactosidase and amylase.

In one embodiment, the pharmacologically active molecule of interest has cytokine activity. Cytokines are discussed in The Cytokine Facts Rook, Callard and Gearing (1994), Academic Press. Preferred molecules, such as polypeptides with cytokine activity are interleukins, including Interleukin-2 (IL-2) and Interleukin 6 (IL-6).

In some embodiments, the *Helicobacter* vector and plasmid vector systems comprise a nucleic acid construct as described above that is introduced into a *Helicobacter* or other suitable host cell, to provide transformed cells. Thus, a further aspect provides a method comprising introducing nucleic acid as disclosed into a non-pathogenic *Helicobacter.* Transformation of a culture of host cells, such as *Helicobacter,* may employ any available technique. For *H. pylori* cells, suitable techniques may include calcium chloride transformation, electroporation and transfection using bacteriophage.

The introduction of the plasmid vector into a *Helicobacter* cell may be followed by causing or allowing expression from the nucleic acid, *e.g.,* by culturing *H. pylori* under conditions suitable for expression of the gene. Growing the *Helicobacter* in culture under conditions for expression of the pharmacologically active molecule of interest may be employed to verify that the *Helicobacter* contain the encoding nucleic acid and is able to produce the encoded molecule.

In a further aspect, the present invention provides a method of delivering a therapeutic or prophylactic dose of a biologically active agent *in vivo,* the method comprising administering to a subject an effective amount of the non-pathogenic preparation of the *H. pylori* compositions and vaccines of the present invention.

It will be appreciated that the methods of the present invention and the use of a non-invasive or non-pathogenic *Helicobacter* as described herein provide a wide range of therapeutic methods which would enable the skilled person to manipulate, for instance, the immune response of a subject. Thus, in one aspect, a method of regulating the survival, growth, differentiation, effector functions or susceptibility to infection of cells or tissues is provided which comprises administering to a subject a non-invasive or non-pathogenic *Helicobacter* as defined herein.

In another aspect, a method of boosting an immune response against tumor cells or an infection colonizing a mucosal surface or adjacent or distant tissue is described which comprises administering to a subject a non-invasive or non-pathogenic *Helicobacter* as defined herein.

In yet another aspect, a method of modulating the type of immune response (antibody versus cell-mediated) against a pathogenic infectious agent is described which comprises administering to a subject a non-invasive or non-pathogenic *Helicobacter* as defined herein.

In another aspect, a method of modulating the infiltration of normal tissues with inflammatory or tumor cells is described which comprises administering to a subject a non-invasive or non-pathogenic *Helicobacter* as defined herein.

In some aspects, a method of controlling the rate of growth, rate of invasion or survival of tumor cells is described which comprises administering to a subject a non-invasive or non-pathogenic *Helicobacter* as defined herein.

In yet another aspect, a method of inducing apoptosis in tumor cells is described which comprises administering to a subject a non-invasive or non-pathogenic *Helicobacter* as defined herein.

Other aspects describe for a method of down-regulating an immune response which comprises administering to a subject a non-invasive or non-pathogenic bacterium which expresses a pharmacologically active molecule of interest as defined herein.

In another aspect, a method of treating an allergic autoimmune or other immune dysregulative disease state is described which comprises administering to a subject a non-invasive or non-pathogenic *Helicobacter* which expresses a pharmacologically active molecule of interest.

The subject can be any primate, equine, bovine, porcine, ovine, rodent, fish, or bird. In one embodiment, the subject is human. Administration may conveniently be nasal or oral.

In a therapeutic context, i.e., where the pharmacologically active molecule of interest is a biologically active agent that provides a beneficial effect to the subject, the amount of the agent and/or treatment regimen will preferably be provided in a "therapeutically effective amount", this being sufficient to show benefit to a subject. Such benefit may be at least amelioration or a reduction in the severity or occurrence of at least one symptom. In a prophylactic context, the amount may be sufficient to reduce the deleterious effect on the subject of a subsequent pathogenic challenge, for instance by enhancing the immune response. The actual amount administered, and rate and time-course of administration will depend on the aim of the administration, e.g., the biological effect sought in view of the nature and severity of the challenge, and is the subject of routine optimization. Prescription of treatment, including prophylactic vaccination, for example, decisions on dosage etc, is within the responsibility of general practitioners and other medical doctors.

A composition comprising *Helicobacter* may be administered in accordance with the present disclosure alone or in combination with other treatments, either simultaneously or sequentially.

The present disclosure also provides a pharmaceutical composition comprising a *Helicobacter* as disclosed. Such a pharmaceutical composition is in one embodiment preferably suitable for application to a mucosal membrane.

Pharmaceutical compositions according to the present disclosure , and for use, may comprise, in addition to the *Helicobacter,* a pharmaceutically acceptable excipient, carrier, buffer, stabilizer or other materials well known to those skilled in the art. Such materials should be nontoxic and should not interfere with the efficacy of the pharmacologically active molecule of interest. The nature of the carrier or other material may depend on the route of administration. For oral administration a parenterally acceptable aqueous solution may be employed which is pyrogen-free and has suitable pH, isotonicity and stability. Those of relevant skill in the art are well able to prepare suitable solutions. Preservatives, stabilizers, buffers, antioxidants and/or other additives may be included, as required. As discussed, a pharmaceutical comprising a *Helicobacter* for administration in accordance with the present invention may comprise one or more nutrient substances, e.g., an energy source such as glucose, amino acids and so on.

In another aspect, a method of manufacture of a pharmaceutical formulations provided comprising formulating *Helicobacter* as disclosed with a suitable carrier medium suitable for administration to an individual. In one embodiment, the pharmaceutical is suitable for application to a mucosal membrane of an individual.

In yet another aspect, a non-pathogenic *Helicobacter* expressing a heterologous pharmacologically active molecule of interest for pharmaceutical use is provided, e.g., for use in a method of treatment of the human or animal body by surgery or therapy, including prophylaxis ("vaccination").

In one embodiment the method can be used to treat, prevent or palliate a disease such as cancer. The methods and delivery system can also be used to treat or prevent a disease or condition of the immune/hematopoietic system, a disease or condition of the reproductive system, a disease or condition of the musculoskeletal system, a disease or condition of the cardiovascular system, a disease or condition described as mixed fetal, a disease or condition of the excretory system, a disease or condition of the neural/sensory system, a disease or condition of the endocrine system, a disease or condition of the respiratory system, a disease or condition of the digestive system and a disease or condition associated with connective/epithelial tissue or disease or condition caused by bacterial, viral or parasitic infection.

In another embodiment, the *Helicobacter* delivery system described herein is capable of concomitant or sequential delivery of a number of different nucleic acid molecules, which encode products capable of treating a number of conditions or diseases described herein. Moreover, preferred delivery systems would also deliver compositions capable of producing additional desirable physiological effects such as appetite suppression or enhancement.

An example of suicide system in *H. pylori* has been described by Panthel et al. 2003 (Infection & Immunity, 71: 109-116). This system introduces a plasmid into *H*. *pylori* which contains the PhiX174 lysis gene E. To eradicate the strain, incubation at 42°C for 5 hours was used. In *vivo* this would mean that the animal would consume a drink at 45-50°C to raise the temperature of the gastric environment above 42°C.

A second example is the L-Dap selection system, commonly used to allow survival of bacterial mutants on supplemented plates (see, for example, Kirata et al. 1997 (Infection & Immunity, 65: 4158-4164)). In this system the animal subject must supplement their diet with a missing substrate i.e., diamino-pimelic-acid (DAP), in order for the DapE deficient *H*. *pylori* mutant to survive. In order to eradicate the mutants, DAP consumption is ceased.

A third possible system relates to metronidazole sensitivity of *H*. *pylori* because of its rdxA gene. Excessive replication of the *rdxA* gene is harmful to mammalian cells and *E. coli.* However, duplication may be tolerated by the bacterium. Therefore a *Helicobacter* species of the present invention can be engineered to contain two copies of rdxA which prevents the normal mutation-dependant *rdxA* loss. The introduction of at least two functional *rdxA* genes into the *Helicobacter* genome will result in a *Helicobacter* strain that is permanently sensitive to metronidazole. Jeong et al. 2000 (J. Bacteriol., 182: 5082-5090) showed that the nitroreductase produced by a functional *rdxA* gene converts metronidazole from a prodrug to a bactericidal compound. The mode of action of the active compound is to cause DNA breaks of the *Helicobacter* genome.

Throughout the specification, unless the context requires otherwise, the word "comprise" or variations such as "comprises" or "comprising", will be understood to imply the inclusion of a stated integer or group of integers but not the exclusion of any other integer or group of integers.

### Definitions:

Prior to setting forth the invention, it may be helpful to an understanding thereof to set forth definitions of certain terms that will be used hereinafter.

The term "a" and "the" as used in the present descriptive is intended to include both one (the singular) and more than one (plural).

The phrase, "effective level" refers to the level of the desired activity of the pharmacologically active molecule of interest and not necessarily limited to the number of molecules. For example, the effective level of amylin (as an exemplary pharmacologically active molecule of interest) may be decreased to stimulate ghrelin secretion by using amylin antagonists, without a necessary concomitant decrease in the amount of free amylin present in a subject.

An "antibiotic resistance gene" as defined herein includes heterologous nucleic acid sequences purposely provided to a vector and used as a selection system. The term "antibiotic resistance gene" does not include other mechanisms or genes that impart antibiotic resistance to naturally occurring micro-flora organisms.

The term "attenuated" as used herein for example to describe a bacterial strain, particularly an E. coli or a Helicobacter strain such as Helicobacter pylori, is defined as a strain that is less virulent and/or toxic (invasive) that a native, wild type bacterial strain.

The term "biologically active" as used herein refers to ability to perform a biological function and with reference to a polypeptide implies that the polypeptide adopts a stable conformation ("folded form") which is the same or closely analogous to its native conformation. When folded correctly or substantially correctly, for example with formation of proper folded units' α-helices, ß-sheets, domains, disulphide bridges etc., a polypeptide should have the ability to perform its natural function. Generally, the unit of function in a polypeptide is a domain.

"Clinical grade vector" as used herein means a plasmid or other expression vector that is capable of being expressed in Helicobacter or a non-pathogenic bacterium engineered to have features of Helicobacter. The clinical grade vectors of the present invention do not use antibiotic resistance markers for selection and/or have been modified to prevent replication outside the host e.g., such as a suicide vector.

"Detectable immune response" as used herein is either an antibody (humoral) or cytotoxic (cellular) response formed in an animal in response to an antigen that can be measured using routine laboratory methods including, but not limited to enzyme-linked immunosorbent assays (ELISA), radio-immune assays (RIA), Enzyme-linked ImmunoSPOT (ELISPOT), immunofluorescence assays (IFA), complement fixation assays (CF), Western Blot (WB) or an equivalent thereto.

"Gene of interest" as used herein refers to any nucleic acid sequence encoding for a pharmacologically active molecule of interest, such as, polypeptide or protein, whose expression is desired. The nucleic acid sequence may or may not include the promoter or other regulatory components. The vectors and plasmid vectors also include constructs capable of producing anti-sense RNA. "Gene therapy" as used herein is defined as the delivery of a gene of interest to an animal in need thereof using a recombinant vector. The gene of interest can be a transgene encoding for a therapeutic or prophylactic protein or polypeptide including, but not limited to cytokines, anti-inflamnmatories, anti-proliferatives, antibiotics, metabolic inhibitors/activators and immunologically active antigens and fragments thereof. Furthermore, "gene therapy" as used herein also includes gene replacement technologies directed at both inherited and non-inherited disorders.

The term *Helicobacter* includes all bacteria of the genus *Helicobacter* including *H. pylori* and *Helicobacter* mustelae. The term also includes bacteria that have similar biology to H. pylori in that they are capable of residing on the gastric mucosa of primates and/or capable of establishing a chronic, but isolated infection of the mucosa. The term also encompasses bacteria that have been modified so that the bacterium has *H. pylori* features, such as the ability to reside on the gastric mucosa.

A "heterologous" polypeptide is a peptide that is not native or that has been mutated from the native form as it existed in Helicobacter, i.e., not expressed by Helicobacter in nature or prior to introduction into Helicobacter, or an ancestor thereof.

"Host" as used herein defines the intended recipient of a therapeutic composition of the present invention. Host includes all animals. Specifically, hosts include, but are not limited to, primates (including man), bovine, equine, canine, feline, porcine, ovine, rabbits, rodents, birds and fish.

"Immunologically inert" as used herein shall mean any substance, including microorganisms such as microflora that does not provoke a significant immune response in its host. Examples of immunologically inert materials as used herein include stainless steel, biocompatible polymers such as poly-L-lactide, medical grade plastics and the microflora organisms of the present invention.

An "insertion construct", as used herein, shall mean a nucleic acid construct that comprises a portion of Helicobacter pylori nucleic acid sequence, such as a portion of a nucleic acid sequence that encodes the HopE gene, and a non-Helicobacter nucleic acid sequence that encodes a molecule of interest.

An "isolated nucleic acid" is a nucleic acid sequence that is not identical to any naturally occurring nucleic acid or any fragment of a naturally occurring genomic nucleic acid sequence spanning more than three separate genes. The term therefore covers, for example, (a) a DNA molecule which has the sequence of part of a naturally occurring genomic DNA molecule but is not flanked by both of the coding sequences that flank that part of the molecule in the genome of the organism in which it naturally occurs; (b) a nucleic acid incorporated into a vector or into the genomic DNA of a prokaryote or eukaryote in a manner such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion protein.

"Percent identity" (homology) of two amino acid sequences or of two nucleic acids is determined using the algorithm of Karlin and Altschul, 1990, Proc. Natl. Acad. Sci. USA. 87:2264-2268, 1990, modified as in Karlin and Altschul (Proc. Natl. Acad. Sci. USA 90:5873-5877, 1993). Such an algorithm is incorporated into the NBLAST and XBLAST programs of Altschul et al. (J. Mol. Biol. 215:403-410, 1990). BLAST nucleotide searches are performed with the NBLAST program, score=100, wordlength=12, to obtain nucleotide sequences homologous to a nucleic acid molecule of the invention. BLAST protein searches are performed with the XBLAST program, score=50, wordlength=3, to obtain amino acid sequences homologous to a reference polypeptide (e.g., SEQ ID NO: 2). To obtain gapped alignments for comparison purposes, Gapped BLAST is utilized as described in Altschul et al. (Nucleic Acids Res. 25:3389-3402, 1997). When utilizing BLAST and Gapped BLAST programs, the default parameters of the respective programs (e.g., XBLAST and NBLAST) are used.

A "pharmacologically active" molecule, as used in the description of the present invention, is defined as a molecule, such as a peptide, protein, nucleic acid, or other organic or inorganic substance that is capable of eliciting a pharmacologically detectable activity or response in a cell, such as in a cell culture, or in a chemical or biochemical reaction media or assay, or in an animal. The pharmacologically active molecules of interest of the present invention may include, for example, biologically active molecules as described herein.

A "molecule of interest" as used in the description of the present invention, is defined as a protein, peptide, enzyme, or other molecule that when provided to a cell or animal, provides a protein, peptide, enzyme or other molecule that is capable of correcting and/or treating a pathology, deficiency or other condition deemed appropriate, such as in the treatment of a disease.

The term "reporter gene" as used herein is a nucleic acid sequence incorporated into (or adjacent to) the heterologous nucleic acid sequence encoding a pharmacologically active molecule of interest that provides the transformed vector expressing the molecule of interest an identifiable phenotype. Non-limiting examples of reporter genes include GFP, ß-galactosidase, amylase and CAT.

"Screening marker" as used herein refers to an identifying characteristic (phenotype) provided to a transformed vector made in accordance with the teachings of the present invention. In one embodiment of the present invention, the screening marker is a reporter gene.

"Selectable marker," "selectable gene," "reporter gene" and "reporter marker" (referred to hereinafter as a "selectable marker") as used herein refer to nucleic acid sequences encoding for phenotypic traits that permit the rapid identification and isolation of a transformed bacterial vector. Generally, bacterial vectors deemed "clinical grade" and made in accordance with the teachings of the present invention are those vectors having selectable markers that do not encode for antibiotic resistance.

A "significant immune response" is any immune response that would provide immunity (i.e., invoke the production of specific antibody) in an animal against a given antigenic molecule or immunogen.

A "therapeutically effective amount" of a pharmacologically active molecule of interest or combination of said molecules as described herein is understood to comprise an amount effective to elicit the desired response but insufficient to cause a toxic reaction. A desired response, for example, may constitute the formation of a sufficient and/or acceptable detectable antibody titer level in a blood sample. The dosage and duration of treatment of the preparation to be administered to a subject will be determined by the health professional attending the subject in need of treatment, and will consider the age, sex, weight, extent of existing diseased state and/or tissue damage of the subject, and specific formulation of Helicobacter and the gene of interest product being used as the treatment for the subject.

A "transgene" as used herein refers to a gene that is inserted, using cDNA technology, into a cell in a manner that ensures its function, replication and transmission as a normal gene.

A "transforming nucleic acid sequence" as used herein means a plasmid, or other expression cassette containing a nucleic acid sequence encoding a pharmacologically active molecule of interest. In some embodiments of the present invention, the nucleic acid sequence can encode for one or more therapeutic agents. "Transforming nucleic acid sequence" can also be used to mean a "transgene" in accordance with certain embodiments of the present invention. In another embodiment of the present invention the transforming nucleic acid sequence includes nucleic acid sequence encoding for a promoter and/or other regulatory elements.

The term "cancer" as used herein refers to neoplastic diseases eg., leukemia, cancers and "hyper-proliferative disorders"). The neoplasm may be located in a tissue selected from the group consisting of: colon, abdomen, bone, breast, digestive system, liver, pancreas, prostate, peritoneum, lung, blood (*e.g*., leukemia), endocrine glands (adrenal, parathyroid, pituitary, testicles, ovary, thymus, thyroid), uterus, eye, head and neck, nervous (central and peripheral), lymphatic system, pelvic, skin, soft tissue, spleen, thoracic, and urogenital.

In one embodiment the term "cancer" also encompasses pre-neoplastic conditions selected from the group consisting of hyperplasia (e.g., endometrial hyperplasia), metaplasia (eg, connective tissue metaplasia) and/or dysplasia (e.g., cervical dysplasia, and bronchopulmonary dysplasia).

In another embodiment, the term "cancer" also encompasses benign dysproliferative disorder selected from the group consisting of: benign tumors, fibrocystic conditions, and tissue hypertrophy.

The term "a disease or condition of the immune/hematopoietic system" as used herein refers to a disease or condition selected from the group consisting of: anemia, pancytopenia, leukopenia, thrombocytopenia, leukemia, Hodgkin's disease, non-Hodgkin's lymphoma, acute lymphocytic anemia (ALL), plasmacytomas, multiple myeloma, Burkitt's lymphoma, arthritis, asthma, AIDS, autoimmune disease, rheumatoid arthritis, granulomatous disease, immune deficiency, inflammatory bowel disease, sepsis, neutropenia, neutrophilia, psoriasis, immune reactions to transplanted organs and tissues, systemic lupus erythematosus, hemophilia, hyper-coagulation, diabetes mellitus, endocarditis, meningitis, Lyme Disease, Celiac disease (gluten sensitivity) and allergies.

The term "a disease or condition of the reproductive system" as used herein refers to a disease or condition selected from the group consisting of: cryptorchism, prostatitis, inguinal hernia, varicocele, leydig cell tumors, verrucous carcinoma, prostatitis, malacoplakia, Peyronie's disease, penile carcinoma, squamous cell hyperplasia, dysmenorrhea, ovarian adenocareinoma, Turner's syndrome, mucopurulent cervicitis, Sertoli-Leydig tumors, ovarian cancer, uterine cancer, pelvic inflammatory disease, testicular cancer, prostate cancer, Klinefelter's syndrome, Young's syndrome, premature ejaculation, diabetes mellitus, cystic fibrosis, Kartagener's syndrome, testicular atrophy, testicular feminization, anorchia, ectopic testis, epididymitis, orchitis, gonorrhea, syphilis, testicular torsion, vasitis nodosa, germ cell tumors, stromal tumors, dysmenorrhea, retroverted uterus, endometriosis, fibroids, adenomyosis, anovulatory bleeding, amenorrhea, Cushing's syndrome, hydatidiform moles, Asherman's syndrome, premature menopause, precocious puberty, uterine polyps, dysfunctional uterine bleeding, cervicitis, chronic cervicitis, mucopurulent cervicitis, cervical dysplasia, cervical polyps, Nabothian cysts, cervical erosion, cervical incompetence, cervical neoplasms, pseudohermaphroditism, and premenstrual syndrome.

The term "a disease or condition of the musculoskeletal system" as used herein refers to a disease or condition selected from the group consisting of bone cancers (*e.g*., osteochondromas, benign chondromas, chondroblastoma, chondromyxoid fibromas, osteoid osteomas, giant cell tumors, multiple myeloma, osteosarcomas), Paget's Disease, rheumatoid arthritis, systemic lupus erythematosus, osteomyelitis, Lyme Disease, gout, bursitis, tendonitis, osteoporosis, osteoarthritis, muscular dystrophy, mitochondrial myopathy, cachexia, and multiple sclerosis.

The term "a disease or condition of the cardiovascular system" as used herein refers to a disease or condition selected from the group consisting of: myxomas, fibromas, rhabdomyomas, cardiovascular abnormalities (e.g., congenital heart defects, cerebral arteriovenous malformatiens, septal defects), heart disease (e.g., heart failure, congestive heart disease, arrhythmia, tachycardia, fibrillation, pericardial Disease, endocarditis), cardiac arrest, heart valve disease (e.g., stenosis, regurgitation, prolapse), vascular disease (e.g., hypertension, coronary artery disease, angina, aneurism, arteriosclerosis, peripheral vascular disease), hyponatremia, hypernatremia, hypokalemia, and hyperkalemia.

The term "a disease or condition described as mixed fetal" as used herein refers to a disease or condition selected from the group consisting of: spina bifida, hydranencephaly, neurofibromatosis, fetal alcohol syndrome, diabetes mellitus, PKU, Down's syndrome, Patau syndrome, Edwards syndrome, Turner syndrome, Apert syndrome, Carpenter syndrome, Conradi syndrome, Crouzon syndrome, cutis laxa, Cornelia de Lange syndrome, Ellis-van Creveld syndrome, Holt-Oram syndrome, Kartagener syndrome, Meckel-Gruber syndrome, Noonan syndrome, Pallister-Hall syndrome, Rubinstein-Taybi syndrome, Scimitar syndrome, Smith-Lemli-Opitz syndrome, thrombocytopenia-absent radius (TAR) syndrome, Treacher Collins syndrome, Williams syndrome, Hirschsprung's disease, Meckel's diverticulum, polycystic kidney disease, Turner's syndrome, and gonadal dysgenesis, Klippel-Feil syndrome, Ostogenesis imperfecta, muscular dystrophy, Tay-Sachs disease, Wilm's tumour, neuroblastoma, and retinoblastoma.

The term "a disease or condition of the excretory system" as used herein refers to a disease or condition selected from the group consisting of: bladder cancer, prostate cancer, benign prostatic hyperplasia, bladder disorders (e.g., urinary incontinence, urinary retention, urinary obstruction, urinary tract infections, interstitial cystitis, prostatitis, neurogenic bladder, hematuria), renal disorders (*e.g*., hydronephrosis, proteinuria, renal failure, pyelonephritis, urolithiasis, reflux nephropathy, and unilateral obstructive uropathy).

The term "a disease or condition of the neural/sensory system" as used herein refers to a disease or condition selected from the group consisting of: brain cancer (*e.g*., brain stem glioma, brain tumors, central nervous system (Primary) lymphoma, central nervous system lvmphoma. cerebellar astrocyroma, and cerebral astrocytoma, neurodegenerative disorders (e.g., Alzheimer's Disease. Creutzfeldt-Jakob Disease, Parkinson's Disease, and Idiopathic Presenile Dementia), encephalomyelitis, cerebral malaria, meningitis, metabolic brain diseases (e.g., phenylketonuria and pyruvate carboxylase deficiency), cerebellar ataxia, ataxia telangiectasia, and AIDS Dementia Complex, schizophrenia, attention deficit disorder, hyperactive attention deficit disorder, autism, and obsessive compulsive disorders.

The term "a disease or condition of the respiratory system" as used herein refers to a disease or disorder selected from the group consisting of: cancers of the respiratory system such as larynx cancer, pharynx cancer, trachea cancer, epiglottis cancer, lung cancer, squamous cell carcinomas, small cell (oat cell) carcinomas, large cell carcinomas, adenocarcinomas, allergic reactions, cystic fibrosis, sarcoidosis, histiocytosis X, infiltrative lung diseases (e.g., pulmonary fibrosis and lymphoid interstitial pneumonia), obstructive airway diseases (e.g., asthma, emphysema, chronic or acute bronchitis), occupational lung diseases (e.g., silicosis and asbestosis), pneumonia and pleurisy.

The term "a disease or condition of the endocrine system" as used herein refers to a disease or condition selected from the group consisting of: cancers of endocrine tissues and organs (*e.g*., cancers of the hypothalamus, pituitary gland. thyroid gland, parathyroid glands, pancreas, adrenal glands, ovaries, and testes), diabetes (e.g., diabetes insipidus, type I and type II diabetes mellitus), obesity, disorders related to pituitary glands *(e.g.,* hyperpituitarism, hypopituitarism, and pituitary dwarfism), hypothyroidism. hyperthyroidism, goiter, reproductive disorders *(e.g.,* male and female infertility), disorders related to adrenal glands *(e.g.,* Addison's Disease, corticosteroid deficiency, and Cushing's Syndrome), kidney cancer *(e.g.,* hypermephroma, transitional cell cancer, and Wilm's tumour), diabetic nephropathy, interstitial nephritis, polycystic kidney disease, glomerulonephritis *(e.g.,* 1gM mesangial proliferative glomerulonephritis and glomerulonephritis caused by autoimmune disorders; such as Goodpasture's syndrome), and nephrocalcinosis.

The term "a disease or condition of the digestive system" as used herein refers to a disease or condition selected from the group consisting of: ulcerative colitis, appendicitis, Crohn's disease, hepatitis, hepatic encephalopatby, portal hypertension, cholelithiasis, cancer of the digestive system *(e.g.,* biliary tract cancer, stomach cancer, colon cancer, gastric cancer, pancreatic cancer, cancer of the bile duct, tumors of the colon (*e.g*., polyps or cancers), and cirrhosis), pancreatitis, ulcerative disease, pyloric stenosis, gastroenteritis, gastritis, gastric atrophy, benign tumors of the duodenum, distension, irritable bowel syndrome, malabsorption, congenital disorders of the small intestine, bacterial and parasitic infection, megacolon, Hirschsprung's disease, aganglionic megacolon, acquired megacolon, colitis, anorectal disorders (*e.g*., anal fistulas, hemorrhoids), congenital disorders of the liver *(e.g.,* Wilson's disease, hemochromatosis, cystic fibrosis, biliary atresia, and alpha 1-antitrypsin deficiency), portal hypertension, cholelithiasis, and jaundice.

The term "a disease or condition of the connective/epithelial" as used herein refers to a disease or condition selected from the group consisting of: connective tissue metaplasia, mixed connective tissue disease, focal epithelial hyperplasia, epithelial metaplasia, mucoepithelial dysplasia, graft v. host disease, polymyositis, cystic hyperplasia, cerebral dysplasia, tissue hypertrophy, Alzheimer's disease, lymphoproliferative disorder, Waldenstron's macroglobulinemia, Crohn's disease, pernicious anemia, idiopathic Addison's disease, glomerulonephritis, bullous pemphigoid, Sjogren's syndrome, diabetes mellitus, cystic fibrosis, osteoblastoma, osteoclastoma, osteosarcoma, chondrosarcoma, osteoporosis, osteocarthritis, periodontal disease, wound healing, relapsing polychondritis, vasculitis, polyarteritis nodosa, Wegener's granulomatosis, cellulitis, rheumatoid arthritis, psoriatic arthritis, discoid lupus erythematosus, systemic lupus erythematosus, scleroderma. CREST syndrome, polymyositis, dermatomyositis, mixed connective tissue disease, relapsing polychondritis, vasculitis, Henoch-Schonlein syndrome, erythema nodosum, polyarteritis nodosa, temporal (giant cell) arteritis, Takayasu's arteritis, Wegener's granulomatosis, Reiter's syndrome, Behcet's syndrome, ankylosing spondylitis, cellulitis, keloids, Ehler Danlos syndrome, Marfan syndrome, pseudoxanthoma elasticum, osteogenesis imperfecta, chondrodysplasias, epidermolysis bullosa. Alport syndrome and cutis laxa.

The phrase "ghrelin-associated diseases and disorders" refers to any condition that can be treated prevented or ameliorated through the modulation of ghrelin activity. These include conditions that are enhanced, exacerbated or stimulated by ghrelin, for example, growth hormone release or drive to eat. The physiological actions of ghrelin are considered to include, by way of example, the stimulation of growth hormone release, the stimulation of hormone secretion from lactotrophs and corticotropes, orexigenic and cardiovascular actions, anti-proliferative effects on thyroid and breast tumors and regulation of gastric motility and acid secretion through vagal mediation. (See WO 2005021026).

Where the definition of terms departs from the commonly used meaning of the term, applicant intends to utilize the definitions provided herein, unless specifically indicated.

The invention will now be further described by reference only to the following non-limiting examples. It should be understood, however, that the examples following are illustrative, and should not be taken in any way as a restriction on the generality of the invention described herein. In particular, while the invention is described in detail in relation to the use of a specific *H. pylori* strain, it will be clearly understood that the findings herein are not limited to this strain.

### EXAMPLE 1, NOT PART OF THE INVENTION, VECTORS AND TRANSGENIC H. pylori ORGANISMS FOR STABLE EXPRESSION OF FOREIGN PROTEINS

The genetic manipulation of *H. pylori* is uncommon. The present example demonstrates the utility of the invention for providing a genetically transformed Helicobacter, particularly transformed *H*. *pylori*. The transformed bacterium are prepared using plasmids and plasmid vectors derived from *Helicobacter*, which have had been subject to prior manipulation in a non-Helicobacter organism, such as *E. coli .*

Several *H*. *pylori* plasmids described in the literature can be successfully converted to *H. pylori*/*E. coli* shuttle vectors. Many strains of *E. coli* have been reported to be naturally competent for DNA uptake. Resistance markers for streptomycin, rifampin and metronidazole have also been successfully transformed into most strains of *H. pylori.* However, while plasmid DNA from *E*. *coli* and other organisms can be introduced into *H*. *pylori,* these plasmids cannot be stably maintained. Moreover, *H. pylori* plasmids cannot be transformed into *E. coli* or *Helicobacter* species. Accordingly, *H. pylori* shuttle vectors must be constructed.

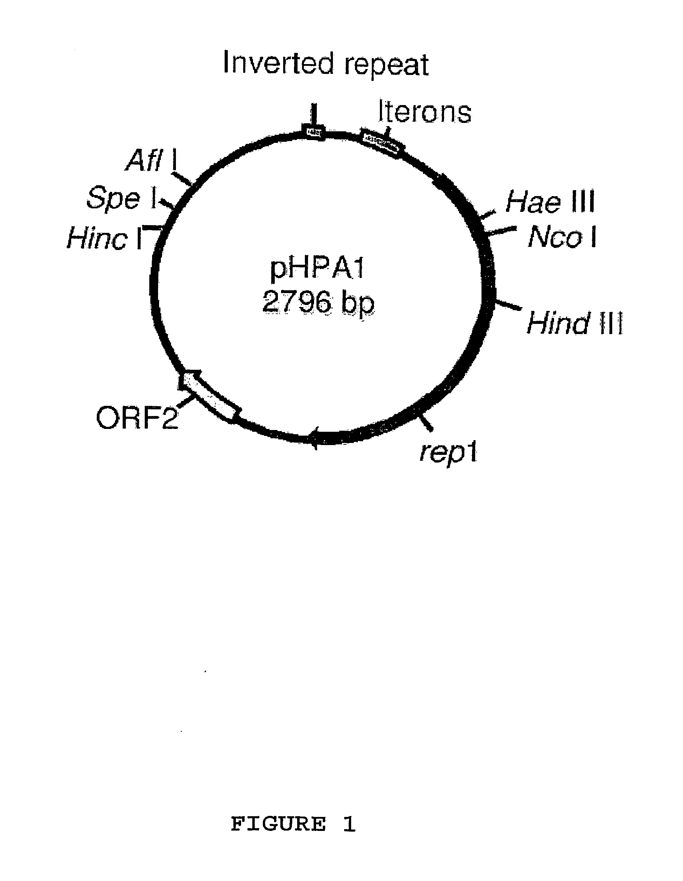

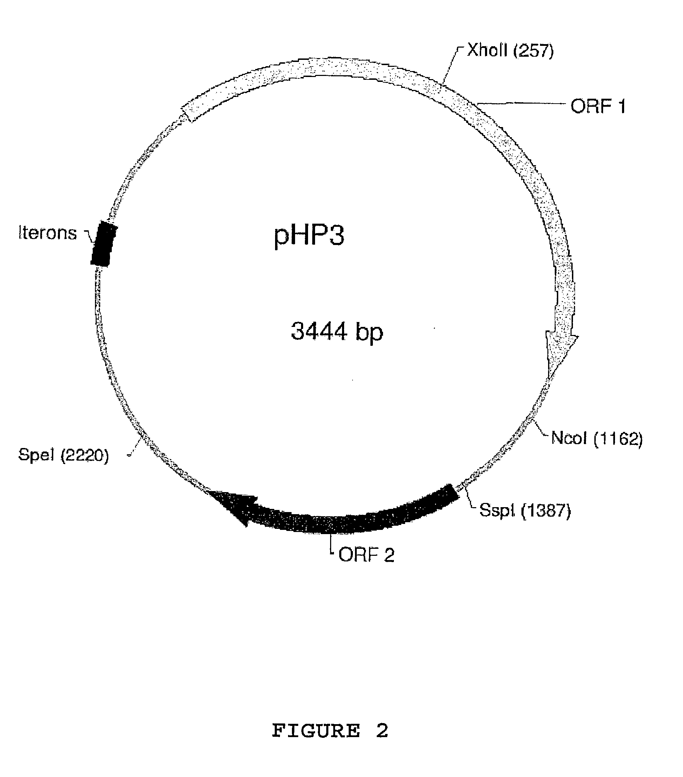

Two plasmids from *H*. *pylori* are illustrated in the schematics shown in Figures 1 and 2. Vectors pHPAI (2.8kb) (Fig. 1) and pHP3 (3.4kb) (Fig. 2) have been sequenced, and it has been revealed that pHPAI replicated via the theta mode of plasmid replication. In contrast to rolling-circle replicating plasmids, theta plasmids do not generate single-stranded DNA intermediates during replication and are thus more stable vector candidates because they are less prone to illegitimate recombination. Furthermore, the pHPAI origin of replication (ori) contains a series of direct repeat sequences (termed "iterons") that are involved in replication control and maintaining stable copy number. Vector pHP3 shares many of these features. The nucleotide sequences for these two vectors are shown below.

Plasmid pHP1 shown in double stranded form (top strand is (+ strand) SEQ ID: 1; bottom strand is (- strand) (SEQ ID NO: 2)

GTCATGCGCGTTGTTTTTAATTACATTTTAAACAACTTGTTGTTTTTACATGTTTTACTCGC 65
CAGTACGCGCAACAAAAATTAATGTAAAATTTGTTGAACAACAACAAAAATGTACAAAATGAGCG

ATGCGCGCGCGTGAGGGATTGGGGGTTGCAACCCCCTAAATAACGAAGCTGTAGGGTTTCTCATT 130
TACGCGCGCGCACTCCCTAACCCCCAACGTTGGGGGATTTATTGCTTCGACATCCCAAAGAGTAA

TTTGTGGTGAAAATGAATAAAACAGAACTTCTTGCCAACACTAACAGAACTTCTTGCCAACACTA 195
AAACACCACTTTTACTTATTTTGTCTTGAAGAACGGTTGTGATTGTCTTGAAGAACGGTTGTGAT

ACAGAACTTCTTGCCAACACTAACAGAACTTCTTGCCAACACTAACAGAACTTCTTTATTTTAAA 260
TGTCTTGAAGAACGGTTGTGATTGTCTTGAAGAACGGTTGTGATTGTCTTGAAGAAATAAAATTT

GTTATGATTATTAACAATTTTTAGACATAATAACAGCGTGTGAAGATACTTTTGTAGCGGTATTT 325
CAATACTAATAATTGTTAAAAATCTGTATTATTGTCGCACACTTCTATGAAAACATCGCCATAAA

CCTATGTGCGGCAAAATTTGGAGCAATTAGCTTGACTTGGTTGAGTTAGTGGGTTGGAGGATAGA 390
GGATACACGCCGTTTTAAACCTCGTTAATCGAACTGAACCAACTCAATCACCCAACCTCCTATCT

GAGGGCGACACCTCGTTAGGAGGTATCAATGTGAAAGTATTTGTCGTATTAGTTCTAGTATTAGT 455
CTCCCGCTGTGGAGCAATCCTCCATAGTTACACTTTCATAAACAGCATAATCAAGATCATAATCA

AATTCTCGCACAATTGCTATATTAGGCTTATTCGTGGTCTAACCCCTTGTTTATGGGGGTTGGCT 520
TTAAGAGCGTGTTAACGATATAATCCGAATAAGCACCAGATTGGGGAACAAATACCCCCAACCGA

CGTTATAAGCATACTGATACGATCACACTTATTATACACCAAAAGATAAGGAGTATAGAGTGGAA 585

GCAATATTCGTATGACTATGCTAGTGTGAATAATATGTGGTTTTCTATTCCTCATATCTCACCTT

TTTGATCAATCAGATTTACAAAAAGCGTTGAAAATATTAGATACACTCCCACAAACCCCACAAAT 650
AAACTAGTTAGTCTAAATGTTTTTCGCAACTTTTATAATCTATGTGAGGGTGTTTGGGGTGTTTA

TGAGCTACAAAAACAAGAAATACAAAACCGCATCAACAAAATAACAGAGACAATCATTAAAGAAT 715
ACTCGATGTTTTTGTTCTTTATGTTTTGGCGTAGTTGTTTTATTGTCTCTGTTAGTAATTTCTTA

TACTATCAAAGCATGAAATCAAGAAAGAAGAACTAGAACCCACTCTAACCCCAAAACCCACACCA 780
ATGATAGTTTCGTACTTTAGTTCTTTCTTCTTGATCTTGGGTGAGATTGGGGTTTTGGGTGTGGT

CTCAAAGAGCCACAAACCACCCCAACACCATGCAAAGATTTAGTGGTTAGCACCCCTAAAGATAA 845
GAGTTTCTCGGTGTTTGGTGGGGTTGTGGTACGTTTCTAAATCACCAATCGTGGGGATTTCTATT

AACCTAATATCACCTACCACAATAACGCTAATAAGGTCAATCTAGGGAAATTGAGCGAAAGGGAA 910
TTGGATTATAGTGGATGGTGTTATTGCGATTATTCCAGTTAGATCCCTTTAACTCGCTTTCCCTT

GCCAATCTTTTATTCGCTATTTTTCAAAAACTCAAAGCCCAAGGGAATACCCTCATTCGTTTTGA 975
CGGTTAGAAAATAAGCGATAAAAAGTTTTTGAGTTTCGGGTTCCCTTATGGGAGTAAGCAAAACT

ACCGCAAGATTTGAAACGCATGCTAAACATAGATATTTCTAATGAGCGCTTATCAGAAGTCGTTA 1040
TGGCGTTCTAAACTTTGCGTACGATTTGTATCTATAAAGATTACTCGCGAATAGTCTTCAGCAAT

TTAAGCTGTGGGATAGCATTAAAACCGCTGATTTTTGGAAAATTAGCGAAACCGAAACTTCAATC 1105
AATTCGACACCCTATCGTAATTTTGGCGACTAAAAACCTTTTAATCGCTTTGGCTTTGAAGTTAG

ATTCAAGAAAATTACATGCTTTTTAGTCGGTGTAAAATTGAATTGAACAAACCGAGTAAAGATTT 1170
TAAGTTCTTTTAATGTACGAAAAATCAGCCACATTTTAACTTAACTTGTTTGGCTCATTTCTAAA

GAAGTATTTAGAAATCCAACTCAACGATAACTATCAAGACTTACTCAACAATCTGGGCATGGGTC 1235
CTTCATAAATCTTTAGGTTGAGTTGCTATTGATAGTTCTGAATGAGTTGTTAGACCCGTACCCAG

AATACACTTCTTTCAATCTGTTAGAATTTCAAAGAGTGAGGGGTAAATACGCTAAAACGCTCTAT 1300
TTATGTGAAGAAAGTTAGACAATCTTAAAGTTTCTCACTCCCCATTTATGCGATTTTGCGAGATA

CGCTTGCTCAAGCAATACAAAAGCACAGGGATTTTGAGCGTGGAATGGACTCAATTCAGGGAGCT 1365
GCGAACGAGTTCGTTATGTTTTCGTGTCCCTAAAACTCGCACCTTACCTGAGTTAAGTCCCTCGA

TTTAGACATTCCAAAAGACTACAAAATGGAAAACATCGATCAAAAAGTCTTAACCCCCTCTCTCA 1430
AAATCTGTAAGGTTTTCTGATGTTTTACCTTTTGTAGCTAGTTTTTCAGAATTGGGGGAGAGAGT

AAGAACTCAGAAAAATCTACCCTTTTGAACACTTGAGCTATAAAAAAGAACGCAAAAGCCATTAC 1495
TTCTTGAGTCTTTTTAGATGGGAAAACTTGTGAACTCGATATTTTTTCTTGCGTTTTCGGTAATG

AAGCGCAAAGTAACCCACATTGATTTTTATTTTGAGCAATTTCCTTAAGGCGAAAATAAGAAACA 1560
TTCGCGTTTCATTGGGTGTAACTAAAAATAAAACTCGTTAAAGGAATTCCGCTTTTATTCTTTGT

AAACAAAGCCGACAAGCAACGCGCTCAAAGGGACATCAAGCTTGTAGCATGGGATATTCACAACC 1625
TTTGTTTCGGCTGTTCGTTGCGCGAGTTTCCCTGTAGTTCGAACATCGTACCCTATAAGTGTTGG

AAATCGCTAAAAGAAACGCAAAAGCCACTATGGAAGCTAGGTTTCTTGAATTGAAAACTTTGATC 1690
TTTAGCGATTTTCTTTGCGTTTTCGGTGATACCTTCGATCCAAAGAACTTAACTTTTGAAACTAG

GGCTATCAGTTCAGGAACAATGACAGTAGGAACAAATTAAAGATTGACAACACCACTTTTGAAAG 1755
CCGATAGTCAAGTCCTTGTTACTGTCATCCTTGTTTAATTTCTAACTGTTGTGGTGAAAACTTTC

AATCAAATGTATTTACATGTATCTTAACCCTAAAAATAAGCATAACCCCCAAAAATTCCTTGTAT 1820
TTAGTTTACATAAATGTACATAGAATTGGGATTTTTATTCGTATTGGGGGTTTTTAAGGAACATA

CCAACAAGACATTCGCATTGGAACTACTATATATCAATAGATACAGCCTAAAAAAAGACAACTT 1885
GGTTGTTCTGTAAGCGTAACCTTGATGATATATAGTTATCTATGTCGGATTTTTTTCTGTTGAA

GCTAGAAGAATTTAACCCCCCAAAATCCACCCTATCACCAACGAACCTATCAAGGAATTTGCAGA 1950
CGATCTTCTTAAATTGGGGGGTTTTAGGTGGGATAGTGGTTGCTTGGATAGTTCCTTAAACGTCT

ATACATCGGCAAAACGATTAACATCACCAACTTCAATGTGGATCAATGCCATGAGGGAATCAGCA 2015
TATGTAGCCGTTTTGCTAATTGTAGTGGTTGAAGTTACACCTAGTTACGGTACTCCCTTAGTCGT

ACTACCTGACAATCACTAGGATCGTGAACTGGACGTAATCGGATCTGTATTTGGTCCAGATGTGG 2080
TGATGGACTGTTAGTGATCCTAGCACTTGACCTGCATTAGCCTAGACATAAACCAGGTCTACACC

ATAAGCCTGGGACTTCTCAAGCCTTTCATTGCTAAAGTGAGAAAATTTGGGGATTGGTTCAAGAA 2145
TATTCGGACCCTGAAGAGTTCGGAAAGTAACGATTTCACTCTTTTAAACCCCTAACCAAGTTCTT

CACTACAGGTGAAAAGACAGATGCATGCTGACTAAACTCATAGAAAAACTGAATCACGAAAGAAA 2210
GTGATGTCCACTTTTCTGTCTACGTACGACTGATTTGAGTATCTTTTTGACTTAGTGCTTTCTTT

GAATGCAAGCAGAAAACAAACACCTAAAAGAACAAGGACTAGAAAAAATCTACACTCAAAAAGAC 2275
CTTACGTTCGTCTTTTGTTTGTGGATTTTCTTGTTCCTGATCTTTTTTAGATGTGAGTTTTTCTG

TACGAGCAGTTAAAAGAACAGCATTTGAAAGAAATTGAAGCACTCAAAAAAGAAATCCAAAAAAC 2340
ATGCTCGTCAATTTTCTTGTCGTAAACTTTCTTTAACTTCGTGAGTTTTTTCTTTAGGTTTTTTG

CAAGCAAGAAACATACACGCAACCAAAAGAATGTAGCCATTTAGCGCATTCTTTTAGCCCTAATT 2405
GTTCGTTCTTTGTATGTGCGTTGGTTTTCTTACATCGGTAAATCGCGTAAGAAAATCGGGATTAA

CATTCTTTCAATCAAAATCCGACTAATTCATCGGCTAAACGCTAAAAATCGCTTAAAACGAAAAA 2470
GTAAGAAAGTTAGTTTTAGGCTGATTAAGTAGCCGATTTGCGATTTTTAGCGAATTTTGCTTTTT

TACAAAGCAAAAAACTTCATTCCCCTTTTAGTCGTTAACCATTTAGCCAATCTAACTAGTTTAGC 2535
ATGTTTCGTTTTTTGAAGTAAGGGGAAAATCAGCAATTGGTAAATCGGTTAGATTGATCAAATCG

ATCTAAAGGCGAATCTATCTTGTGTTAGACATCCAACCTTACCAAAACCGCAGAGCGAGCTTAAG 2600
TAGATTTCCGCTTAGATAGAACACAATCTGTAGGTTGGAATGGTTTTGGCGTCTCGCTCGAATTC

AGAGATTCAAGCGGTTTTGCACGATTGTTTGCTGCCAAGAAAACCAACAAGCGAAGTAAGGCGCA 2665
TCTCTAAGTTCGCCAAAACGTGCTAACAAACGACGGTTCTTTTGGTTGTTCGCTTCATTCCGCGT

TAGACAAAAGCGCATCGCAGTTTGAAAGCGTAGGCGTCAGAAGTGGTTTGCGTTAGAATCAAACA 2730
ATCTGTTTTCGCGTAGCGTCAAACTTTCGCATCCGCAGTCTTCACCAAACGCAATCTTAGTTTGT

AGATAGCGCAAACCTGGCGTTAGGCTAAAAAACCCCTAAAAACTAAAACCCCAAAATATGTAGTGC 2796
TCTATCGCGTTTGGACCGCAATCCGATTTTTTGGGGATTTTTGATTTTGGGGTTTTATACATCACG

Plasmid pHP3 shown in single stranded form (SEQ ID NO: 3):

TCTACACAATTAACAATCTTTAGCTACAATAACAGCGTGTGAAGATGCTTTCACAGCGGT 60
ATTTCCTATGTGCGGCAAAATTTGGAGCAATTAACTTGACTTGGTTGGGTTAGTGGGTTG 120
GAGGATAGAGAGGGCGACACCTCGTTAGGAGGTATCAATGTGAAAGTATTTGTCGTATTA 180
GTTCTAGTATTAGTAATTCTCGCACAATTGCTATATTAGGCTTATTTGTGGTCTAACCCC 240

TTGTTTATGGGGGTTAGATCCTTATAAGCATACTGATACGATCACACTTATTATACACCA 300
AAAGATAAGGAGTATAGAGTGGAATTTGATCAATTAGAATCACAAAGATCAGACTTACAA 360
AAAGTGTTAAAAGAATTAGATACACTCCCAAAAACCCCACAAATTGAGCTACAAAAACAA 420
GAAATACAAAACCGCATCAACAAAATAACAGACACAATCATTAAAGAATTACTATCAAAA 480
CATGAAATCAAAAAAGAAGAACTAGAACCCACTCTAACCCCAAAACCCACACCAACAAAA 540
GAGCCACAAACCACCCCCACACCATGCAAAAATTTAGTGGTTAGCACCCCTAAAGATAAA 600
ACCTATATCACCTACCACAATAACGCTAATAAGGTCAATCTAGGGAAATTGAGCGAAAGG 660
GAAGCCAATCTTTTATTCGCTATTTTCAAAGGCTTAAAGATCAAGGGAATACCCTCATT 720
CGTTTTGAACCGCAAGATTTAAAACGCATGATCATGGTCAAATCCAACTTAACCAACAGG 780
CAATTATTGCAAGTCTTAAAAAATTTGCTTGACAACATTAGCGGTGCTAATTTTTGGATC 840
AATTAGAGAGCATGTTGAAAATGGCGAAATCTATGAAGATCACACTAGCTACATGCTTTT 900
CAAACAATTTGAAATCCGCATCCATAAGCCAACACAAACTATAGAATACTTAGATGTCCA 960
ACTCAATGATAGCTATCAATACTTGCTCAACAATCTAGGAATGGGCGGTCAATACACTTC 1020
TTTCAATCTCTTAGAATTTCAAAGGGTGAGGGGCAAATAGTGAGAGCGTTAAATTTCCCC 1080
CCCCTATTCCCCTTAAAAAGGACCCTTATCCCAGGGAATTTTTGGCCCCAATACAATTAG 1140
GGCCAAAAACCCGGTCCCTTCCATGGCTTAACCAACCCAATTGGGGGATTCCAATTTCCC 1200
CTGGATGGGAATAACCCAAGGCTTTTTTGAAAATTCCACCTACCATTTGGTCCAAAATT 1260
GGATGGACAATTCCAAATTCCAAATCTTCTTTTCCAAGAATGGGGGCCAACCCTTGACAA 1320
ACTCCTTAAACCTTTTCATTCGGCTAAAAGGTTGAAAAACATTTGGAAGATTTGGTTTAA 1380
GGAAATATTTATCGGGTGAAAAGACCAGATGCATGGCTAACTTAAACTCCATAGAAAAAC 1440
TGAATCACGAAAGAAAGAATGCTATCAAAAATGGCATTTACCACTTGATCCAAATCAAAT 1500
TTTCTTACAACTCCAATCGCATTGAAGGAAGCGGTTTGACTTATGAACAAACCGCTCATA 1560
TTTTTGACAAATCCGTTCTCATAACTGAAAAAAACACCAATATCAAACTTGATGATATTT 1620
TTGAAACTATCAATCATTTTGAATGCGTGAATTACTTGCTTGAAAGCTATAAAGAACCTT 1680
TGAGTTTAGAATACTTTAAGAATTTACACAAAATCTTGAAAAAGAATTGTTCTGATGAAG 1740
TTATTGGTGATTTTAAAAAACGCCCTAATTTTGTAGGCAATAGCGCCACAACAAGACCCA 1800
AATTAGTTGAAAGCGAATTGACAAATCTTGTGAAAAATTATCAACGCAACCTTGAAGTGA 1860
GTTTGAAAAACAATATCATGCCTTTCATCATAGAAAACGAACACAAAGCCTTTTACTACA 1920
GGGGCATCAAAGAATATGACAACACAAAAGGCTACTTGAAAGACACCATTTTGCAAAGTC 1980
AAGACAATTTCAATGAAATGGTTAGCTATTTCTTTTCTTGAGTGAAACCGCTTATTTTTG 2040
CTTGTGTGCTTTTGTTTTTTGCGTTTTTAGTTGTAGGTGGTAAGAAATATCGGTTTTTTG 2100
CTTTTCGTTGGTTGTAGGCGATTTTAGATAGCAAAAAACAGCTAAAAAATCCAAGCAACC 2160
TAATTGATTTCAAACCAACTTCATTTCCCTTTTAGTCGTTAGCCATTTAGCCAATCTAAC 2220
TAGTTTAGCATCTAAAAGCGCATATAACTTGAGTTAGCAATCCAACCAATACTAAAACCG 2280
CCTAGCGAAGCGTTAGCGAGCAAAATAAGCGGTTTTAGACCGATTGTTTGCTGACAAGCA 2340
AACACCAATAAGCGAGCGTTAGCGAGCATGGACAAAAGCGCATCGCAGTTTGAAAGCGTA 2400
GGCGTTAGCCGAAGCTGTTTTGCGTAAGCAAATCAAACAAGATAGCGCAAGCCGAGGTGC 2460

AGCCCAAGAATTTGAATTAATCCATGCGGTGTTTAGGGCGTTTTAGCGTGATCGCTTTAT 2520
TACATGTTTTAAACAGCATGCTGTTTTTACATGTTTTACTCGCATGCGCGCGCTAGG 2580
TATTGGTGGTTGGAATAGCCTAAATAACGCAGCTGTATGGTTTCTCATTTTTCGGTGACA 2640
ATGAATAAGGGGTAGTTCTTGCGAGTCATAAGTGTAGTTCTTGCGAGTCATAAGTGTAGT 2700
TCTTGCGAGTCATAAGTGTAGTTCTTGCGAGTCATAAGTGTAGTTCTCTTCACAATATCT 2760
ACACAATTCACAATCTCTAGCTACAATAACAGCGTGTGAAGATGCTTTCACAGCGGTATT 2820
TCCTATGTGCGGCAAAATTTGGAGCAATTAGCTTTAAAAGCTAGTGGGTTGGAGTTTGT 2880
AGCGGGTATGCACTCCGTTAGGAGGCACACCATGAAAGCATTTTTGATAGTAGTGATTTT 2940
AGTGGTAATCTTGACACAGCCACTATATTAAAACCTTAGCGTTTTAATAACCCTTATAAG 3000
TCCGCCAAGACTTCTTAAGGGTTTCACTCCTGTTATTATATCGTCTTTTGAAAAATAAGC 3060
ATTAAAAGGCGCTTAAATGCCCATGAATACGAATTTTGAACAGCTTAGAAAACAAGAATT 3120
GGAATTACGAAAATTATTAGAAGAATTAGAAACGCTCCCACAAACCCCACAAATTAAACT 3180
GCAAAAACAAAAAATACAAACTTACATAGACAAGATAACACCAAGTATTTTGAGCGGTTT 3240
TGATCAAAAATTCAAAGAAATTATAGAAAATCTATCAAATGAATTTGAAAAAGAAAAATC 3300
CACACCACTCAAAGAGCCACAAACCACCCCCACACCATGCAAAGATTTAGTGGTTAGCAC 3360
CCCTAAAGATAACACCTATACCACCTACCACAATAACGCTAATAAGGTCAATCTAGGGAA 3420
ATTGAGCGAAAGGAAGCCAATCT 34440

An additional nucleotide sequence that was cloned is provided at SEQ ID NO: 4, which includes a 135 bp segment that encodes a peptide of 45 amino acids (SEQ ID NO: 5). This smaller 45 amino acid peptide is an immunogenic polypeptide of the Hepatitis C virus (HCV) core antigen. The nucleic acid sequence encoding the 45 amino acid peptide is shown below with the indicated 135 nucleotides underscored (SEQ ID NO: 5).

CATGAGCACG AATCCTAAAC CTCAAAGAAA AACCAAACGT AACACCAACC
GTCGCCCACA GGACGTCAAG TTCCCGGGTG GCGGTCAGAT CGTTGGTGGA
GTTTACTTGT TGCCGCGCAG GGGCCCTAGA TTGGGTGTGC GCGCGACGAG
GAAGACTTCC GAGCGGTCGC AACCTCGAGG TAGACGTCAG CCTATCCCCA
AGGCACGTCG GCCCGAGGGC AGGACCTGGG CTCAGCCCGG GTACCCTTGG
CCCCTCTATG GCAATGAGGG TTGCGGGTGG GCGGGATGGC TCCTGTCTCC
CCGTGGCTCT CGGCCTAGCT GGGGCCCCAC AGACCCCCGG CGTAGGTCGC
GCAATTTGGG TAAGGTCATC GATACCCTTA CGTGCGGCTT CGCCGACCTC
ATGGGGTACA TACCGCTCGT CGGCGCCCCT CTTGGAGGCG CTGCCAGGGC
CCTGGCGCAT GGCGTCCGGG TTCTGGAAGA CGGCGTGAAC TATGCAACAG

GGAACCTTCC TGGTTGCTCT TTCTCTATCT TCCTTCTGGC CCTGCTCTCT
TGCCTGACTG TGCCCGCTTC AGCCTACCAA - SEQ ID NO:4.

AATCCTAAAC CTCAAAGAAA AACCAAACGT AACACCAACC GTCGCCCACA
GGACGTCAAG TTCCCGGGTG GCGGTCAGAT CGTTGGTGGA GTTTACTTGT
TGCCGCGCAG GGGCCCTAGA TTGGGTGTGC GCGCG - SEQ ID NO:5.

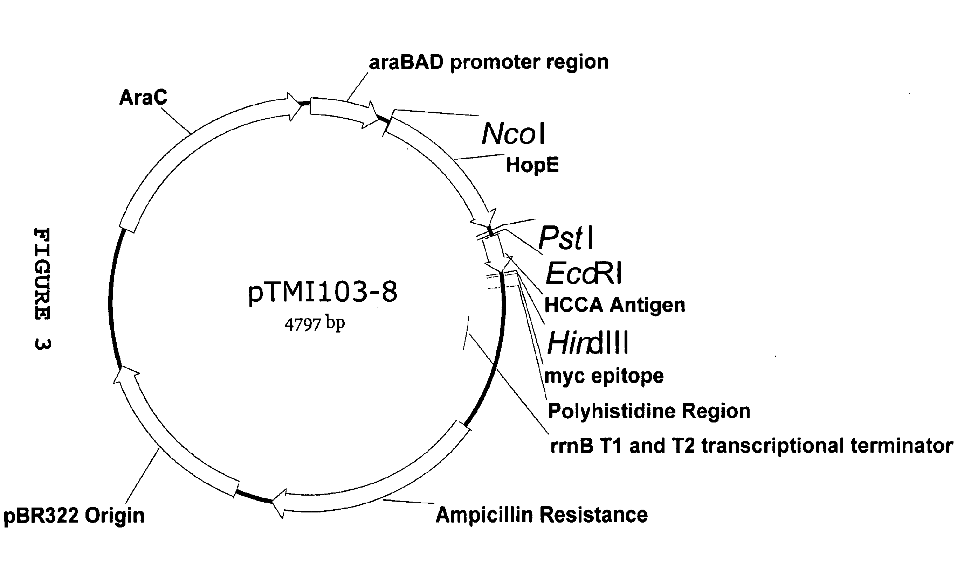

The nucleic acid of SEQ ID NO: 4 was cloned into the *hopE* gene (SEQ ID NO: 6, shown below), of *H. pylori* 26695 at nt504 of SEQ ID NO: 4 (noted in bold/underscore; corresponding to amino acid residue 168 of the protein product) so that the expressed product would be located as part of the surface exposed loop of the HopE gene product. This construct, designated as vector pTMI03-8 (Figure 3) was expressed on the surface of *E. coli.*

ATGCCATAGC ATTTTTATCC ATAAGATTAG CGGATCCTAC CTGACGCTTT
TTATCGCAAC TCTCTACTGT TTCTCCATAC CCGTTTTTTG GGCTAACAGG
AGGAATTAAC C

1 ATGGAATTTA TGAAAAAGTT TGTAGCTTTA GGGCTTCTAT CCGCAGTTTT
51 AAGCTCTTCG TTGTTAGCCG AAGGTGATGG TGTTTATATA GGGACTAATT
101 ATCAGCTTGG ACAAGCCCGT TTGAATAGTA ATATTTATAA TACAGGGGAT
151 TGCACAGGGA GTGTTGTAGG TTGCCCCCCA GGTCTTACCG CTAATAAGCA
201 TAATCCAGGA GGCACCAATA TCAATTGGCA TGCTAAATAC GCTAATGGGG
251 CTTTGAATGG TCTTGGGTTG AATGTGGGTT ATAAGAAGTT CTTCCAGTTC
301 AAGTCTTTTG ATATGACAAG CAAGTGGTTT GGTTTTAGAG TGTATGGGCT
351 TTTTGATTAT GGGCATGCCA CTTTAGGCAA GCAAGTTTAT GCACCTAATA
401 AAATCCAGTT GGATATGGTC TCTTGGGGTG TGGGGAGCGA TTTGTTAGCT
451 GATATTATTG ATAACGATAA CGCTTCTTTT GGTATTTTTG GTGGGGTCGC
501 TATCGGCGGT AACACTTGGA AAAGCTCAGC GGCAAACTAT TGGAAAGAGC
551 AAATCATTGA AGCTAAGGGT CCTGATGTTT GTACCCCTAC TTATTGTAAC
601 CCTAACGCTC CTTATAGCAC CAAAACTTCA ACCGTCGCTT TTCAGGTATG
651 GTTGAATTTT GGGGTGAGAG CCAATATTTA CAAGCATAAT GGCGTAGAGT
701 TTGGCGTGAG AGTGCCGCTA CTCATCAACA AGTTTTTGAG TGCGGGTCCT
751 AACGCTACTA ATCTTTATTA CCATTTGAAA CGGGATTATT CGCTTTATTT
801 AGGGTATAAC TACACTTTTT

CTCGAGATCT GCAGCTGGTA CGATATGGGA ATTCGAAGCT TTCTAGAACA
AAAACTCATC TCAGAAGAGG ATCTGAATAG CGCCGTCGAC CATCATCATC
ATCATTGAGT TTAACGGTCT CCAGCTTGGC TGTTTTGGCG GATGAGAGAA
GATTTTCAGC CTGATACAGA TTAAATC SEQ ID NO:6

Briefly, one method of accomplishing the isolation of *hopE* gene is amplification from *H. pylori* 22695 by using *Taq* DNA polymerase. The upstream primer 5'-AAGGATCCGATAGGAATGTAAAGGAATGG-3' (SEQ ID NO: 7) containing a *Bam*HI site and the downstream primer 5'-CCGAATTCTAAAGGCATGAACGCTTGCA-3' (SEQ ID NO: 8) containing an *Eco*RI site can be constructed by using a DNA synthesizer, such as the Perkin-Elmer Applied Biosystems, Inc. model 332 (ABI; Mississauga, Ontario, Canada). The resulting PCR fragment can be blunt-end cloned into the *Eco*RV site in pBluescript II KS(+) in the same orientation as the lac promoter.

PCR primers can then be designed to insert two unique restriction enzyme sites into the *hopE* gene for insertion of the 135bp immunogenic coding sequence from the Hepatitis C virus (HCV) core antigen. The PCR amplification using *Taq* DNA polymerase can be performed using a touchdown amplification procedure as follows. The PCR Thermocycler is programmed for an initial denaturation step of 96°C for 4 min, followed by 18 cycles at an initial annealing temperature of 65°C (for 90s), which is decreased by 0.5°C for each successive cycle, an extension step at 72°C for 6 min., and denaturation at 96°C for 1 min. Subsequent to completion of the first 18 cycles, an additional 14 amplification cycles can be performed by using 72°C extension and 96°C denaturation steps with a constant 55°C annealing temperature. The resulting amplicon is then purified by column, precipitated with ethanol, and made blunt by digestion with the Klenow fragment of DNA polymerase. The PCR products can be digested with restriction enzyme to remove the template DNA, and religated into an appropriate vector such as pTMI03.8 under the control of the arabinose inducible promoter, and transformed into *E. coli* JM105.

Recombinant clones can be identified by using oligonucleotide primer 5'-AGATCTAAGGACGTC-3' (SEQ ID NO: 9) plus the reverse sequencing primer in PCR amplification reactions. Identified clones can be sequenced to verify that the inserted restriction endonuclease sites are in frame and that no errors had been introduced into the *hopE* gene. The 135bp immunogenic coding sequence from the Hepatitis C virus (HCV) core antigen can then be inserted using standard techniques.

Once vector pTMI03-8 had been created, it was transformed into *E. coli,* which was grown at 37°C, cells were then harvested and the expression of the DCV insert was continued by Western Blot.

Briefly, a procedure for this is as follows. Cells are harvested from about 20 plates and resuspended in 20% sucrose with 50mg of DNase I (Boehringer Mannheim) in 10mM Tris-HCI (pH 8.0). The cells are then disrupted with a French press at 15,000 lb/in². Broken cells are overlaid on a sucrose step gradient of 1ml of 70% and 6ml of 70% sucrose in 10mM Tris-HCI (pH 8.0). The outer membrane fraction is collected and pelleted at 150,000 x g, and the pellet is resuspended in 100 ml of distilled water. Alternatively, outer membranes from 500 ml of log-phase culture can be solubilized in 10mM Tris-HCl (pH 8.0)-3% n-octyl-polyoxyethylene incubated at 23°C for 1 hour and centrifuged for 30mm at 173,000 x g. The pellet is resuspended in 10mM Tris-HCI - 3% n-octyl-polyoxyethylene-5mM EDTA (pH 8.0), incubated at 23°C for 1 hour, and centrifuged for 30min at 173,000 x g, and the supernatant collected. A Western immunoblot indicated the presence of HCV/hopE in the supernatant of the second solubilization step. The supernatant containing HCV/hopE is mixed with an equal volume of 0.125 M Tris-HCI (pH 6.8), 4% (wt/vol) sodium dodecyl sulfate (SDS), and 20% (vol/vol) glycerol and subjected to SDS-12% polyacrylamide gel electrophoresis (PAGE). If required the HCV/*hopE* band can be excised from an unstained portion of the gel and eluted overnight at 4°C into 10mM Tris-HCl (pH 8.0), 1mM EDTA (pH 8.0), and 100mM NaCl. The elution supernatant can then be run on an SDS-PAGE gel to check for purity and a Western immunoblot using standard techniques undertaken. For example, isolated outer membranes can be loaded at a concentration of 15 µg/lane. Electrophoresis is then carried out by SDS-PAGE on a discontinuous 12% polyacrylamide gel. Proteins are then stained with Coomassie brilliant blue. For Western immunoblotting, unstained gels can be electroblotted onto immobilon-P membranes (Millipore, Bedford, Mass.). After blocking for 2 h at 2°C with 3% bovine serum albumin (BSA; Boehringer Mannheim)-0.1% Tween 20 (Sigma) in phosphate-buffered saline (PBS), the membranes are then incubated with a 1/10,000 dilution of anti-HCV rabbit antiserum in 1% BSA-0.05% Tween 20 in PBS for 1 h at 37°C. The membranes are then washed with PBS and incubated with a 1/5,000 dilution of an alkaline phosphatase-conjugated secondary antibody (Bio-Rad, Richmond, California) for 1 h at 37°C. The bound antibodies are detected with 5-bromo-4-chloro-3-indolylphosphate (BCIP, Calbiochem. La Jolla, California) and nitroblue tetrazolium (NBT, Sigma).

### EXAMPLE 2, NOT PART OF THE INVENTION, EXPRESSION VECTORS AND SELECTION OF ANTIGENS FOR STABLE EXPRESSION

Because HopE is a native protein of *H. pylori,* it is tolerated by this organism and can thus form a construct useful for expressing foreign antigens or other heterologous gene products in *H. pylori.* Other *H*. *pylori*/*E. coli* shuttle vectors that can be readily developed as described above include, for example, vectors comprising two plasmid ori sites and markers which are suitable for each host. Markers might include genes for chloramphenicol/kanamycin resistance as well as promoters that can be recognized by both the *E. coli* and *H. pylori* transcriptional systems.

The requirements for replication *E. coli* can be achieved by using any of a number of known *E. coli* plasmids (e.g., pBR322).

Constructed shuttle vectors can be tested for replication in both *E. coli* and *H. pylori* in vitro and compared to existing shuttle plasmids described in the literature. The choice of antigen or other heterologous gene product to be expressed would ideally be one that is not toxic to *H. pylori* and *E. coli* and that is highly immunogenic (or possesses another desirable property) when delivered to a selected site in a mammal. In the case of an expressed antigen for immunization of the mammal, such as site may be a mucosal site.

As described in Example 1, *hopE*/HCV core antigen fusion protein can be expressed at the *E.* coli surface. The product of pTMI03-S (Figure 3) is preferably targeted to the *H. pylori* outer membrane, so that it would display the HCV core antigen in a mucosal environment.

Tetanus toxoid (TT) has been studied extensively as an antigen in humans, and immune responses to it are well characterized. TT elicits good mucosal immune responses when administered orally or intranasal when displayed on the surface of bacterial spores (Duc & Cutting, 2003, Expert Opinion Biol Ther, 3(8), 1263-70). The tetanus toxin C fragment can be fused to the hopE gene product as described above or engineered to contain a membrane anchor and cell surface target sequence. The advantage of this system is the existence of well-characterized murine models for assessment of the effectiveness of the vaccination procedure, whereas; in the case of HCV where the known murine models typically rely on immune-deficient mice. The gB protein of cytomegalovirus (CMV) has been shown to immunize mice against a lethal dose of an engineered vaccine virus expressing the gB antigen. Accordingly, shuttle vectors such as those described herein, comprising gB antigen in place of an HCV antigen can be constructed using the protocol described in Example 1.

A number of promoters can be used in the shuttle vectors. Ideally the promoter used is inducible either by a natural in vivo colonization mechanism of *Helicobacter* or by induction with an innocuous foodstuff or chemical that can be consumed. For example, the promoter from the *H. pylori* histidine kinase HP 165 may be used. The promoter from the *H. pylori* histidine kinase HP 165 is reported to be induced by acidic pH and may be a virulence factor related to gastric mucosa colonization. The benefit of this promoter is that a construct can be made in vitro. The foreign antigen will only be expressed when exposed to the acidic environment of the gastric mucosa.

Other promoters include the arabinose inducible promoter used in pTMI03.S and FlaB sigma 54 promoters (Josenhans et al., 1998, EEMS Microbial Lett., 16 1(2), 263-73), the T7 promoter used in constitutive and inducible *E. coli* systems and the nir promoter of *Salmonella* which is induced in anaerobic environments (Chatfield et al., 1992, Biotechnology, 10(8): 888-92). The ability of any of these promoters to function in *H. pylori* can be tested using the system developed by Angelini et al. (2004) (Plasmid, 51:101-107) that uses CAT and GFP reporters as readout of promoter activity in a *H. pylori* plasmid vector.

The target sites of expression will depend on the antigen or other gene product used. Initial studies focused on HopE proteins and fusion polypeptides which target the expressed polypeptide *(e.g.,* antigen) to the cell surface of *H. pylori .*

Plasmid stability is also very important and, while the use of antibiotic resistance genes as selective determinants for plasmid maintenance is useful *in vitro,* is less practical *in vivo.* An alternative is a balanced-lethal system, for example, the *asd* gene that is used inactivated in *Salmonella.* The asd gene, which exists natively in *H*. *pylori,* encodes aspartate-β-semialdehyde dehydrogenase (an enzyme in biosynthetic pathway for diaminopimelic acid (DAP), an essential component of the cell wall peptidoglycan of gram-negative bacteria. In the absence of DAP, asd mutants undergo lysis. Since DAP is not present in mammalian tissues, this balanced-lethal system imposes a requirement that all living *H. pylori* carry the recombinant asd gene-containing plasmid.

In order use the asd gene system the genomic copy of the asd gene is inactivated using standard gene knockout protocols. This strain of *H*. *pylori* will then only grow with the supply of DAP or with a plasmid that contains the asd gene.

Other systems that can be used for similar purposes include *E. coli* enterotoxin or cholera toxin (CT) as mucosal adjuvants. Adjuvants can also be used to boost the mucosal immune response. Two such adjuvants are CT and *E.* coli enterotoxin (LT) wherein expressed antigens are fused to the LTB and CTB mutants that maintain their strong mucosal adjuvant properties but have reduced toxicity.

### EXAMPLE 3, NOT PART OF THE INVENTION VIRULENCE , LD₅₀

As described in Examples 1 and 2, *Helicobacter*-based vectors such as pHP3 and pHP623 are capable of providing protection against infection in a mammal, such as a mouse or human. In the present example, a murine model is used to demonstrate the utility of using the *Helicobacter*-based systems to provide delivery of a pharmacologically active molecule of interest to a mammal, including a human. The murine model is employed to demonstrate the activity of a transgenic strain of *H. pylori* to elicit a serological response to an expressed surface antigen *in vivo.*

Mice are infected with wild-type *H. pylori,* while other mice are inoculated by gavage with temperature-sensitive *H. pylori* as described in Example 2. Sera from both control and test animals are assayed for antibody and gastric histology are performed on sacrificed animals in accordance with the schedule shown in Table 1. A mouse urea breath test can also be used.

A 50% decrease in virulence (from 75% to 40%) was observed. Specific antibody titer increased 4 fold above baseline, indicating a serological response. Serum samples were taken at baseline, 12, 24 and 48 weeks. At these times 10, 10 and 20 animals were sacrificed and gastric histology performed.

### EXAMPLE 4, NOT PART OF THE INVENTION COMPARISON OF VIRULENCE AND ANTIGENICITY OF TEMPERATURE SENSITIVE H. pylori STRAINS

In order detect change in virulence related to expression/modification of an outer membrane protein; mice were inoculated with temperature-sensitive *H. pylori* as described in Example 3. An equal size control group of mice were infected with a wild type *H. pylori* strain. Noninvasive means were used to determine presence or absence of *H*. *pylori.* Mice were bled at 3 and 6 months for antibody determination. At sacrifice, histology was performed to assay gastritis and confirm colonization.

### EXAMPLE 5, NOT PART OF THE INVENTION, LD₅₀ STUDY TO EVALUATE TEMPERATURE-SENSITIVE H. pylori VACCINE EFFICACY FOR A PNEUMOCOCCAL ANTIGEN

In order to demonstrate the *Helicobacter-*based vaccine protection effect from a standard pathogen *(pneumococcus),* mice were inoculated with temperature sensitive *H. pylori* by gavage. An equal sized control group was infected with the wild type *H. pylori* strain. Non-invasive means were used to determine presence or absence of *H. pylori* as described in Example 4. At 6 months post infection, all mice were given intraperitoneal challenge with 10 times the LD₅₀ of live virulent pneumococci type 4 (-20 CFU/mouse), as per the method of Aaberge et al. (1995, Microb. Pathog., 18:141-152).

Allowing for 75% lethality in the controls, the study has a power of 0.8 to detect a 50% decrease in mortality (75% vs 50%).

### EXAMPLE 6, NOT PART OF THE INVENTION, DETERMINATION OF H. pylori STATUS OF MICE: BREATH TEST METHOD

In the present example, the urea breath test used in humans was adapted for use in mice.

Ten mice were fed a diet devoid of urease (uncooked soy). Mice were then administered 3.7 kBq ^{[14]} C urea in 200µl flavored citrate by gavage and placed in air-filled 2L plastic Ziploc bags for 20 minutes. Mice were then removed without exchanging the air within the bag. Hyamine, 0.1 mmol in ethanol, was then introduced and scintillant was added to the hyamine solution and counted for 10 min or up to a count of 1,000 dpm.

### EXAMPLE 7, NOT PART OF THE INVENTION, HUMAN STUDIES

To confirm virulence and antibody response in humans, a strain of *H. pylori* like the "Baylor Strain" will be employed, and the following criteria will be adopted:
1. The infected individuals will have no symptoms, no more than mild histological damage, and no evidence of infection with hepatitis viruses or HIV.
2. The isolate will be a single strain, cagA negative, and sensitive to metronidazole, clarithromycin, tetracycline, and amoxicillin.
3. Volunteers to receive a challenge will be healthy with normal gastric histology, no history of peptic ulcer, no young children at home, no regular contact with young children, and no allergies to the antibiotics that might be required to treat the infection.

Challenge will consist of 40 mg famotidine at bedtime followed by administration of *H. pylori* in beef broth orally in the morning. Subjects are contacted daily for 14 days. A 13c-UBT is performed after 7 and 14 days and endoscopy with quantitative culture and histology after 2 weeks and 3 months. Antibiotics are used to eradicate the infection.

### EXAMPLE 8, NOT PART OF THE INVENTION, DEVELOPMENT OF EXTERNAL CHEMICAL MARKER FOR DETECTION OF WILD TYPE AND/OR TSHP In Vivo

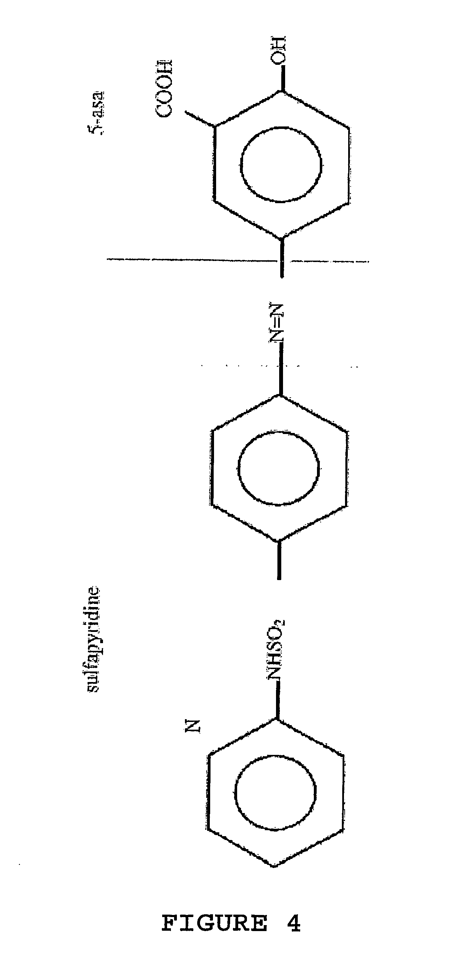

An example of a chemical marker that may be used for the detection of wild type or TSHP in *vivo* is sulfasalazine (SSN), the structure of which is shown in Figure 4. Studies in germ free mice and conventional rats have shown that intestinal bacteria are solely responsible for the diazo-bond reduction, resulting in the reductive catabolism of SSN and the release of sulfapyridine and 5-aminosalicylate. The enzyme(s) which catalyses this reaction is referred to as diazoreductase(s) (synonym azoreductase(s)). Conventional rats given SSN excrete 5-aminosalicylate and sulfapyridine (and their respective conjugates) in urine and feces, whereas germ-free rats show no evidence of SSN degradation.

Several bacterial species have been shown to have diazoreductases (AZR's). Preliminary bioinformatic studies have indicated that *H. pylori* may not contain the AZR gene. The presence of similar analogous sequences has also produced a negative result. Under these circumstances a transgenic strain of *H. pylori* (TSHP) that has a viable and functional azoreductase (azr+ TSHP) can be used to assess the use of these markers.

Plasmid pTM103-02 is digested by *Eco*RI and *Hind*III, and ligated with the Azoreductase (AZR) gene from *Bacillus subtilis* treated with *Eco*RI and *Hind*III to generate a vector containing both *Hop*E 168aa and AZR named pTMI03-azr. This plasmid is transformed into *E. coli* to assess whether expression of *HopE* and the *B. subtilis* AZR occurs. pTMI02 when similarly treated with full-length hepatitis C core antigen (HCCA) demonstrated transport of *HopE*::HCCA to *E. coli* outer membrane employing western blots and anti-*HopE* Abs.

Mice (n =30) are infected with the azr + TSHP by gavage and once AZR expression in vivo to produce 5-aminosalicylate and sulfapyridine (and their respective conjugates) in urine and feces is established, human trials can begin.

### EXAMPLE 9, NOT PART OF THE INVENTION, USE OF DIAGNEX BLUE AS A MARKER

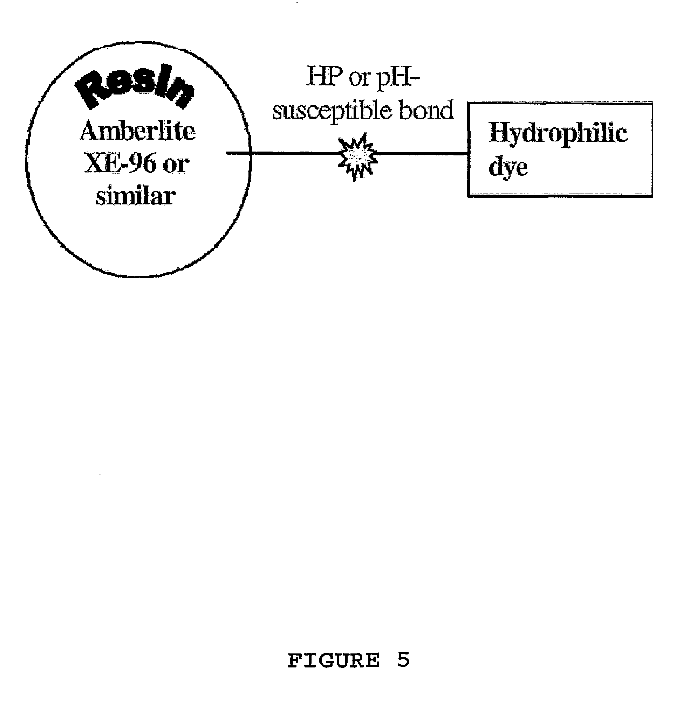

The diagnostic agent "diagnex blue" consists of an ion exchange resin (Amberlite XE-96) conjugated with a dye (Azure-A). This test relies on the fact that the resin-dye combination disassociates at pH less than 2.5 after which the dye is absorbed and appears in the urine. Persons without dye in the urine are achlorhydric. This principle is shown in Figure 5.

The same principle can be used to test for *H. pylori.* For example, a dye-resin combination that disassociates at pH >7.0 could detect urease if the resin was given with urea. This would produce a pH > 7.0 in the mucosal layer where *H. pylori* resides, thus releasing the dye.

Mice (n =30) are inoculated with a wild type *H. pylori* strain while germ-free mice (n =30) are used as controls (Pilot study). After an optimal period allowing for the *H. pylori* to establish an active infection, the test group and the controls are introduced with a predetermined quantity of the resin-dye complex by gavage. This will be followed by a urea solution. (Range 0.01M to 0.5M). The mice are kept in metabolic cages and the excretion of the azure dye are monitored and quantified. Different ratios of the resin and urea concentrations are tested to verify the optimal combinations to be used.

### EXAMPLE 10 DELIVERY FORMULATIONS

For purposes of delivery forms, the present example is provided to demonstrate the utility of the present invention as providing an aerosol spray preparation. In some of these embodiments, the aerosol spray may take the form of pressurized packs or a nebulizer, with the use of a suitable propellant. In the case of a pressurized aerosol, the dosage unit can be determined by providing a valve to deliver a metered amount. The formulation would be prepared as a powder for administration by inhalation. Administration by inhalation can also be carried out by atomizing solutions or suspensions which contain the compositions according to the invention.

The compositions according to the invention may also be formulated in a liquid for oral digestion for administration to a subject as an intravenous preparation.

All of the various preparations of the invention may be prepared by procedures familiar to those skilled in the art, if appropriate, using further suitable pharmaceutical auxiliaries. Compositions according to the invention advantageously contain the species of *Helicobacter,* alone or in combination with other desired ingredients.

Any of the above individual or combination of *Helicobacter* formulations may be included in a pharmaceutical composition comprising the pharmaceutically acceptable composition according to the present invention. The pharmaceutical compositions as described herein may be in solid (*e.g.* powder, particles, granules, sachets, tablets, capsules etc.), semi-solid (gels, pastes etc.) or liquid (solutions, dispersions, suspensions, emulsions, mixtures etc) form and adapted for administration via *e.g.* the gastrointestinal tract and gastric mucosa. The pharmaceutical compositions may thus be in powder or particulate form adapted to be dispersed in an aqueous medium before use.

A pharmaceutical composition in liquid form may be in the form of a dispersion comprising the *Helicobacter* composition and an electrolyte solution such as, *e.g.* a composition that is adapted to physiological conditions *e.g.* a physiologically acceptable solution.

A pharmaceutical composition according to the invention may further comprise another therapeutically, prophylactically and/or diagnostically active substance.

In another aspect, the invention relates to a pharmaceutical kit comprising a first and a second container, the first container comprising a recombinant *Helicobacter* composition comprising the plasmid and/or plasmid vector according to the invention and the second container comprising a dispersion medium for the *Helicobacter* composition, accompanied by instructions for administering and/or dosing the *Helicobacter* composition in the dispersion medium before use.

The *Helicobacter* composition according to the present invention contained in the kit may be in powder or particulate form.

A pharmaceutical kit according to the present invention may include instructions with recommendations for the time period during which the *Helicobacter* composition should be administered after dispersion in the dispersion medium.

### EXAMPLE 11 IMMUNE MODULATION WITH HELICOBACTER - VACCINE PREPARATION

The TH1 response (T-helper cell type 1) is a cell mediated response. Over activity of this is a presumed cause of diseases such as rheumatoid arthritis (RA) and Lupus. In contrast, TH-2 is an antibody type serological response characteristic of vaccines. The present example demonstrates the utility of the present invention for providing a technique for achieving a TH-2 type response in an animal when treated with a *Helicobacter*-based vaccine treatment preparation according to the present invention.

Although *Helicobacter pylori* produces an antibody response (TH2), it has been noted recently that the main response in the mucosa of the stomach may be a TH1 cell mediated response. Therefore, it is envisioned that the invention may provide for both types of immune response when provided to the mucosa of an animal.

Use of the *Helicobacter* vectors and vector plasmid systems as described herein may be used to invoke antibody response in an animal. By way of example, a system employing a gene expression cassette in a construct that provided for the transformation of the bacterium, *Clostridium,* and the subsequent secretion of a protein (S-layer protein) from the surface of the transformed *Clostridium,* this resulting in initiation of mucosal vaccination, is described in WO-0194599. These constructs may also include a secretory leader sequence selected from ORF1, ORF3, ORF5-7, ORF7 or ORF11.

In accordance with some embodiments of the vaccine, the *Helicobacter*-based vectors and vector plasmids may comprise a sequence encoding a bacterial surface layer protein. A surface layer protein is defined herein as any molecule of proteinaceous nature, including e.g., protein, glycol-protein or lipoprotein occurring in the outer membrane of a bacterium and capable of being exposed on the surface of the bacterium. S-layer proteins may be continuously and spontaneously produced in larger amounts than any other class of protein in the cell.

A process for preparation of a recombinant cell preparation comprising a gram negative host cell, *Clostridium,* having the S-layer protein, is also provided in WO-97/28263. The process may be modified and followed in accord with the procedures described herein to incorporate an S-protein as part of the *Helicobacter* constructs of the present invention.

Accordingly, in some of the vector and vector plasmid constructs, a fusion protein is provided that comprises a *Helicobacter* sequence and a non-*Helicobacter* pharmacologically active molecule of interest. In order to enhance the immunogenicity of a vaccine employing the *Helicobacter* constructs of the present invention, the *Helicobacter* sequence of the fusion protein may comprise a sequence encoding an S-layer protein. *Bacillus* constructs that include the S-layer protein as part of a fusion protein have been reported to express the S-layer protein at the *Bacillus* surface. (See WO-95/19371, describing *Bacillus sphaericus),* thus enhancing the immunogenicity of the preparation.

Mucosal immunization is already provided against some diseases, including an oral polio vaccine and an oral (drinkable) vaccine against cholera and diarrhea due to E. *coli* (an inactivated vaccine). In some embodiments, it is contemplated that the vaccines of the invention may thus comprise an inactivated vaccine.

The present invention contemplates a live vaccine, as such will provide a single-dose, long lasting vaccination, because the carrier organism, *Helicobacter,* will continue to produce the antigen, *i.e., non-Helicobacter* pharmacologically active molecule of interest, and boost immunity in vivo. In addition, the vaccines will be administered in combination with an adjuvant. These adjuvants' comprise molecules such as aluminum hydroxide or lipid vesicles that increase the exposure time for the vaccine by slowing its removal forte site of administration. Adjuvants' also act by evoking production of immunomodulatory peptides called cytokines and chemokines (Brewer et al. 1997, J. Cytokines Cell Mol. Ther., 4:223-246). Thus, the present vaccines may comprise cytokine adjuvant to enhance immune response. The transformed *Helicobacter* or *E. coli* bacterium, when administered orally or gastrically to a mammal such as a human or animal, will provide for the gastro-intestinal colonization, production and presentation of the desired polypeptide, through the gastric wall, which is the natural site of colonization. The gastro-intestinal tract is surrounded by an immense immune apparatus specialized in mounting immune response of various types. Gastro-intestinal colonization by recombinant *Helicobacter* vaccine or peptide producer strain thus enables a much longer immune stimulus than traditional vaccination. Additionally, antigen can be presented preferentially to the gut wall or the lumen.

### EXAMPLE 12 HELICOBACTER AND USES THEREOF AS AN APPETITE SUPPRESSANT

The present example is provided to demonstrate the utility of the present invention as a method for employing *Helicobacter* in preparations and treatment regimens that provide for appetite suppression. In particular, delivery to the gut mucosa of a construct that comprises attenuated *Helicobacter* together with a non-*Helicobacter* pharmacologically active molecule of interest that regulates the level of ghrelin or an agonist of ghrelin, is expected to provide an effective means for providing suppression of the gut-brain axis that regulates appetite and satiety.

Studies have suggested that ghrelin is an appetite stimulant, i.e., ghrelin increases food intake in mice (Asakawa et al. 2003, Gut, 52(7):947-52). Ghrelin has also been reported to reduce fat utilization in adipose tissue in rodents (Tschop et al., 2000, Nature, 407: 708-13), as well as to be involved in rat adipogenesis (Choi et al. (2003), Endocrinology, 144 (3):751-9). Ghrelin has also been reported to be a hunger signal, prompting the subject to eat when nutrition availability is low.

The teachings of United States patent #6,967,237 - Bednarek (2005), relating to ghrelin analogs and nucleic acid constructs, and of United States Patent application publication 20050191317 - Bachmann et al. (2005), relating to ghrelin-carrier conjugates, supplement and/or further enhance the understanding and appreciation of the present invention.

Ghrelin, an endogenous ligand for the growth hormone secretagogue receptor (GHS-R), stimulates growth hormone (GH) release from cultured pituitary cells in a dose-dependent manner, and is produced and secreted from the Alike cells found mainly in the oxyntic glands of the gastric fundus. Ghrelin is now known to play a role in not only GH release, but also in controlling the appetite and body weight.

Both parenterally and intracerebro-ventricularly administered ghrelin have been shown to stimulate food intake and increase the body weight of mice and rats with free access to food, even those animals with GH deficiency. The control of appetite and body weight may be independent of GH release.

Ghrelin, a 28-amino-acid peptide, is activated when its third serine residue is acylated by n-octanoic acid, and GHS-R is responsive to the first four or five residues including the octanylated serine residue of the whole ghrelin peptide. GHS-R has been shown to be present in the pituitary, hypothalamus, adrenal glands, thyroid, pancreas, myocardium, spleen and testes. Ghrelin stimulates the expression of both NPY and AGRP mRNA in the hypothalamus. The central orexigenic effect of ghrelin is mediated by the NPY/AGRP-expressing neurons in the hypothalamus. Ghrelin has also been reported to suppress vagal afferent activity. The peripheral orexigenic effect of ghrelin may be mediated, at least in part, by its suppressive effect on the vagal afferent activity. IL-1β is a pro-inflammatory cytokine that mediates the cachectic process by stimulating the expression and release of leptin, and/or by mimicking the effect on the hypothalamus of excessive negative-feedback signaling from leptin.

It is proposed that antagonists to ghrelin if provided to the animal at the gut mucosa will reduce food intake by an animal and reduce body weight gain.

### EXAMPLE 13 CELL WASTING ATTENDANT CANCER AND AIDS

The present example demonstrates the utility of the present invention for use as a preparation that will prevent or inhibit cell wasting, particularly cell wasting associated with diseased states of AIDS and cancer.

Cachexia is a condition characterized by wasting, emaciation, feebleness and inanition. It was recently reported that the levels of both ghrelin peptide and ghrelin mRNA in the stomach were up-regulated in a mouse model of cancer cachexia. In cachectic mice with increased plasma levels of IL-1β, the plasma concentrations of ghrelin also increased with the progression of cachexia. This result suggests that a close relationship might exist between the ghrelin dynamics and the cachectic process mediated by IL-1. IL-1β is an anorexigenic substance, just like CCK, leptin, gastrin-related protein and bombesin, and antagonizes the actions of ghrelin.

Asakawa et *al.* reported that parenterally administered IL-1β decreased NPY mRNA expression in the hypothalamus and preproghrelin mRNA expression in the stomach, and that intraperitoneally administered ghrelin inhibited the severity of IL-1β-induced anorexia.

*Helicobacter pylori* infection is known to be a major pathogenetic factor in the development of gastritis, peptic ulcer disease and gastric malignancy. Attachment of *H. pylori* to the gastric mucosa induces inflammation, which is associated with the release of various cytokines, including IL-1β.

It has been observed clinically that *H. pylori* eradication is often followed by improvement of some nutritional parameters, such as the body weight and the serum levels of total cholesterol, total protein and albumin. *H. pylori* infection has been reported to be capable of modifying the plasma and gastric ghrelin dynamics in Mongolian gerbils. In humans, however, *H. pylori* infection has been reported not to be associated with any changes of the plasma ghrelin levels, although eradication of *H. pylori* has been shown by some to be associated with increases of the plasma ghrelin levels.

It is proposed that *H. pylori* may be used as a carrier to provide amylin to a patient in need thereof, by, for example, acting as a carrier vehicle, to the gastric mucosa. In some embodiments, the *Helicobacter* carrier will be constructed so as to include amylin, amylin agonist, analogs, and derivatives, and amylin agonists (including calcitonins, calcitonin gene-related peptides), and analogs therefore to decrease ghrelin levels.

Amylin antagonists can increase ghrelin levels. Modulation of the effective levels of amylin, with amylin, amylin agonists, amylin antagonists, or other compounds that decrease the effective level of amylin such as antibodies, may inhibit, or stimulate in the case of antagonists and antibodies, ghrelin secretion. Hence, some embodiments of the method are directed to modulating endogenous levels of ghrelin by increasing the effective level of amylin or amylin agonists in the body, by direct or indirect means, or by decreasing the effective level of amylin using amylin antagonists or inhibiting amylin production.

### EXAMPLE 14 TREATMENT OF GAUCHERS DISEASE

The present example demonstrates the utility of the invention for use as a treatment for a disease resulting from an enzyme deficiency, such as Gaucher's disease. Gaucher's disease is the most common lysosomal storage disorder in humans, and results from a deficiency of the enzyme, glucocerebrosidae (GC). (Nolta et al., (1992), J. Clin. Invest. 90 (2) :342-348) .

Enzyme replacement therapy is provided with a *Helicobacter* vaccine construct that comprises a sequence encoding chemical chaperones. (Sawker et al., (2002), PNAS USA 99(24): 15428-15433). An enhanced level of functional β-glycosidase (β-Glu, glucocerebrosidase) may thus be obtained. In particular, the chemical chaperone deoxynojirimycin (NN-DNJ) is to be used in the *H. pylori* construct and administered to the patient orally or intragastrically.

As part of yet another embodiment of the methods, a *Helicobacter-*based construct as described herein comprising a vector having a non-*Helicobacter* pharmacologically active molecule of interest, in this case, encoding glucocerebrosidase (GC). Retroviral mediated transfer of glucocerebrosidase cultured Gaucher bone marrow is described as one approach for treating Gauchers disease in Nolta *et al.* (1992). However, this approach is extremely invasive. Alternative enzyme replacement therapy employing the *Helicobacter*-based constructs of the invention that include a sequence encoding for the deficient enzyme, glucocerebrosidase, provides a much more attractive and less expensive alternative to such a therapy.

### EXAMPLE 15 TREATMENT OF LYMPHOMA

The present example is presented to demonstrate the utility of the present invention to provide a useful preparation that is suitable for treating and/or inhibiting a bacterial induced malignancy, such as lymphoma, particularly MALT lymphoma, using a vaccination preparation comprising the *Helicobacter* vector and/or plasmid vectors as described herein.

Sutton et al. (2004) (Vaccine, 22 (20): 2541-6) report protection against a bacteria-induced malignancy, specifically primary gastric MALT lymphoma, as a result of vaccination/immunization of an animal against *Helicobacter felis.*

*Helicobacter pylori* constructs of the present disclosure that include a vector and/or plasmid vector suitable for providing an immunizing preparation that includes an immunogenic antigen of interest other than *Helicobacter felis,* may also be used to provide vaccination protection against a bacterial-induced malignancy, and in particular, against primary gastric MALT lymphoma. By way of example, some embodiments of the plasmid vector would include a fusion protein comprising a *Helicobacter pylori* encoding sequence and a non-*Helicobacter pylori* encoding sequence that is, for example, other than a *Helicobacter felis* antigen species.

### EXAMPLE 16, NOT PART OF THE INVENTION, LIVE VACCINE DELIVERY SYSTEM WITH H. pylori

The present example demonstrates the utility of using *H*. *pylori* in a live vaccine, and in particular the use of *H. pylori* as part of a live viral preparation to deliver a protein of interest to the gastric mucosa of an animal.

In particular, the present example demonstrates the utility of the present invention for providing delivery of an antigen of interest to an animal at the gastric mucosa though the outer surface of a recombinant *Helicobacter pylori* outer membrane.

The present studies were primarily done with *H. pylori* strain B128, variant 7.13 (Franco et *al.* (2005). The *sacB* cassette, conferring sucrose sensitivity and kanamycin resistance, was inserted into the hopE gene of *H. pylori.*

*H. pylori* is demonstrated herein to be a useful vehicle for vaccine delivery, and provides an improved bacterial delivery modality for the treatment of an animal, particularly a human. Factors important in establishing the utility of the *H. pylori* based vaccines in the treatment of humans include the following:
1. A majority of persons infected with *H. pylori* are asymptomatic;
2. infection with *H. pylori* induces both adaptive and innate immune responses;
3. Infection with *H. pylori* can persist in the gastric mucosa, facilitating long-term exposure to antigens;
4. Infection with *H. pylori* produces molecules that disrupt the gastric epithelium, thereby facilitating exposure of bacteria to the submucosal immune system;
5. Genome data and molecular techniques are readily available for *H. pylori,* thereby facilitating its genetic manipulation.

HopE is an outer membrane protein of *H. pylori.* The present example demonstrates that the nucleic acid sequence encoding this protein can be modified so as to include a desired sequence encoding a molecule of interest ("X"), and *H. pylori* containing this modified HopE - sequence may then be used in a vaccine to deliver the molecule of interest ("X") to the mucosal surface of an animal.

In the present example, the p60 protein and the HCCA protein are used to demonstrate proof of principle of delivery, and are simple examples of proteins that may be used to provide such an engineered *H. pylori* HopE gene.

### Methods:

### Bacterial Strains and Culture

*Helicobacter pylori* strain B128 (7.13) (52) or 26695 (64) were cultured on Columbia agar (Oxoid, Basingstoke, United Kingdom) supplemented with 5% horse blood and incubated under microaerophilic conditions (10% CO2) at 37°C. *H. pylori* were transformed by natural transformation (65,66). Transformants were selected using chloramphenicol (10 µg/mL) or kanamycin (10 µg/mL). *Escherichia coli* strain DH5-α (67) was grown in LuriaBertani (LB) medium at 37°C and transformed by electroporation (68). Transformants were selected using 5% (w/v) sucrose, kanamycin (50 ug/mL) or ampicillin (100 ug/mL). Genomic DNA was extracted using the DNeasy Tissue Kit (Quiagen). Plasmid extractions were performed using the QlAprep Spin Miniprep Kit (Quiagen).

### Construction of Recombinant DNA Molecules

Recombinant DNA molecules were constructed by assembling individual PCR products using splicing by overlap extension (SOE) PCR (69). Primers were designed to introduce overlaps of 12bp at the termini of PCR products, allowing the products to be joined together during PCR (Figure 8). All preparative amplifications were performed using Pwo polymerase (Roche Pharmaceuticals).

Recombinant DNA was constructed to insert a chloramphenicol resistance gene into hopE. A region including a section of, and extending upstream, of hopE was amplified using the primers HopEF3 and HopER3 (Product A). The *opa* promoter and chloramphenicol resistance gene were amplified from pHe12 (70) using primers CATF and CATR (Product B). A region including a section of, and extending downstream of *hop*E was amplified using the primers HopEF4 and HopER5. Products A, B, and C were joined together using primers HopEF3 and HopER5 as described by Chalker and associates (69).

Allelic replacements were achieved using the sacB based selection system (71). The *sacB* cassette, conferring sucrose sensitivity and kanamycin resistance, was inserted into the gene of interest. Recombinant DNA was transferred into sucrose-sensitive *H. pylori.* Transformants were sucrose resistant, resulting from replacement of *sacB* with transforming DNA.

The sacB cassette was joined to *hopE* DNA in a number of stages. Firstly, the *hop*E gene was amplified using the primers HopEF6 and HopER6. Purified amplicon was digested Ncol and *Bgl*ll (Roche Pharmaceuticals). Amplicon and similarly digested pBADMyc-HisB (Invitrogen Life Technologies) were ligated using the Quick-stick Ligation Kit (Bioline) and transferred to *E.coli,* producing plasmid pBADl. Secondly, a region of DNA, including 804 bp of the intergenic region between *hop*E and the downstream putative mraW gene (coding 5 - *S*-adenosylmethyltransferase), was amplified using primers HopEF7 and HopER7. Purified amplicon was digested with *Bgl*ll and Xball, ligated to similarly digested pBADl, and transferred to *E. coli* to produce plasmid pBAD2. Lastly the sacB cassette was amplified from pENKSF (59) using the primers KanSacF and KanSacR. The amplicon and pBAD2 were digested with *Bgl*ll, ligated and transferred to *E.coli* to produce plasmid pBAD3.

Similarly the sacB cassette was inserted into *cag*A and *vac*A. For *cag*A, in the first stage the region directly downstream of cagA was amplified using primers cagAF2 and cagAR2 and digested with Xbal and *Bgl*ll. In the second stage a section of *cag*A extending to the 3' end was amplified using primers cagAF1 and cagAR1 and digested with *Bgl*ll and Ncol. In the final stage, the sacB cassette was excised from pBAD3 using *BgI*ll, and inserted into similarly digested pBADMyc-HisB. For *vac*A, in the first stage a section including the C-terminal region of *vac*A was amplified using primers vacAF4 and vacAR4, and digested with *Hind*lll and Xbal. The fragment was cloned into similarly digested pUC19 to produce pUC1. Secondly, a section of DNA including the N-terminal region of *vac*A was amplified using primers vacAF3 and vacAR3, and digested with *Eco*Rl and *Kpn*l. The fragment was cloned into similarly digested pUC1 to produce pUC2. Lastly, the sacB cassette was amplified using the primers KanSacF and KanSacR, and digested with *BgI*ll to produce pUC3.

Recombinant molecules were constructed to insert DNA coding antigenic sections of the Hepatitis C virus core antigen (HCCA) or *L. monocytogenes* p60 protein into hopE, replacing the sacB cassette. Recombinant DNA was produced using SOE PCR, joining PCR products A, B and *C.* A region extending from the start of *hopE* to nt 573 was amplified using primers HopEF8 and HopER1 (product A). The region of sequence coding HCCA antigen from aa 7-53 was produced by amplifying primers HCCAF1, HCV.2a, HCV3.s, HCCAR1 (product B). The region downstream of *hop*E nt 573 was amplified using HopEF2 and HopER8 (product C). Product resulting form SOE PCR was cloned into plasmid pCR4Blunt-TOPO (Invitrogen Life Technologies) to produce pCR4HCH.

Similarly DNA coding sections of the p60 antigen was inserted into *hop*E and *cag*A. For *hop*E, the region extending from the start of hopE to nt 573 was amplified using primers HopEF8 and HopER9 (product A). The p60 antigen was produced by annealing primers Lmp60F2 and Lmp60R2 (product B). The region directly downstream of hopE nt 573 was amplified using primers HopEF9 and HopER8 (product C). Product resulting form SOE PCR was cloned into Smal digested pUC19 plasmid to produce pUCHLm. For cagA, a region, including the 3 end of cagA was amplified using primers CagAF6 and CagER6 (product A). The p60 antigen was produced by annealing primers Lmp60F2 and Lmp60R2 (product B). The region directly downstream of cagA was amplified using cagAF6 and cagAR8 (product C). Product resulting from SOE PCR was cloned into Smal digested pUC19 to produce pUCCLm.

For vacA, a region of vacA was amplified from strain 26695, including the gene promoter and signal sequence, using primers vacAF1 and vacAR1 (product 1). The p60 antigen was produced by annealing primers Lmp60F3 and Lmp60R3 (product B). A section of *vac*A including the region coding the autotransporter and mature VacA was amplified using vacAF2 and vacAR2 (product C). Product resulting form SOE PCR digested with Xbal and *Kpnl,* and cloned into similarly digested pUC2 to produce pUCVLm.

### Western Blot Analysis

To determine if recombinant bacteria produced a fusion between HopE and the antigenic epitope, Western Blot analyses was performed

*H. pylori* were harvested from a 24 h blood agar into HEPES (pH 7.4) and were pelleted by centrifugation at 4500 rcf for 5 min and were disrupted by sonication. Lysate was centrifuged at 4500 rcf for 5 min to pellet unlysed bacteria, which were discarded. To harvest the total membrane fraction, the supernatant was centrifuged at 100 000 rcf for 60 min. All procedures were performed at 4°C. Samples were resuspended in HEPES buffer and added to an equal volume to Laemmli buffer. Proteins were separated in a 12% Tris-HCI Ready Gel (BioRad) by sodium dodecyl sulfate (SDS)-polyacrylamide gel electrophoresis (PAGE) before electro-transfer to 0.45 pM nitrocellulose (Biorad). For the detection of HopE and other proteins, membranes were blocked with 10% or 5% non-fat dairy milk (NFDM) in Tris buffered saline (TBS) pH 7.4. Membranes were probed with primary antibodies or secondary antibodies in 1 % NFDM in TBS. Polyclonal rabbit anti-HopE antibody was donated (Astra Zenica) and used at a dilution of 2:4000. Monoclonal mouse anti-p60 (K3A7) was donated (University of Wurzburg, Germany) and was used at a dilution of 2:4000. Mouse anti-HCCA (C7-50; Sigma) was at a dilution of 2:4000. Secondary antibodies were conjugated to alkaline phosphatase, and were detected using nitro blue tetrazolium chloride / 5-Bromo-4-chloro3-indolyl phosphatase solution (Roche Applied Science).

### lmmuno-based Assays of Surface Localization

*H. pylori* were harvested from a 24 h blood agar plate into phosphate buffered saline (PBS; pH 7.4). Bacteria were rinsed a total of 3 times and standardized to and OD (600nm) of 0.5. Rinsed bacteria were bound to a poly-L-lysine coated chamber slide overnight at 4°C. Bound bacteria were fixed with 0.25% glutaraldehyde, which was subsequently blocked with 100 mM glycine buffer. Between subsequent steps, slides were rinsed three times in PBS containing 0.05% Tween 20 (PBST). Slides were locked with 3% bovine serum albumin (BSA) in PBST for 2 h at 37°C. Primary antibodies were diluted 1:200 in PBST containing 1% BSA and incubated for 1.5 h at room temperature. Secondary antibodies conjugated to Alexafluor 488 were diluted 1:400 in PBST and incubated for 2 h at 37°C. Slides were rinsed and surface bound antibodies were detected using fluorescence microscopy. Whole cell based ELISA was performed similarly to indirect immunofluorescence studies with the following exceptions: bacteria were bound to Maxisorp (Nunc) plates either overnight or for 1 hr at 4°C; primary antibody was diluted 1:300; secondary antibody conjugated to alkaline phosphatase was diluted 1:1000; nitrophenyl phosphate (Sigma) was used as a substrate for detection at 405nm using a microtiter plate reader.

### Results

### Construction of Recombinant H. pylori

Recombinant *H. pylori* B128 (7.13) or 26695 were produced that harbored the sacB cassette in either the *hopE, cag*A or *vac*A gene. The sacB cassette was replaced with DNA coding antigens at specific positions within these genes. Both HCCA and p60 antigen DNA were inserted into hopE at a position corresponding to amino acid 168 of mature HopE, within a putative loop structure (51). p60 antigen DNA was also inserted directly upstream of the cagA stop codon, corresponding the C-terminal of CagA. Fusions of VacA and antigenic proteins were performed in strain B128 (7.13), which does not produce VacA. p60 antigen DNA was joined to the 26695 promoter, signal sequence and autotransporter DNA, prior to insertion into B128 (7.13) vacA.

### Analysis of Fusion Proteins and Surface Presentation

To date only HopE fused to HCCA or p60 antigens were analyzed. Western Blot analysis of *H. pylori* B128 producing the HCCA (B128:HCCA:*hop*E) or p60 antigen (B128:p60:*hop*E) DNA inserted into *hop*E showed that the antigens were fused to HopE (Figure 9). Specifically, analyses performed using the anti-HopE antibody showed that in recombinant *H. pylori* native HopE (about 31 kDa) was replaced with either HopE fused to either p60 antigen (about 32 kDa) or HCCA (about 35 kDa). Further analysis using antigen specific antibodies detected similar sized proteins.

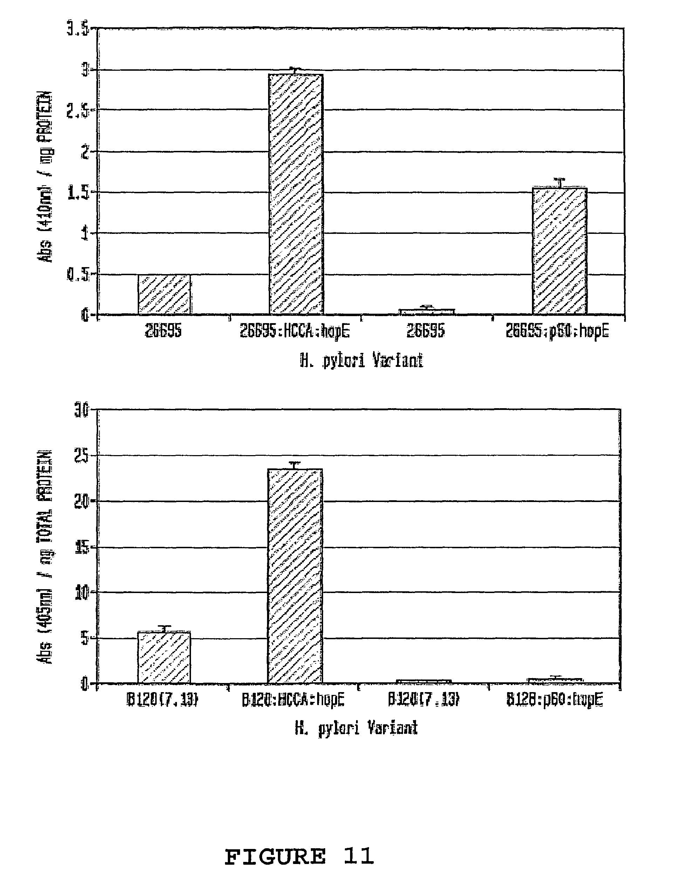

To examine surface presentation, two indirect immuno-based assays of surface localization were employed. Immuno fluorescence based microscopy analysis of *H. pylori* B128:HCCA:*hop*E indicated surface presentation of HopE fused to the HCCA (Figure 10). However, surface localization of HopE fused to p60 antigen could not be confirmed. These results were confirmed by whole cell based ELISA analysis (Figure 11B). The p60 antigen was only detected using this method after overnight binding of bacteria to ELISA plates (Figure 11A).

Studies in this example were done primarily with *H. pylori* strain B128, variant 7.13 (Franco, 2005)(52). The sacB cassette, conferring sucrose sensitivity and kanamycin resistance, was inserted into the hopE gene of H. pylori. DNA coding the HCCA or p60 epitope was joined to hopE using Splicing by Overlapping Extension (SOE) PCR. Recombinant DNA was transferred into sucrose-sensitive *H. pylori* using natural transformation and homologous recombination. Recombinant *H. pylori* was sucrose resistant, resulting from replacement of sacB with transforming DNA. The following oligonucleotide primers presented in Table 1 were used in the assays described herein:

**Table 1 - Oligonucleotide Primers:**

| Primer | Sequence |
|---|---|
| CagAF1 | AAAACCATGGATTTCAGTAGGGTAGAGCAA |
| caAF11 | ATCAAGAAAGAATTGAATG4GA |
| CagAF2 | AAAAAGATCTAGGATTAAGGAATACCAAAAACGCA |
| CagAF6 | ACCGAA TAAAGGATTAAGGAATACCAAA |
| CagAR1 | AAAAAGATCTTTAAGATTTTTGGAAACCACCTT |
| CagAR2 | AAAATCTAGAGGTTATTTTAGGTTGCACGCATTTT |
| CagAR6 | AGCTTCAGATTTTTGGAAACCACCTT |
| CagAR8 | GTTATTTTAGGTTGCACGCATT |
| CATF | TTTTAATCCGCCATATTGTGTTGAA |
| CATR | AAGGGTCGTTTAAGGGCACCAATAACT |
| HCCAF1 | GGTCCTCAGCGGAAGACGAAGCGTAATACAAACAGGAGACCACA |
| HCCAR1 | AACATCGCTGGTTTTGCGAGTTGCTCTTACCCCCAAACG |
| HCV2s | CCACCGACTATTTGACCCCCTCCAGGAAATTTAACATCTTGTGGTCTCCT |
| HCV3.s | ATAGTCGGTGGCGTGTATTTACTACCCAGGCGAGGACCGCGTTTGGGGGT |
| HopEF1 | AAAAAGATCTAATGGAATTTATGAAAAAGTTTGTAGCT |
| HopEF2 | ACCAGCGATGTTTGTACCCCTACTTATTGTAACCCTAA |
| HopEF3 | AATGGGAATTTATGAAAAAGTTTGTAGCT |
| HopEF4 | TAAACGACCCTTTAAAAGGGTGTCTTTA |
| HopEF6 | AAAACCATGGATGGAATTTATGAAAAAGTTTGTAGC |
| HopEF7 | AAAAAGATCTTAAACCCTTTAAAAGGGTGTCTT |
| HopEF8 | ATGGAATTTATGAAAAAGTTTGTAGC |
| HopEF9 | ACCGAAGATGTTTGTACCCCTACTTATTG |
| HopER1 | CCGCTGAGGACCCTTAGCTTCAATGA |
| HopER3 | GGCGGATTAAAAAGTGTAGTTATACCCTAAA |
| HopER5 | CGGCTTGAAACACCAAAGTC |
| HopER6 | AAAAAGATCTAAAAGTGTAGTTATACCCTAAATAA |
| HopER7 | AAAATCTAGACTTCTGGGCTTGGAGTGATG |
| HopER8 | CTTCTGGGCTTGGAGTGATG |
| HopER9 | AGCTTCAGGACCCTTAGCTTCAATGATT |
| KanSacF | AAAAAGATCTCGAACCATTTGAGGTGATAG |
| KanSacR | AAAAAGATCTTATAGCCCATTTTCATGCTCTT |
| Lmp60F1 | GGTCCTGAAGCTGCTAAACCTGCTCCTGCTCCTAGCACCAATCAACAACAAACCGCTCCTAAAGCTCCTACCGAAGATCTT |
| Lmp60F2 | AAATCTGAAGCTGCTAACCTGCTCCTGCTCCTAGCACCAATCAACAACAAACCGCTCCTAAAGCTCCTACCGAATAAAGG |
| Lmp6DF3 | CATGCCGAAGCTGCTAAACCTGCTCCTGCTCCTAGCACCAATCAACAACAAACCGCTCCTAAAGCTCCTACCGAACCCGAC |
| Lmp60R1 | AACATCTTCGGTAGGAGCTTTAGGAGCGGTTTGTTGATTGGTGAGGAGCAGGAGCAGGTTTAGCAGCTTCAGGACC |
| Lmp60R2 | CCTTTATTCGGTAGGATTTAGGAGCGGTTTGTTGTTGATTGGTGCTAGGAGCAGGAGCAGGTTTAGCAGCTTCAGATTT |
| Lmp60R3 | GTCGGGTTCGGTAGGAGCTTTAGGAGCGGTTTGTTGTTGATTGGTGCTAGGAGCAGGAGCAGGTTTAGCAGCTTCGGCATG |
| vacAF1 | AAAGGTACCAAAGCCGATAGCATCAGAGAA |
| vacAF2 | ACCGAACCCGACAATTACAAGTATCTTAT |
| vacAF3 | AAAGAATTCAATTTGGTTTCAAGCTCAAATCAGA |
| VacAF4 | AAATCTAGAACTACATCTGCCACTAATGTGAA |
| vacAR1 | AGCTTCGGCATGACTTTGTTGCGGTGTGAT |
| vacAR2 | AAATCTAGATTAGAAACTATACCTCATTCCTA |
| vacAR3 | AAAGGTACCGAGCTTGTTGATATTGACTTTGT |
| vacAR4 | AAAAAGCTTCATTCTCAGTAGGCGTAGAAT |

**Table 2 - Strains of H. pylori used and developed:**

| Strain | Antigen DNA Inserted | Insertion Site | Designation |
|---|---|---|---|
| B128 (7.13) | | | B128 (7.13) |
| B128 (7.13) | p60 | *hop*E | B128:p60:*hop*E |
| B128 (7.13) | HCCA | *hop*E | B128:HCCA:*hop*E |
| B128 (7.13) | p60 | *vac*A | B128:p60:*vac*A |
| 26695 | | | 26659 |
| 26695 | p60 | *hop*E | 26695:p60:*hop*E |
| 26695 | HCCA | *hop*E | 26695:HCCA:*hop*E |
| 26695 | p60 | *cag*A | 26695:p60:*cag*A |

Natural transformation of *H. pylori* strain B128 yielded recombinant bacteria which produced *hopE* fused to either p60 or HCCA DNA as confirmed by sequence analysis. Resulting fusion proteins were larger than unaltered HopE (Figure 8). Confirmation of HCCA surface localization was shown using fluorescence microscopy (Figure 9), and was corroborated by results from whole cell based ELISA analysis.

### EXAMPLE 17 DETERMINATION OF SITES IN UREASE GENE FOR THE FLAG TAG INSERTION

It is thought that presentation of vaccine antigens on the *H. pylori* cell surface or on secreted proteins will allow development of antigen specific protective immune responses. In this study, the proteins UreA and UreB were considered as presentation molecules. These molecules associate to form the multi-subunit enzyme urease. Urease was chosen for several reasons, it is:
1. One of the most abundant of H. pylori proteins (>5% of total protein);
2. Antigenic during H. pylori infection;
3. Structure is known;
4. Activity is easily detected by colony colour
5. Active urease can be selected for directly during culture
6. Urease is thought to be essential in vivo, allowing selection of active urease.

The association of urease subunits is complexed, and it is unknown whether the enzyme will tolerate any insertions. Therefore initial tolerance of insertion was investigated using the FLAG tag (8 aa) and later the FLAG tag adjacent to the p60 antigen (total 31 aa). FLAG is a short immunogenic tag for which commercial antibodies are available. The *L*. *monocytogenes* p60 protein is a highly immunogenic murein hydrolase that is essential for cell division. The protein is secreted in large quantities into growth media by Listeria spp., is approximately 60-kDa in size, and is encoded by the Iap (invasion-associated protein). Sections of p60 used in this study comprised of CD4 T cell and B cell epitopes of the p60 protein, and has been shown to confer protection against murine listeriosis when presented by a vaccinating strain of Salmonella expressing the epitope on its surface.

As alternative antigen carriers, the related HcpA and HcpC protein are also being considered. The crystal structure has been resolved for HcpC. These molecules belong to the Sel-1 like family of proteins, are most likely secreted and they are "seen" by the immune system in humans. They are not needed in bacterial culture; the possibility that they are needed or contribute to vigor during infection remains to be tested.

Based on the foregoing, the aims of this project was to determine sites in urease tolerant of the FLAG tag insertion; insertion of Listeria p60 protein epitopes into tolerant sites of urease in mouse colonizing strains of *H. pylori* and insertion of *Listeria* p60 protein epitopes into tolerant sites of HcpA and HcpC in mouse colonizing strains of *H. pylori.*

### Methods

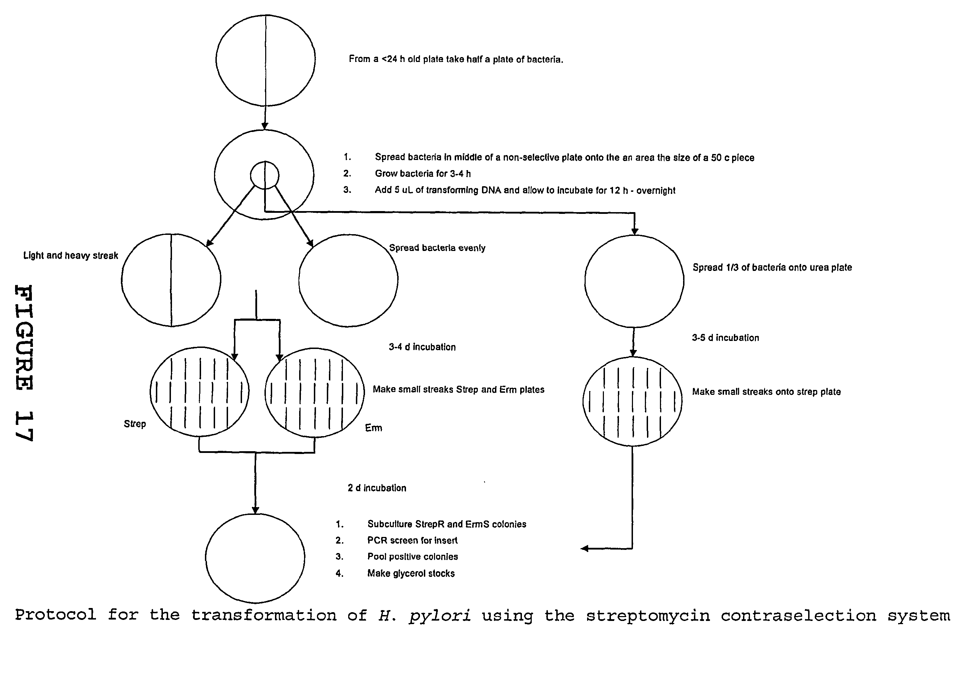

Bacteria were generally cultured on blood based media. Strains used were streptomycin resistant (StrR) variant of 26695 (mutation of rpsL at codon 43) and also X47 (already StrR resistant due to mutation of rpsL codon 88). Gene fusion employed the streptomycin counterselection system developed in the Douglas Berg Lab (Washington University), the primers described in Table 3 and natural transformation (Figure 17).

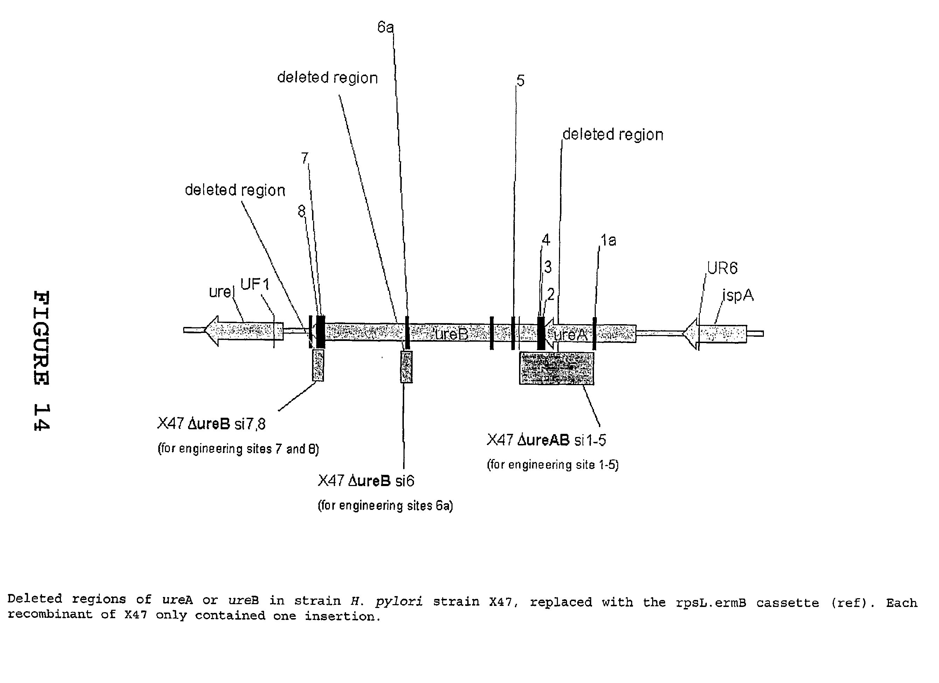

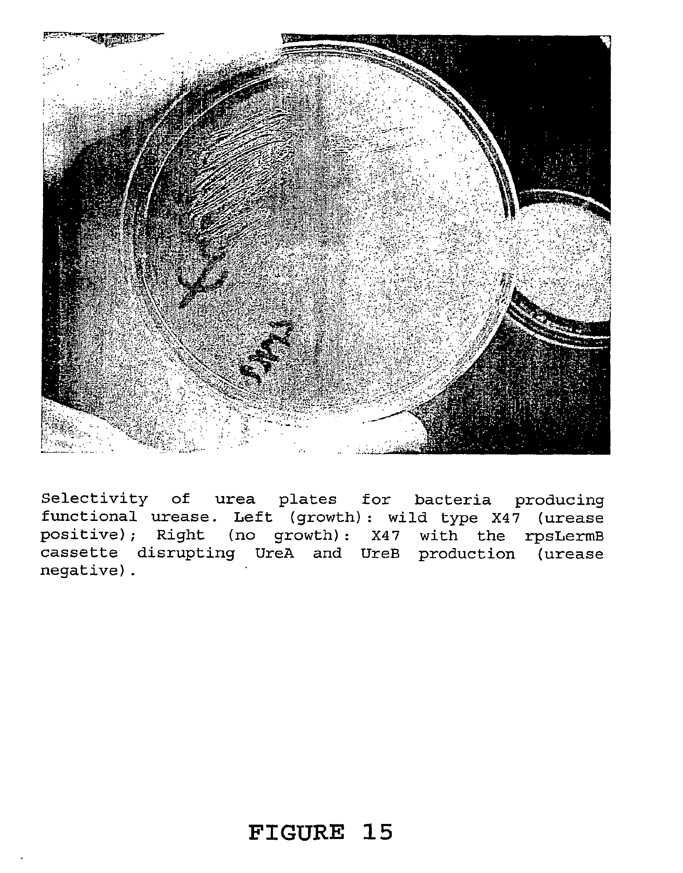

Recipient strains were produced by inserting the rspLermB cassette into the gene of interest (Figure 14). Replacement of the rpsLermB cassette with PCR products containing FLAG and flanking sequences for homologous recombination conferred streptomycin resistance and urease activity. Transformants with functional urease were selected for on agar plates containing streptomycin and urea and phenol red (Table 4). Only those bacteria producing a functional recombinant urease were able to grow on urea plates (Figure 15).

**Table 3. Primers designed in this study. Upstream and downstream refers to the position of the flanking region for homologous region relative to the site for antigen insertion. Designations are for genome 5'-3'**

| | Primer Name | Primer Sequence (5'-3) |
|---|---|---|
| Insertion of rpslermB cassette into ureA/ureB | | |
| UR2 - large urease deletion for engineering sites 1-8 | UF1 | cgactttggttaacccgcaaatcccat |
| UF1 - large urease deletion for engineering sites 1-8 | UR2 | ttcaatagctataaattatttaataagtaagcaattcaacgacactaccgctcacat |
| UR6 - large urease deletion for engineering sites 1-8 | UF5 | gtgtttaatccatagttataaagcatccgcttcttccataatatgggcacta |
| UF5 - large urease deletion for engineering sites 1-8 | UR6 | gggcgtggtggattatgtgtatta |
| UF1 - urease deletion for engineering site 6 | UR2-1 | ttcaatagctataaattatttaataagtaacgctgattcaaggatccgccctcaa |
| UR6 - urease deletion for engineering site 6 | UF5-1 | gtgtttaatccatagttataaagcatcccaagtggtggcacaccataa |
| UR6 - urease deletion for engineering sites 1-5 | UF5-234 | gtgtttaatccatagttataaagcatcccattggcctcaataggggtat |
| UF1 - urease deletion for engineering sites 1-5 | UR2-234 | ttcaatagctataaattatttaataagtaacggtggcggtaaaaccct |
| UF1 - urease deletion for engineering sites 7,8 | UR2-56 | ttcaatagctataaattatttaataagtaagtgagcttggcgcaactcttta |
| UR6 - urease deletion for engineering sites 7,8 | UF5-56 | gtgtttaatccatagttataaagcatcgtgagcggtagtgtcgttgaat |
| ermR - amplification of rpsL-ermB cassette | rpsL-F | gatgctttataactatggattaaacac |
| rpsL-F - amplification of rpsL-ermB cassette | ermR | ttacttattaaataatttatagctattgaa |

| Insertion of FLAG tag into sites | | |
|---|---|---|
| UR6 - insertion of FLAG into site 1a (downstream flank) | si1aF1 | tttatcatcgtcatctttataatcvnnvnnvnnvnnvnnvnnggcctcaataggggtatgca |
| UF1 - insertion of FLAG into site 1a (upstream flank) | si1aR1 | gattataaagatgacgatgataaannbnnbnnbnnbnnbnnbaatggtaaattagttcctggtga |
| UR6 - insertion of FLAG into site 2 (downstream flank) | Rec1F1 | tttatcatcgtcatctttataatcvnnvnnvnnvnnvnngtcatcgcttttagcgccatgaa |
| UF1 - insertion of FLAG into site 2 (upstream flank) | Rec1R1 | gattataaagatgacgatgataaannbnnbnnbnnbnnbaactatgtaaaaacaattaaggag |
| UR6 - insertion of FLAG into site 3 (upstream flank) | Rec2R1 | gattataaagatgacgatgataaataagaaatgaaaaagattagcagaaaagaat |
| UF1 - insertion of FLAG into site 3 (downstream flank) | si3R1 | tttatcatcgtcatctttataatcvnnvnnvnnvnnvnnctccttaattgtttttacatagttgt |
| UR6 - insertion of FLAG into site 4 (downstream flank) | si4F1 | tttatcatcgtcatctttataatccatttcttactccttaattgtttttaca |
| UF1 - insertion of FLAG into site 4 (upstream flank) | si4R1 | gattataaagatgacgatgataaannbnnbnnbnnbnnbnnbaaaaagattagcagaaaagaatatgtt |
| UR6 - insertion of FLAG into site 5 (downstream flank) | si5F1 | tttatcatcgtcatctttataatcvnnvnnvnnvnnvnnvnnttcttctttgctagggttgttgga |
| UF1 - insertion of FLAG into site 6 (upstream flank) | si5R1 | gattataaagatgacgatgataaannbnnbnnbnnbnnbnnbttggatctaatcatcactaacgct |
| UR6 - insertion of FLAG into site 6a (downstream flank) | si6aF1 | tttatcatcgtcatctttataatcvnnvnnvnnvnnvnnvnntttatccaagtggtggcacaccat |
| UF1 - insertion of FLAG into site 6a (upstream flank) | si6aR1 | gattataaagatgacgatgataaannbnnbnnbnnbnnbnnbagcattaaagaagatgttcagttcgct |
| UR6 - insertion of FLAG into site 7 (downstream flank) | si7F1 | tttatcatcgtcatctttataatcvnnvnnvnnvnnvnnvnnttcaggattgacttcaatgtgagcggta |
| UF1 - insertion of FLAG into site 7 (upstream flank) | si7R1 | gattataaagatgacgatgataaannbnnbnnbnnbnnbnnbacttaccatgtgttcgtggatggcaa |
| UR6 - insertion of FLAG into site 8 (downstream flank) | si8F1 | tttatcatcgtcatctttataatcvnnvnnvnnvnnvnnvnngccatccacgaacacatggtaagtt |
| UF1 - insertion of FLAG into site 8 (upstream flank) | si8R1 | gattataaagatgacgatgataaannbnnbnnbnnbnnbnnbaaagaagtaacttctaaaccagcca |

| Screening for FLAG insertion | | |
|---|---|---|
| | FLAGF1 | tttatcatcgtcatctttataat |

| Sequencing of potential insertion sites in urease | | |
|---|---|---|
| | USeqF1 | cccaacatataacaatacaagtcctagcat |
| | USeqF2 | ccccatgtcatgcaaagtgtctt |
| | USeqF3 | cagtagcaggacctacgctaa |
| | USeqR1 | cgtggcaagcatgatccatgaagt |

| To produce p60FLAG fusion | | |
|---|---|---|
| Complementary to p60FLAGF1 | p60FLAGF1 | gaagctgctaaacctgctcctgctcctagcaccaatcaacaacaaaccgctcctaaagctcctaccgaa gattataaagatgacgatgataaa |
| Complementary to p60FLAGR1 | | tttatcatcgtcatctttataatcttcggtaggagctttaggagcggtttgttgttgattggtgctagg |
| | p60FLAGR1 | agcaggagcaggtttagcagcttc |
| UR6 - insertion of Lmp60FLAG into site 1a (downstream) | silaF2 | agcaggagcaggtttagcagcttcvnnvnnvnnvnnvnnvnnggcctcaataggggtatgca |
| UR6 - insertion of Lmp60FLAG into site 2 (downstream) | | agcaggagcaggtttagcagcttcvnnvnnvnnvnnvnnvnngtcatcgcttttagcgccatgaa |
| UR6 - insertion of Lmp60FLAG into site 3 (downstream) | si3F2 | agcaggagcaggtttagcagcttcvnnvnnvnnvnnvnnvnnctccttaattgtttttacatagttgt |
| UR6 - insertion of Lmp60FLAG into site 4 (downstream) | si4F2 | agcaggagcaggtttagcagcttccatttcttactccttaattgtttttaca |
| UR6 - insertion of Lmp60FLAG into site 5 (downstream) | si5F2 | agcaggagcaggtttagcagcttcvnnvnnvnnvnnvnnvnnttcttctttgctagggttgttgga |
| UR6 - insertion of Lmp60FLAG into site 6a (downstream) | si6aF2 | agcaggagcaggtttagcagcttcvnnvnnvnnvnnvnnvnntttatccaagtggtggcacaccat |
| UR6 - insertion of Lmp60FLAG into site 7 (downstream) | si7F2 | agcaggagcaggtttagcagcttcvnnvnnvnnvnnvnnvnnttcaggattgacttcaatgtgagcggt a |
| UR6 - insertion of Lmp60FLAG into site 8 (downstream) | si8F2 | agcaggagcaggtttagcagcttcvnnvnnvnnvnnvnnvnngccatccacgaacacatggtaagtt |

| Insertion of rpsLermB cassettte into HcpA | | |
|---|---|---|
| Not applied to date | hcpAF1 | gaagaggctccattaatggcaa |
| Not applied to date | hcpAR1 | gtgtttaatccatagttataaagcatccgttttagcttgagcgaaatcttgcttct |
| Not applied to date | hcpAF2 | ttcaatagctataaattatttaataagtaacttgcgacttaaaccatgctgaa |
| Not applied to date | hcpAR2 | ctagccatagcgagagtctgaa |

| Insertion of the Lmp60 fusion into HcpA | | |
|---|---|---|
| Not applied to date | hcpA-NR1 | agcaggagcaggtttagcagcttcaggttctgccattaaccctctcaaacacaacgt |
| Not applied to date | hcpA-NF1 | gattataaagatgacgatgataaagaacctgagccagacgctaaagagcttgtt |
| Not applied to date | hcpA-CR1 | agcaggagcaggtttagcagcttcaagttctatttttaattccttgagagcgt |
| Not applied to date | hcpA-CF1 | gattataaagatgacgatgataaataatttcaatgaagttagctaaacgctgcgtt |

| Insertion of Lmp60FLAG fusion into HcpC | | |
|---|---|---|
| hcpN-R1 - insertion of Lmp60FLAG at N-terminus (downstream) | 1098C6n | cccgggtcttacgataaagcttttagaaca |
| hcpC-NF1 - insertion of Lmp60FLAG at N-terminus (upstream) | 1098A1C | cctcaaaccacaagacattgctaacatcgtgct |
| 1098C6n - insertion of Lmp60FLAG at N-terminus (downstream) | hcpC-NR1 | agcaggagcaggtttagcagcttcttgttctgccattagcccccctaaacacaa |
| 1098A1C - insertion of Lmp60FLAG at N-terminus (upstream) | hcpC-NF1 | gattataaagatgacgatgataaagaacaagagcaagaccctaaagagcttgt |
| Not applied to date | hcpC-CR1 | agcaggagcaggtttagcagcttcaactttgattttgagctgcttgagaata |
| Not applied to date | hcpC-CF1 | gattataaagatgacgatgataaatagtttgaataaggcttgatcaaacggctt |

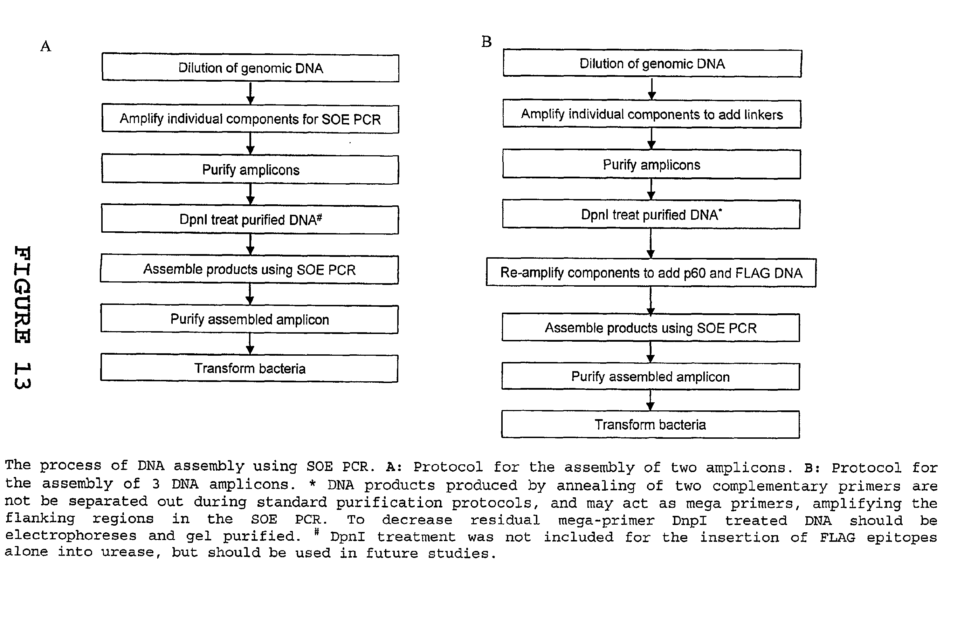

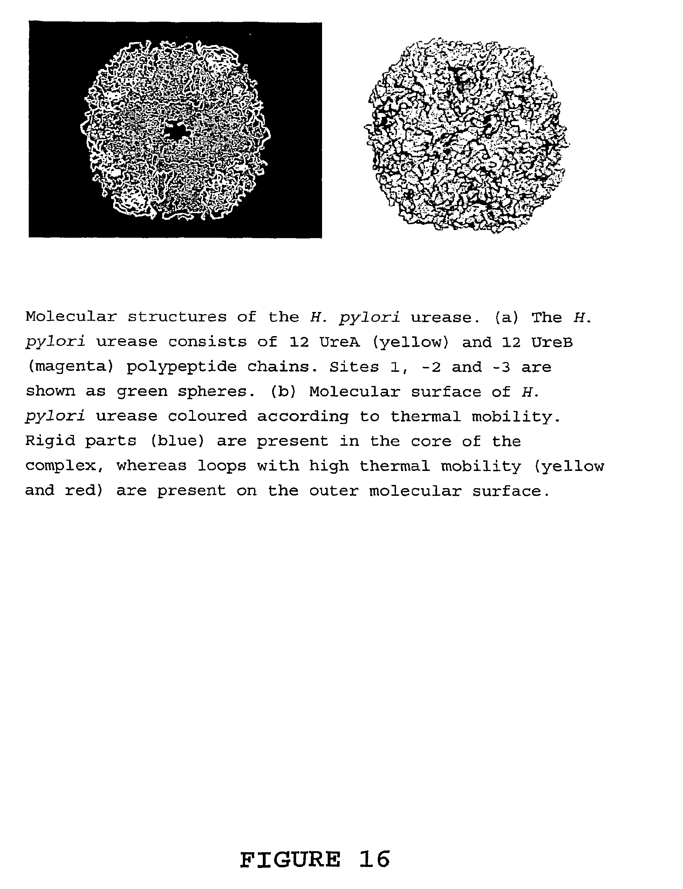

8 site in urease were selected (Figure 16), and 2 sites in each HcpA and HcpC likely to tolerate insertion of amino acids (Figure 12b; Figure 12c). PCR products carrying desired epitope (FLAG) flanked by 5 or 6 semi-random codons, selected to exclude 2 of the 3 possible stop codons, were spliced together in a second round of PCR and used for DNA transformation of recipient strains (Figure 13a). Similar methodology was performed for the insertion of p60 eptiopes adjancent to FLAG (p60FLAG). However genomic template DNA was diluted and resulting amplicons were DpnI to remove genomic DNA (Figure 13b)

### Results

The ureA and ureB genes were deleted, and also three small sections of ureA or ureB corresponding to 3 different groups of potential insertion sites, were deleted and replaced with the rpsLermB contraselection cassette by Dr. SungSook Choi (Sahmyook University, currently on sabbatical in Berg Lab) (Figure 14). The three rpsLermB insertions were replaced by DNA transformation with assembled PCR products containing the FLAG tag at each of the 8 sites within ureA or B. Bacterial transformants made with these PCR products were selected that contained functional urease and three were confirmed to contain the FLAG tag at sites 3, 4 and 8 by DNA sequencing (Figure 12a). Assembled PCR products were amplified to insert p60 antigen adjacent to the FLAG tag in to urease at the 8 sites. H. pylori were transformed with the assembled products, resulting in the isolation of recombinants that were detected by PCR to contain the p60 FLAG tag at site 1a, 3 or 4 in functional urease (Figure 12a).

### EXAMPLE 18 ANALYSIS OF H. pylori UREASE FOR THE INSERTION OF FLAG-TAGS

The three-dimensional structure of the *H. pylori* urease complex has been reconstructed from the deposited coordinates (PDB-id: 1e9y) by applying the crystallographic symmetry operators. The urease forms a large spherical complex and consists of 12 UreA (SwissProt-id: P14916) and 12 UreB (SwissProt-id: P69996) polypeptide chains (Fig. 16a). This complex was visually inspected for surface exposed loops that also show high thermal mobility. Figure 16b shows the molecular surface coloured according to thermal mobility.

The visual inspection revealed three sites that seem to be suitable for the insertion of Fag-tags (DYKDDDDK peptides). The sites are ranked according to their suitability:

Site 1 - This site seems to be most suitable. Residues 324 to 333 from UreB are completely solvent exposed and show high thermal flexibility (red surfaces in Fig. 16b). The high flexibility seems to be caused by the lack of stabilizing interactions with the urease core. Therefore the conformation of the peptide chain is not restrained and seems to be suitable to accommodate the insertion of larger inserts. By replacing residues L324DKSIKEDVQ333 of UreB (P69996) against the Flag octapeptide (note that L324 is a lysine in the crystal structure). Since the loop is relatively long several replacements and insertions are possible and should be tested. Although this loop is very flexible, it is remarkable well conserved in several UreB sequences. Unless it is involved in an unknown cellular function (adhesion to the *H. pylori* cell wall for example) no disturbance of the molecular function of urease should be

Site 2 - Residues H224GAKSDDN231 of UreA (P14916) seem to be suitable for the replacement against the Flag octapeptide but not for the insertion of larger fragments, such as the Hepatitis C coat protein for example, because this loop is located close to a three-fold symmetry axis. The loop is located close to the UreA C-terminus, it is solvent exposed, flexible and shows little interaction with the urease core. Therefore mutations should not effect urease activity. The seven amino acids between N231 and the UreA C-terminus interact with symmetry related subunits. Therefore I would recommend to maintain these residues although the multiple sequence alignment shows that the UreA C-termini possess little sequence conservation.

Site 3 - Residues E101ANGKLV107 of UreA could also be replaced against DYKDDDDK. The insertion of one additional amino acid should be tolerated by the overall structure, because this loop shows high thermal flexibility. The multiple sequence alignment reveals that this loop contains insertions in UreA homologues from different species.

These sites are considered best based on the HP urease structure alone. These sites have been checked on the basis of a superposition of the HP urease structure onto homologous urease structures from other bacteria. These superpositions are useful to identify rigid and well conserved sites and to distinguish them from flexible surface loops that might be useful for the insertion of epitopes. The urease structures where all rather similar, suggesting that there is little structural flexibility due to functional restrains. However, the superposition confirmed that sites 1 to 3, the UreA C-terminus and the UreB N-terminus are suitable to accommodate the tags. In addition we found three additional sites on UreB that should be considered as backups.

The numbering refers to the SwissProt entry P69996:

Site 4 (UreB) .NNPSKEE(65) NNNNNN DYKDDDDK NNNNNN L(66)DLIIT. . .

Site 5 (UreB) .HIEVNPE(541) NNNNNN DYKDDDDK NNNNNN T(542)YHVFV...

Site 6 (UreB).YHVFVDG(549) NNNNNN DYKDDDDK NNNNNN K(550)EVTSKP...

Since N- and C-termini are freely accessible I would recommend putting the Flag-tag either at the C-terminus (together with three to four padding residues)
...DILKQLKIKV XXX (Flag-Tag)
or right after the leader peptide between residues 25 and 26 such as
...GGLMA XXX (Flag-Tag) XXX EQDPK...
where "X" denotes the padding residues. Besides HcpC (Hp1098) I would also consider HcpA (Hp0211). So far we know that HcpA elicits the stronger IFN-gamma release which correlates well with a better protection in the mouse immunization model. The corresponding constructs for HcpA would be
...ALKELKIEL XXX (Flag-Tag)
and
...RGLMA XXX (Flag-Tag) XXX EPDAK...

From the structural point of view there is no need for inserting a linker at all, because both termini are freely accessible. Therefore by skipping the linker for the C-terminal construct and just put the Flag-tag right after the native C-terminus. For the N-terminal constructs we would put a two amino acid long linker between the N-terminal signal peptide and the Flag-tag. The reason for this is, that there is some ambiguity for the prediction of the exact cleavage site of the leader-peptidase and we would like to avoid that the leader-peptidase cleaves somewhere inside the Flag-tag. Possible constructs for HcpA and -C could look like
(HcpA/Hp0211)
MLGNVKKTLFGVLCLGTLCLRGLMA EP (Flag-Tag) EPDAKELVNLGIESA...
(HcpC/Hp1098)
MLENVKKSFFRVLCLGALCLGGLMA EQ (Flag-Tag) EQDPKELVGLGAKSY. . .

For larger tags that fold into defined three-dimensional structures we might have to consider linkers.

### Media Preparation

The combination of the following antibiotics and growth supplements allowed the culture of *H. pylori* on a regular basis free from fungal and bacterial contamination.
1. Mix media components being sure to allow sufficient volume for the addition of antibiotics
2. Once autoclaved let media come down to 50°C in a water bath
3. Pre warm 100 mL of antibiotic stock, such that the final media concentration is isovitalex (0.4% v/v), amphotericin B (8 µg/mL), vancomycin (6 µg/mL) and trimethoprim (5 µg/mL). Also pre-warm blood.
4. Add pre-warmed blood to autoclaved media
5. Mix thoroughly, trying to avoid making bubbles
6. Add pre-warmed 100 mL of antibiotics solution to the autoclaved media
7. Mix thoroughly, trying to avoid making bubbles
8. Add any ethanol soluble antibiotics to the media (erythromycin)
9. Pour plates.

Media for mice studies is additionally supplemented with nalidixic acid (10 µg/mL), polymyxin B (10 µg/mL) and bacitracin (200 µg/mL).

### Urea Selection Plates

**TABLE 4**

| MEDIA COMPOSITION FOR THE SELECTION OF UREASE POSITIVE *H. PYLORI* | | |
|---|---|---|
| Component | Weight/Volume | Direction |
| Brucella Broth | 28 g | Prior to autoclaving |
| Bacto Agar | 15 g | Prior to autoclaving |
| Phenol Red | 100 mg | Prior to autoclaving |
| Horse Serum | 70 mL | After autoclaving |
| Urea (40% w/v) | 1.5 mL | After autoclaving |
| HCl (1N)* | Until yellow (∼10mL?) | After autoclaving or until plates are yellow-orange |
| Vancomycin (6mg/mL) | 1 mL | After autoclaving |
| Water | to make up TOTAL | |
| TOTAL VOLUME | 1L | |

### Storage Medium

Tryptic soy broth (g/L) and 20% (v/v) glycerol. Swizzle 24 h cultures in this medium, and snap freeze using dry ice. Stocks are not thawed to be samples and are cells scraped from the frozen surface for culturing. The stock can be re-used multiple times.

### Liquid Medium

Brucella broth (g/L), supplemented with 7% (v/v) horse serum and vancomycin (6 µg/mL). Generally use 10 mL in a 50 mL screw cap conical flask, shaking at 150 rpm in an control gas incubator at 37°C.

### Assembly by SOE PCR

All constructs were assembled by SOE PCR, and used to transform bacteria. The process for which is shown in Figure 13.

### Transformation of H. pylori

A diagrammatic representation of the Berg transformation protocol is shown in Figure 17.

### EXAMPLE 19 INFLUENZA HAEMAGGLUTININ EPITOPES FUSION TO UREASE

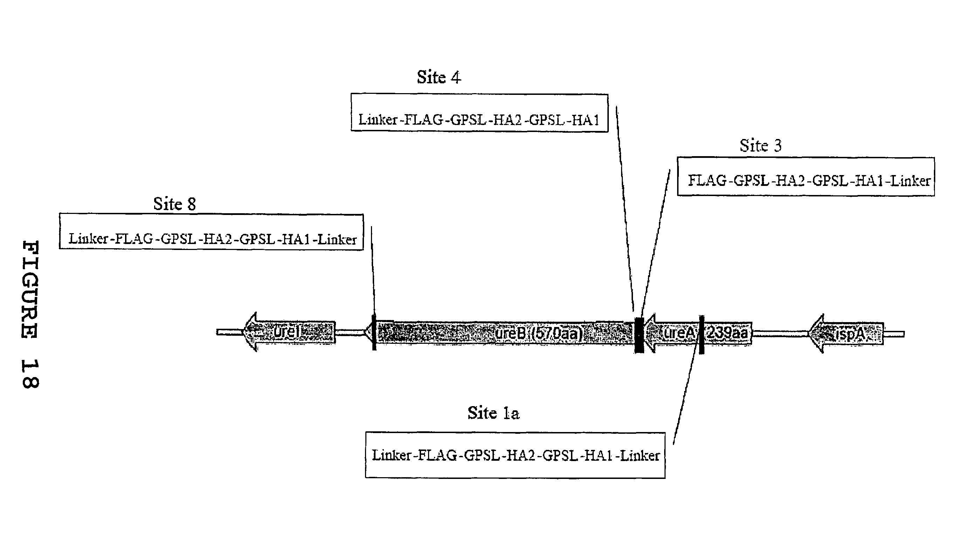

A/PR/8/34 haemagglutinin (HA) epitopes were used as antigens for presentation by recombinant urease and delivery by *H*. *pylori.* The HA epitopes chosen for the project are the HA T-cell epitope (amino acid sequence: SFERFEIFPKE) and the HA B-cell epitope (amino acid sequence: WLTEKEGSYP). The combination of these epitopes in a vaccine should result in the elicitation of both humoral and cell mediated responses in the host. All constructs were assembled by SOE PCR using the process shown in Figure 13. The corresponding nucleic acid sequence of A/PR/8/34 haemagglutinin (HA) epitopes were constructed by PCR using primers listed below and inserted in the urease as depicted in Figure 18. Transformation of *H. pylori* was performed according to the diagrammatic representation of transformation protocol shown in Figure 17 and functional recombinant urease fusions were selected.

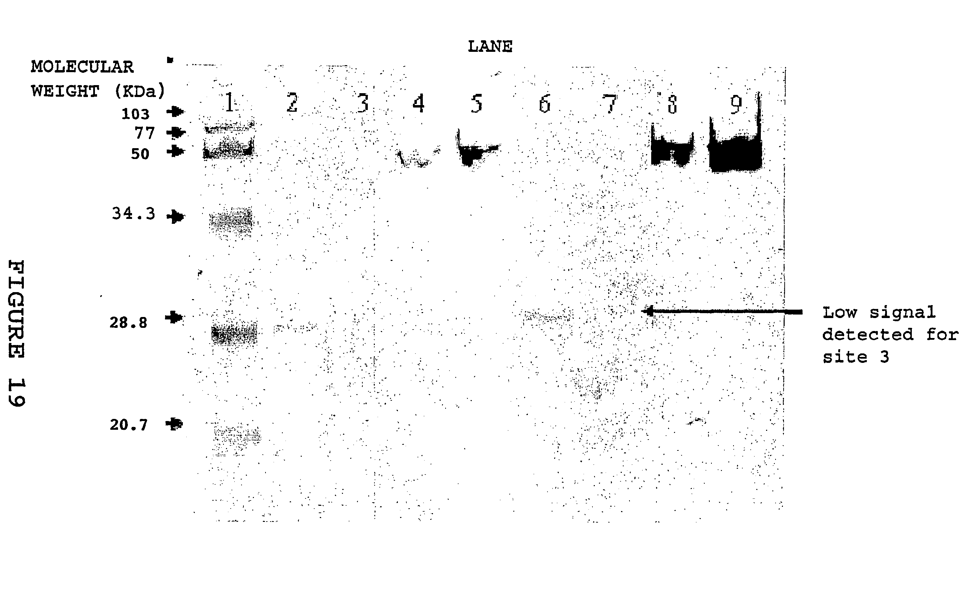

To detect epitopes within recombinant urease, a Western blot analysis was performed. The inclusion of a FLAG protein tag sequence adjacent to the influenza epitopes allowed the detection of recombinant protein using commercially available antibodies. Western blot analysis showed that FLAG was inserted at site 1a on ureA, the smaller protein subunit, and sites 4 and 8 on ureB, the larger protein subunit. A very low signal detected for FLAG protein tag at site 3. (Figure 19). This band was the incorrect size, corresponding to FLAG tag on ureA, rather than *ure*B.

### EXAMPLE 20 IMMUNIZATION USING INFLUENZA HAEMAGGLUTININ EPITOPES FUSION TO UREASE

In order to determine the immunogenicity of genetically modified Helicobacter pylori, C57BL/6 mice were immunized, intra-gastrically with 200µl of 10⁹ cfu / ml of *H. pylori* expressing HA epitopes at sites 1a, 3, 4 or 8 of the urease (sila, si3, si4 and si8). 73 days later, serum was collected and anti-HA IgG titres determined by ELISA. Briefly, plates were coated with the B-cell epitope peptide coupled to Ribonuclease H via its C-terminus (SFERFEIFPKEC) at 5 mg/ml and incubated overnight at 4°C. After blocking plates with 2% PBST-BSA at 37°C for 2 h, serum samples were added and plates were incubated for 1 h at room temperature. Anti-HA IgG antibodies were detected using an alkaline-phosphatase-labelled goat anti-mouse IgG secondary antibody. Nitrophenyl phosphate was used as the substrate and the reaction was stopped after 30 minutes with NaOH. Optical density (OD) was measured at 405nm. Figure 20 shows that mice immunized with live recombinant *H. pylori* produced antigen specific antibody after 73 days colonization in stomach. 5 out of 20 of immunized mice have produced antigen specific antibody in sera. 11 mice among 20 immunized mice have been found to be colonized by *H. pylori* after 73 days. These serological positive mice were found in groups immunized with sila, si4 and si8 recombinant *H. pylori.* There is no antigen specific immune response in the group with si3 recombinant *H. pylori* immunization.

Primers used in this study included:

| Primer Name | Sequence 5' to 3' |
|---|---|
| si1aF3 | GGC CCT TCT TTA GGA AAA ATT TCA AAT CTT TCA AAG CTV NNV NNV NNV NNV NNV NNG GCC TCA ATA GGG GTA TGC ACG GTT |
| si3F3 | GGC CCT TCT TTA GGA AAA ATT TCA AAT CTT TCA AAG CTV NNV NNV NNV NNV NNV NNC TCC TTA ATT GTT TTT ACA TAG TTG T |
| si4F3 | GGC CCT TCT TTA GGA AAA ATT TCA AAT CTT TCA AAG CTC ATT TCT TAC TCC TTA ATT GTT TTT ACA |
| si8F3 | GGC CCT TCT TTA GGA AAA ATT TCA AAT CTT TCA AAG CTV NNV NNV NNV NNV NNV NNG CCA TCC ACG AAC ACA TGG TAA GTT |
| HAFLAG | TTT ATC ATC GTC ATC TTT ATA ATC TAA GCT AGG CCC AGG ATA GCT CCC TTC TTT TTC GGT TAA CCA TAA GCT AGG CCC TTC TTT AGG AAA AAT TTC AAA T |
| HAFLAG | ATT TGA AAT TTT TCC TAA AGA AGG GCC TAG CTT ATG GTT AAC CGA AAA AGA AGG GAG CTA TCC TGG GCC TAG CTT AGA TTA TAA AGA TGA CGA TGA TAA A |
| UseqSite8R | CAG AGA AAT GTT CGC TCA TCA TGG T |
| UF1 | CGA CTT TGG TTA ACC CGC AAA TCC CAT |
| UR6 | GGG CGT GGT GGA TTA TGT GTA TTA |
| UF7 | GGT GGA GTG CAG AAT AAG TGA TGA CAA |
| UR7 | CCA TGC GAT AGA GTT TGG CAT GGT GT |
| si1aR1 | GAT TAT AAA GAT GAC GAT GAT AAA NNB NNB NNB NNB NNB NNB AAT GGT AAA TTA GTT CCT GGT GA |
| si3R1 | TTT ATC ATC GTC ATC TTT ATA ATC VNN VNN VNN VNN VNN CTC CTT AAT TGT TTT TAC ATA GTT GT |
| si4R1 | GAT TAT AAA GAT GAC GAT GAT AAA NNB NNB NNB NNB NNB NNB AAA AAG ATT AGC AGA AAA GAA TAT GTT |
| si8R1 | GAT TAT AAA GAT GAC GAT GAT AAA NNB NNB NNB NNB NNB NNB AAA GAA GTA ACT TCT AAA CCA GCC A |
| UseqF1 | CCC AAC ATA TAA CAA TAC AAG TCC TAG CAT |
| UseqF3 | CAG TAG CAG GAC CTA CGC TAA |
| UseqR1 | CGT GGC AAG CAT GAT CCA TGA AGT |

### Bibliography

The references listed below as well as all references cited in the Specification supplement, explain, provide a background for or teach methodology, techniques and/or compositions employed herein.
1. US 6,570,004- Blaser et al. (2003).
2. US 6,680,179 - Collins et al. (2004).
3. US 6,383,496 - Curtiss et al. (2002).
4. US 6,150,170 - Powell et al. (2000).
5. US 6,410,012 - Seizmore et al. (2002).
6. US 6,550.419 - Hone et al. (2002).
7. US 6,531,313 - Goudsmit et al. (2003).
8. US 6,682,729 - Powell et al. (2004).
9. US Patent Publication 2005/0075298 - Chen et al. (2005).
10. US Patent Publication 2002/0176848 - Seizemore et al. (2002).
11. US Patent Publication 2005/0096288 - Guevara et al. (2005).
12. US Patent Publication 2004/0236072 - Olmsted et al. (2004).
13. US Patent Publication 2004/0203039 - Hensel et al. (2004).
14. US Patent Publication 2004/0005325 - Kusters et al. (2004).
15. US Patent Publication 2002/0032152 - Torossian (2002).
16. US Patent Publication 2003/0170264 - Turner et al. (2003).
17. US Patent Publication 2003/0204068 - Blasec et al. (2003).
18. US Patent Publication 2002/0161192 - Meyer et al. (2002).
19. WO96/33274 - Covacci et al. (1996).
20. WO99/21959 - Ellis et al. (1999).
21. WO01/94599 - Burman et al. (2001).
22. WO2005 021026 - Baron, et al. (2005).
23. Graham et al (2002), Gastroenterology, 123:1637-1648.
24. Liu et al (2005), World J. Gastroentero., 11(14): 2154-2156.
25. Conway (2005), Curr. Pharm. Res., 11(6): 775-90.
26. Sawker et al (2002), PNAS USA, 99(24): 15428-15433.
27. Sutton et al (2004), Vaccine, 22(20): 2541-6.
28. Kang et al (2005), World J. Gastroentero., 11(3): 454-456.
29. Moschos et al. (2004), Immunology and Cell Biology, 82(6): 628-637.
30. Reddy et al. (2004), Inter. J. Antimicrob. Agents, 24(6): 536-47.
31. Bai et al. (2003), Sheng Wugong Cheng Xu Bao, 19(4):433-8.
32. Nolta et al. (1992), J. Clin. Invest, 90(2): 342-348.
33. Shi et al. (2005), Helicobacter, 10(1): 71-9.
34. Deml et al. (2005), Infection Immunity, 73(8): 4732-42.
35. Cosima et al. (2005), Trends in Immunology, 26(4): 199-207.
36. Velin et al. (2005), Gastroenterology, 129(1): 142-155.
37. Kong et al. (2000), NAR, 28(17) 3216-3223.
38. Mao et al. (2003), World J. Gastroentero., 9(7): 1529-1536.
39. Chu et al. (2005), World J. Gastroentero, 11(23): 3518-22.
40. Kathy Parton. (2000), Institute of Veterinary, Animal and Biomedical Sciences, "Helicobacter mustelae as vector for biological control in stoats" Wildlife Health and Conservation Research Program; Landcare Research (Funding Body).
41. Forrester & Parton (2000), NZ Vet. J., 48: 65-69.
42. Spranger et al. (2005), Br. Nutr., 93 (6):765-71.
43. Garbom et al. (2004), Infect Immun., 72(3): 1333-1340.
44. Tschop et al. (2000), Nature, 407:908-13.
45. Choi et al. (2003), Edocrinology, 144 (3).
46. Remington's Pharmaceutical Sciences, 20th edition, Mack Publishing Company.
47. Jones et al. (2005), Nat. Med. 11 (7): 786-90.
48. Curtis (2005), In Mestecky, et al. (eds.), Mucosal Immunity, 3 ed. Academic Press, pp. 1009-1037.
49. Portal-Calthay & Perez-Perez (2006), Clin. Sci (Lond), 110:305-314.
50. Amieva et al. (2003), Science, 300:1430-1343.
51. Bina et al. (2000), J. Bacteriol., 182:2370-2375.
52. Franco et al (2005), PNAS USA, 102: 10646-10651.
53. Doig et al. (1995), J. Bacteriol, 177: 5447-5452.
54. Doig & Trust (1994), Infect Immun, 62, 4526-4533.
55. Odenbreit et al. (2000), Science, 287, 1497-1500.
56. Orsini et al. (1998), Helicobacter, 3: 15-20.
57. Hohlfeld et al. (2006), Mol Microbiol, 59: 1624-1637.
58. Sarker et al. (2004), Acta Paediatr, 93: 1432-1436.
59. Fischer et al. (2001), Infect Immun:, 67: 69-6775.
60. Chien et al. (1999), J Clin Microbiol, 37: 1393-1397.
61. Bubert et al. (1992), J Bacteriol, 174: 8166-8171.
62. Spreng et al. (2003), Vaccine, 21: 746-752.
63. Kuhn & Goebel (1989), Infect Immun, 57: 55-61.
64. Akopyants et al. (1995), Infect Immun, 63: 116-121.
65. Bijlsma et al. (1999), Infect Immun, 67: 2433-2440.
66. Wang et al. (1993), J Gen Microbiol, 139: 2485-2493.
67. Hanahan (1983), J. Mol Biol, 166: 557-580.
68. Dower et al. (1988), Nucleic Acids Res, 16: 6127-6145.
69. Chalker et al. (2001), J Bacteriol, 183: 1259-1268.
70. Heuermann & Haas (1998) Mol Gen Genet, 257: 519-528.
71. Copass et al. (1997) Infect Immun, 65: 1949-1952.

## Claims

1. A construct comprising a first *Helicobacter pylori* sequence being a first portion of a native UreA or UreB gene sequence and a second *Helicobacter pylori* sequence being a second portion of a native UreA or UreB gene and inserted within and between said first and second portions is a non*-Helicobacter* nucleotide sequence of interest, for use in a method of treatment of non*-Helicobacter* associated disease, wherein on expression of said construct said non*-Helicobacter* nucleotide sequence of interest is expressed together with a functional urease and delivered to the mucosal of said animal.

2. A construct according to claim 1, for use in a method of treatment according to claim 1, wherein the non*-Helicobacter* nucleotide sequence of interest encodes an antigen.

3. A construct according to any one of claims 1 or 2, for use in a method of treatment according to claim 1 or 2, wherein the non*-Helicobacter* nucleotide sequence of interest is inserted into UreA at amino acid position 103 or amino acid position 231 or amino acid position 239 or into UreB at amino acid position 2 or amino acid position 66 or amino acid position 327 or amino acid position 542 or amino acid position 550.

4. A *Helicobacter pylori* strain transformed with a construct according to any one of claims 1 to 3 to produce a recombinant *Helicobacter* for use in a method of treatment of non*-Helicobacter* associated disease.

5. A recombinant *Helicobacter pylori* strain according to claim 4, for use in a method of vaccination against said non*-Helicobacter* nucleotide sequence of interest.

6. A method for preparing a molecule of interest for delivery to the mucosal of an animal in need thereof comprising the steps of:
i) inserting a gene encoding a molecule of interest into the gene encoding the urease enzyme of *Helicobacter pylori* to produce an in-frame gene so that the urease enzyme function is not impaired when the in-frame gene is expressed; and
ii) transforming said construct into a *Helicobacter pylori.*

## Patentansprüche

1. Konstrukt, umfassend eine erste *Helicobacter pylori-*Sequenz*,* welche ein erster Teilbereich einer nativen UreA- oder UreB-Gensequenz ist, und eine zweite *Helicobacter pylori*-Sequenz, welche ein zweiter Teilbereich eines nativen UreA- oder UreB-Gens ist, und insertiert darin und zwischen den ersten und zweiten Teilbereichen ist eine Nicht-*Helicobacter*-Nucleotidsequenz von Interesse, zur Verwendung in einem Verfahren zur Behandlung einer Nicht-*Helicobacter*-assoziierten Krankheit, wobei bei der Expression des Konstrukts die Nicht-*Helicobacter*-Nucleotidsequenz von Interesse zusammen mit einer funktionalen Urease exprimiert und zur Mukosa des Tiers zugeführt wird.

2. Konstrukt nach Anspruch 1 zur Verwendung in einem Verfahren zur Behandlung nach Anspruch 1, wobei die Nicht-*Helicobacter*-Nucleotidsequenz von Interesse ein Antigen codiert.

3. Konstrukt nach einem der Ansprüche 1 oder 2 zur Verwendung in einem Verfahren zur Behandlung nach Anspruch 1 oder 2, wobei die Nicht-*Helicobacter*-Nucleotidsequenz von Interesse in UreA an Aminosäureposition 103 oder Aminosäureposition 231 oder Aminosäureposition 239 oder in UreB an Aminosäureposition 2 oder Aminosäureposition 66 oder Aminosäureposition 327 oder Aminosäureposition 542 oder Aminosäureposition 550 insertiert ist.

4. *Helicobacter pylori*-Stamm transformiert mit einem Konstrukt nach einem der Ansprüche 1 bis 3 zur Produktion eines rekombinanten *Helicobacter* zur Verwendung in einem Verfahren zur Behandlung einer Nicht*-Helicobacter*assoziierten Krankheit.

5. Rekombinanter *Helicobacter pylori-*Stamm nach Anspruch 4 zur Verwendung in einem Vakzinierungsverfahren gegen die Nicht-*Helicobacter*-Nucleotidsequenz von Interesse.

6. Verfahren zur Herstellung eines Moleküls von Interesse für die Zuführung zur Mukosa eines Tiers, welches dieses benötigt, umfassend die Schritte:
i) Insertion eines Gens, welches ein Molekül von Interesse codiert, in das Gen, welches das Ureaseenzym von *Helicobacter pylori* codiert, um ein In-frame-Gen zu produzieren, sodass die Enzymfunktion der Urease nicht beeinträchtigt ist, wenn das In-frame-Gen exprimiert wird; und
ii) Transformation des Konstrukts in ein *Helicobacter pylori.*

## Revendications

1. Construction comprenant une première séquence de *Helicobacter pylori* qui est une première partie d'une séquence de gène natif UreA ou UreB et une seconde séquence de *Helicobacter pylori* qui est une seconde partie d'un gène natif UreA ou UreB et une séquence nucléotidique d'intérêt n'appartenant pas à *Helicobacter* étant insérée dans et entre lesdites première et seconde parties, pour son utilisation dans un procédé de traitement d'une maladie non associée à *Helicobacter,* dans laquelle lors de l'expression de ladite construction, ladite séquence nucléotidique d'intérêt n'appartenant pas à *Helicobacter* est exprimée conjointement avec une uréase fonctionnelle et délivrée à la muqueuse dudit animal.

2. Construction selon la revendication 1, pour son utilisation dans un procédé de traitement selon la revendication 1, dans laquelle la séquence nucléotidique d'intérêt n'appartenant pas à *Helicobacter* code un antigène.

3. Construction selon l'une quelconque des revendications 1 et 2, pour son utilisation dans un procédé de traitement selon la revendication 1 ou 2, dans laquelle la séquence nucléotidique d'intérêt n'appartenant pas à *Helicobacter* est insérée dans UreA à la position d'acide aminé 103 ou à la position d'acide aminé 231 ou à la position d'acide aminé 239 ou dans UreB à la position d'acide aminé 2 ou à la position d'acide aminé 66 ou à la position d'acide aminé 327 ou à la position d'acide aminé 542 ou à la position d'acide aminé 550.

4. Souche de *Helicobacter pylori* transformée avec une construction selon l'une quelconque des revendications 1 à 3, pour produire une *Helicobacter* recombinante utilisable dans un procédé de traitement d'une maladie non associée à *Helicobacter.*

5. Souche de *Helicobacter pylori* recombinante selon la revendication 4, pour son utilisation dans un procédé de vaccination contre ladite séquence nucléotidique d'intérêt n'appartenant pas à *Helicobacter.*

6. Procédé de préparation d'une molécule d'intérêt à délivrer à la muqueuse d'un animal en ayant besoin, comprenant les étapes suivantes :
i) l'insertion d'un gène codant une molécule d'intérêt dans le gène codant l'enzyme uréase de *Helicobacter pylori* pour produire un gène *in situ* de façon que la fonction de l'enzyme uréase ne soit pas altérée quand le gène *in situ* est exprimé ; et
ii) la transformation de ladite construction dans une *Helicobacter pylori.*
